(19) Europäisches Patentamt
European Patent Office
Office européen des brevets

(11) **EP 1 895 302 A2**

(12) **EUROPEAN PATENT APPLICATION**

(43) Date of publication:
**05.03.2008 Bulletin 2008/10**

(51) Int Cl.:
**G01N 33/574** (2006.01)

(21) Application number: **07122383.8**

(22) Date of filing: **07.04.2004**

(84) Designated Contracting States:
**AT BE BG CH CY CZ DE DK EE ES FI FR GB GR HU IE IT LI LU MC NL PL PT RO SE SI SK TR**

(30) Priority: **08.04.2003 DK 200300541**
**16.07.2003 DK 200301085**

(62) Document number(s) of the earlier application(s) in accordance with Art. 76 EPC:
**04726097.1 / 1 613 966**

(71) Applicants:
• **Colotech A/S**
**2100 Copenhagen Ø (DK)**
• **Raskov, Hans Henrik**
**2900 Hellerup (DK)**

(72) Inventors:
• **Raskov, Hans Henrik**
**2900 Hellerup (DK)**

• **Albrethsen, Jacob**
**1666 Copenhagen V (DK)**
• **Gammeltoft, Steen**
**2900 Hellerup (DK)**
• **Bøgebo, Rikke Maria**
**2720 Vanløse (DK)**

(74) Representative: **Plougmann & Vingtoft A/S**
**Sundkrogsgade 9**
**P.O. Box 831**
**2100 Copenhagen Ø (DK)**

Remarks:
This application was filed on 05-12-2007 as a divisional application to the application mentioned under INID code 62.

(54) **A method of detecting of colorectal cancer in human samples**

(57) The present invention relates to a method of diagnosing colorectal cancer in human samples using several novel protein markers. The markers have been identified by assaying a number of tissue and serum samples from healthy individuals and persons diagnosed with colorectal cancer by means of protein chip technology using mass spectrometry. Differential expression pattern of these markers are indicative of a person having colorectal cancer and/or predictive of the stage of the disease in a colorectal cancer patient. The diagnosis is based on comparing at least one intensity value, obtained using the method, to a reference value.

Fig. 1

EP 1 895 302 A2

## Description

## Field of invention

The present invention relates to a method of diagnosing colorectal cancer in human samples using several novel protein markers. Differential expression pattern of these markers are indicative of a person having colorectal cancer and/or predictive of the stage of the disease in a colorectal cancer patient.

## Background

Colorectal cancer is one of the world's most common cancers and the second leading cause of death due to cancer in the western world. Investigations of colorectal cancer show that most colorectal cancers develop from adenomatous polyps. The polyps are usually small and pre-neoplastic growths that develop on the lining of the colon and can over time progress into colorectal cancer. Colorectal cancer occurs as a result of a sequence of mutations during a long period of time and these mutations mark the several different pathological stages of the disease. A model put forward by Fearon and Vogelstein describes colorectal cancer progression from normal epithelia to metastasis through the phases of dysplasia, early, intermediate and late adenoma and carcinoma.

A rare, inherited condition called familial polyposis (FAP) causes hundreds of polyps to form in the colon and rectum and unless this condition is treated, FAP is almost certain to lead to colorectal cancer. These individuals are therefore in a special need for an accurate screening test, where biopsies can be taken from a polyp during colonoscopy and analysed for neoplastic changes.

Several mutations in oncogenes and tumour-suppresser genes have been identified in colorectal cancers and some of them have been associated with the phases of the disease mentioned above.

The risk factors for developing colorectal cancer seem to be age, diet, colon polyps, personal medical history, family medical history and inflammatory bowel disease (Ulcerative colitis and Crohn's disease).

Colorectal cancer incidences and mortality rates increase with age and sharply so after the age of 60. It is estimated that more than one-third of colorectal cancer deaths could be avoided if people over the age of 50 had regular screening tests, since over 90% of all cases occur in people 50 and older. This is due to the fact that colorectal cancer is one of the most preventable cancers, if it is detected at its early stages. If screening tests were performed on the risk groups for colorectal cancer, it could help to prevent deaths due to the disease by finding pre-cancerous polyps so they can be removed before they turn into cancer.

Studies have shown that women with a history of cancer of the ovary, uterus, or breast have a somewhat increased chance of developing colorectal cancer. The risk of developing colorectal cancer the second time seems to be evident as well. So these findings suggest that personal medical history seems to be relevant in terms of the assessment of risk for colorectal cancer. The same seems to be true for family medical history. First-degree relatives (parents, siblings, children) of a person who has had colorectal cancer are somewhat more likely to develop this type of cancer themselves. Ulcerative colitis is a chronic condition where the lining of the colon becomes inflamed and persons having this condition are considered at a greater risk of developing colorectal cancer than others.

The usual diagnostic methods for colorectal cancer are procedures such as sigmoidoscopy and colonoscopy, that involve looking inside the rectum and the lower colon (sigmoidoscopy) or the entire colon (colonoscopy) and allowing for removal of polyps or other abnormal tissue for examination under a microscope. A polypectomy is the removal of polyp(s) during a sigmoidoscopy or colonoscopy, which is a procedure often performed on individuals suffering from FAP and individuals with sporadic, recurrent colorectal polyps. Another way is to do X-rays of the large intestine, which is a technique that can reveal polyps or other changes in the intestine. A much less cumbersome method, but less indicative, is the faecal occult blood test (FOBT). It is a test used to check for hidden blood in the stool, as it has been observed that cancers or polyps can bleed, and FOBT is able to detect small amounts of bleeding in the stool.

The potential use of mass spectrometry as an aid for diagnosing cancer has been demonstrated in WO 01/25791 A2, disclosing protein markers from prostate cancer patients being differently expressed as compared to samples from healthy subjects or patients with benign prostate hyperplasia (BPH).

Several studies describe useful markers for the diagnosis of colorectal cancer. US 6,455,668 describes a screening method for identifying bioactive agents being capable of binding to a colorectal cancer modulating protein (BCMP). It further describes a method for screening drug candidates, wherein a gene expression profile is used including CJA8, or fragments thereof. The expression profile can further include markers selected from the group consisting of CZA8, BCX2, CBC2, CBC1, CBC3, CJA9, CGA7, BCN5, CQA1, BCN7, CQA2, CGA8, CAA7 and CAA9 (WO 00/55633). Another publication, US 2001/0044113, describes the use of PKC isozyme, in combination with more conventional cancer markers such as bcl-2, bax and c-myc, to detect changes in colonocyte gene expression associated with early stages of colon tumorigenesis by isolation of poly A+ RNA from faeces. It should also be mentioned that the use of an undefined Defensin-polypeptide (Defensin-X) in diagnosing cancer is described in WO 99/11663.

There is, however, still unmet need for a simple diagnostic and/or prognostic test to provide an indication of whether or not an individual has colorectal cancer. It would also be of tremendous help to have a test giving indication of the status during surveillance of the disease.

**Summary of the Invention**

The present invention relates to a method of diagnosing colorectal cancer in a sample using novel protein markers. The markers have been identified by assaying a number of tissue and serum samples from healthy individuals and persons diagnosed with colorectal cancer by means of protein chip technology using mass spectrometry.

Differential expressions patterns of these markers are indicative of a person having colorectal cancer and/or predictive of the stage of the disease in a colorectal cancer patient. The diagnosis is based on comparing at least one intensity value, obtained using the method, to a reference value.

**Detailed description of the invention**

It is an object of preferred embodiments of the present invention to provide a method for diagnosing colorectal cancer in a sample from a mammal, the method comprising obtaining a sample from said mammal and assaying said sample by a quantitative detection assay, and determining the intensity signal of at least one marker.

In this text the words protein, peptide, polypeptide are used interchangeably, and all describe a chain of amino acids. In some cases the chain of amino acids have so called post translational modifications or bind certain ligands (for example ions). In some cases the chain of amino acid is a full-length (native) protein, in some cases it is a smaller fragment of a full-length protein. The mass values correspond solely to the measured mass.

The present invention relates to a number of markers. The at least one marker, such as two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, twenty-six, twenty-seven, twenty-eight, twenty-nine, thirty, thirty-one, thirty-two, thirty-three, thirty-four, thirty-five, thirty-six, thirty-seven, thirty-eight, thirty-nine, forty, forty-one, forty-two, forty-three, forty-four, forty-five, forty-six, forty-seven, forty-eight, forty-nine, fifty, fifty-one, fifty-two, fifty-three, fifty-four, fifty-five, fifty-six, fifty-seven, fifty-eight, fifty-nine, sixty, sixty-one, sixty-two, sixty-three, sixty-four, sixty-five, sixty-six, sixty-seven, sixty-eight, sixty-nine, seventy, seventy-one, seventy-two, seventy-three, seventy-four, seventy-five, seventy-six, seventy-seven, seventy-eight, seventy-nine, eighty, eighty-one, eighty-two, eighty-three, eighty-four, eighty-five, eighty-six, eighty-seven, eighty-eight, eighty-nine, ninety, ninety-one, ninety-two, ninety-three, ninety-four, ninety-five, ninety-six, ninety-seven, ninety-eight, ninety-nine, hundred, hundred and one, hundred and two, hundred and three, hundred and four, hundred and five, hundred and six, hundred and seven, hundred and eight, hundred and nine, hundred and ten, hundred and eleven, hundred and twelve, hundred and thirteen, hundred and fourteen, hundred and fifteen, hundred and sixteen, hundred and seventeen, hundred and eighteen, hundred and nineteen, hundred and twenty, hundred and twenty-one, hundred and twenty-two, hundred and twenty-three, hundred and twenty-four, hundred and twenty-five, hundred and twenty-six, hundred and twenty-seven, hundred and twenty-eight, hundred and twenty-nine, hundred and thirty, hundred and thirty-one, hundred and thirty-two, hundred and thirty-three, hundred and thirty-four, hundred and thirty-five, hundred and thirty-six, hundred and thirty-seven, hundred and thirty-eight, hundred and thirty-nine, hundred and forty, hundred and forty-one, hundred and forty-two, hundred and forty-three, hundred and forty-four, f hundred and orty-five, hundred and forty-six, hundred and forty-seven, hundred and forty-eight, hundred and forty-nine and hundred and fifty markers, can be selected from the group consisting of the polypeptides having apparent molecular weight of 66800 Da, 66500 Da, 66300 Da, 64860 Da, 60730 Da, 60500 Da, 60475 Da, 46000 Da, 45500 Da, 44300 Da, 33000 Da, 28040 Da, 28025 Da, 28010 Da, 28000 Da, 27700 Da, 19966 Da, 19900 Da, 19865 Da, 16150 Da, 15935 Da, 15580 Da, 15200 Da, 15140 Da, 14470 Da, 14300 Da, 14100 Da, 14030 Da, 13870 Da, 13747 Da, 11723 Da, 13700 Da, 13331 Da, 13265 Da, 12000 Da 11989 Da, 11987 Da, 11900 Da, 11700 Da, 11650 Da, 11550 Da, 11500 Da, 11133 Da, 11080 Da, 10830 Da, 9950 Da, 9700 Da, 9600 Da, 9197 Da, 9140 Da, 9090 Da, 9079 Da, 8971 Da, 8940 Da, 8931 Da, 8930 Da, 8652 Da, 8580 Da, 8230 Da, 7469 Da, 7324 Da, 7023 Da, 6880 Da, 6850 Da, 6660 Da, 6650 Da, 6635 Da, 6450 Da, 6436 Da, 6435 Da, 6430 Da, 6125 Da, 6110 Da, 6090 Da, 5920 Da, 5906 Da, 5905 Da, 5900 Da, 5871 Da, 5857 Da, 5540 Da, 5360 Da, 5330 Da, 5266 Da, 5260 Da, 5234 Da, 5075 Da, 4977 Da, 4749 Da, 4660 Da, 4640 Da, 4634 Da, 4500 Da, 4480 Da, 4460 Da, 4330 Da, 4300 Da, 4290 Da, 4281 Da, 4270 Da, 4266 Da, 4264 Da, 4168 Da, 4136 Da, 4039 Da, 4024 Da, 4000 Da, 3984 Da, 3980 Da, 3960 Da, 3895 Da 3882 Da, 3878 Da, 3816 Da, 3777 Da, 3712 Da, 3680 Da, 3651 Da, 3574 Da, 3570 Da (def 2), 3487 Da, 3480 Da (def 3),3450 Da (def 1), 3444 Da, 3408 Da, 3372 Da, 3280, 3275 Da, Da, 3160, Da, 2960 Da, 2955 Da, 2933 Da, 2878 Da, 2850 Da, 2840 Da, 2799 Da, 2693 Da, 2462 Da, 2450 Da, 2364 Da, 2330 Da, 2275 Da, 2230 Da, 2210 Da, 1945 Da,1930 Da, 1688 Da, 1536 Da, 1365 Da, 1256 Da, 1042 Da, 1026 Da, and 1005 Da.

Thereafter, the method in a preferred embodiment comprises comparing said intensity signal(s) with reference value(s) and identifying whether the intensity signal of at least one marker from the sample is significantly different from a reference value.

It is an object of the present invention to provide a method of diagnosing colorectal cancer in a sample from a mammal. The method comprises obtaining a sample from said mammal, detecting in the sample from the mammal at least one marker by a quantitative detection assay and determining the intensity signal of the least one marker, wherein the marker is selected from the group consisting of the polypeptides having apparent molecular weight of:

66800 Da, 66500 Da, 66300 Da, 64860 Da, 60730 Da, 60500 Da, 60475 Da, 46000 Da, 45500 Da, 44300 Da, 33000 Da, 28040 Da, 28025 Da, 28010 Da, 28000 Da, 27700 Da, 19966 Da, 19900 Da, 19865 Da, 16150 Da, 15935 Da, 15580 Da, 15200 Da, 15140 Da, 14470 Da, 14300 Da, 14100 Da, 14030 Da, 13870 Da, 13747 Da, 11723 Da, 13700 Da, 13331 Da, 13265 Da, 12000 Da 11989 Da, 11987 Da, 11900 Da, 11700 Da, 11650 Da, 11550 Da, 11500 Da, 11133 Da, 11080 Da, 10830 Da, 9950 Da, 9700 Da, 9600 Da, 9197 Da, 9140 Da, 9090 Da, 9079 Da, 8971 Da, 8940 Da, 8931 Da, 8930 Da, 8652 Da, 8580 Da, 8230 Da, 7469 Da, 7324 Da, 7023 Da, 6880 Da, 6850 Da, 6660 Da, 6650 Da, 6635 Da, 6450 Da, 6436 Da, 6435 Da, 6430 Da, 6125 Da, 6110 Da, 6090 Da, 5920 Da, 5906 Da, 5905 Da, 5900 Da, 5871 Da, 5857 Da, 5540 Da, 5360 Da, 5330 Da, 5266 Da, 5260 Da, 5234 Da, 5075 Da, 4977 Da, 4749 Da, 4660 Da, 4640 Da, 4634 Da, 4500 Da, 4480 Da, 4460 Da, 4330 Da, 4300 Da, 4290 Da, 4281 Da, 4270 Da, 4266 Da, 4264 Da, 4168 Da, 4136 Da, 4039 Da, 4024 Da, 4000 Da, 3984 Da, 3980 Da, 3960 Da, 3895 Da 3882 Da, 3878 Da, 3816 Da, 3777 Da, 3712 Da, 3680 Da, 3651 Da, 3574 Da, 3570 Da (def 2), 3487 Da, 3480 Da (def 3),3450 Da (def 1), 3444 Da, 3408 Da, 3372 Da, 3280, 3275 Da, Da, 3160, Da, 2960 Da, 2955 Da, 2933 Da, 2878 Da, 2850 Da, 2840 Da, 2799 Da, 2693 Da, 2462 Da, 2450 Da, 2364 Da, 2330 Da, 2275 Da, 2230 Da, 2210 Da, 1945 Da,1930 Da, 1688 Da, 1536 Da, 1365 Da, 1256 Da, 1042 Da, 1026 Da, and 1005 Da.

The method further comprises comparing said intensity signal(s) with reference value(s) and identifying whether the intensity signal of at least one marker from the sample is significantly different from the reference value for said marker.

In one aspect of the present invention a method is provided for diagnosing colorectal cancer by means of a serum sample from a mammal. The method comprises obtaining a serum sample from said mammal, detecting in the serum sample from the mammal at least one marker by a quantitative detection assay and determining the intensity signal of the at least one marker, wherein the marker is selected from the group consisting of the polypeptides having apparent molecular weight of:

66500 Da, 60500 Da, 46000 Da, 45500 Da, 44300 Da, 28040 Da, 27700 Da, 33000 Da, 19900 Da, 16150 Da, 15935 Da, 15580 Da, 15200 Da, 15200 Da, 13700 Da, 11900 Da, 11700 Da, 11650 Da, 11550 Da, 11500 Da, 11080 Da, 10830 Da, 9140 Da, 8940 Da, 8930 Da, 8230 Da, 6880 Da, 6650 Da, 6660 Da, 6450 Da, 6430 Da, 6125 Da, 6110 Da, 6090 Da, 5920 Da, 5900 Da, 5540 Da, 5330 Da, 5260 Da, 4660 Da, 4640 Da, 4460 Da, 4330 Da, 4300 Da, 4290 Da, 4000 Da, 3980 Da , 3960 Da, 3680 Da, 3280 Da, 3275 Da, Da, 3160 Da, 2955 Da, 2450 Da, and 1536 Da.

The method further comprises comparing said intensity signal(s) with reference value(s) and identifying whether the intensity signal of at least one marker from the sample is significantly different from the reference value for said marker.

In another aspect of the present invention a method is provided for diagnosing colorectal cancer in a tissue sample from a mammal. The method comprises obtaining a tissue sample from said mammal, detecting in the tissue sample from the mammal at least one marker by a quantitative detection assay and determining the intensity signal of the at least one marker, wherein the marker is selected from the group consisting of the polypeptides having apparent molecular weight of:

15140 Da, 11989 Da, 11987 Da, 9700 Da, 9600 Da, 9197 Da, 9079 Da, 8971 Da, 8652 Da, 8580 Da, 7324 Da, 7023 Da, 5871 Da, 5857 Da, 5360 Da, 5234 Da, 5075 Da, 4749 Da, 4634 Da, 4281 Da, 4266 Da, 4168 Da, 4039 Da, 4024 Da, 3984 Da, 3878 Da, 3777 Da, 3712 Da, 3651 Da, 3574 Da, 3487 Da, 3444 Da, 3408 Da, 3372 Da, 2933 Da, 2878 Da, 2840 Da, 2799 Da, 2693 Da, 2462 Da, 2364 Da, 2330 Da, 1930 Da, 1688 Da, 1365 Da, 1256 Da, 1042 Da, 1026 Da, and 1005 Da,

the method further comprises comparing said intensity signal(s) with reference value(s) for said marker(s) and identifying whether the intensity signal of at least one marker from the sample is significantly different from the reference value.

In yet another aspect of the present invention a method is provided for diagnosing colorectal cancer by means of a plasma sample from a mammal. The method comprises obtaining a plasma sample from said mammal, detecting in the plasma sample from the mammal at least one marker by a quantitative detection assay and determining the intensity signal of the at least one marker, wherein the marker is selected from the group consisting of the polypeptides having apparent molecular weight of:

66800 Da, 66500 Da, 66300 Da, 64860 Da, 60730 Da, 60475 Da, 19966 Da, 19865 Da, 14470 Da, 14300 Da, 14100 Da, 14030 Da, 13870 Da, 13747 Da, 11723 Da, 9950 Da, 8931 Da, 7469 Da, 6635 Da, 6435 Da, 5905 Da, 5266 Da, 4977 Da, 4480 Da, 4136 Da, and 3895 Da,

the method further comprises comparing said intensity signal(s) with reference value(s) for said markers and identifying whether the intensity signal of at least one marker from the sample is significantly different from the reference value for said marker.

Another embodiment of the present invention provides a use of at least one marker selected from the group consisting of the polypeptides having apparent molecular weight of

66800 Da, 66500 Da, 66300 Da, 64860 Da, 60730 Da, 60500 Da, 60475 Da, 46000 Da, 45500 Da, 44300 Da, 33000 Da, 28040 Da, 28025 Da, 28010 Da, 28000 Da, 27700 Da, 19966 Da, 19900 Da, 19865 Da, 16150 Da, 15935 Da, 15580 Da, 15200 Da, 15140 Da, 14470 Da, 14300 Da, 14100 Da, 14030 Da, 13870 Da, 13747 Da, 11723 Da, 13700 Da, 13331 Da, 13265 Da, 12000 Da 11989 Da, 11987 Da, 11900 Da, 11700 Da, 11650 Da, 11550 Da, 11500 Da, 11133 Da, 11080 Da, 10830 Da, 9950 Da, 9700 Da, 9600 Da, 9197 Da, 9140 Da, 9090 Da, 9079 Da, 8971 Da, 8940 Da, 8931 Da, 8930 Da, 8652 Da, 8580 Da, 8230 Da, 7469 Da, 7324 Da, 7023 Da, 6880 Da, 6850 Da, 6660 Da, 6650 Da, 6635 Da, 6450 Da, 6436 Da, 6435 Da, 6430 Da, 6125 Da, 6110 Da, 6090 Da, 5920 Da, 5906 Da, 5905 Da, 5900 Da, 5871 Da, 5857 Da, 5540 Da, 5360 Da, 5330 Da, 5266 Da, 5260 Da, 5234 Da, 5075 Da, 4977 Da, 4749 Da, 4660 Da, 4640 Da, 4634 Da, 4500 Da, 4480 Da, 4460 Da, 4330 Da, 4300 Da, 4290 Da, 4281 Da, 4270 Da, 4266 Da, 4264 Da, 4168 Da, 4136 Da, 4039 Da, 4024 Da, 4000 Da, 3984 Da, 3980 Da, 3960 Da, 3895 Da 3882 Da, 3878 Da, 3816 Da, 3777 Da, 3712 Da, 3680 Da, 3651 Da, 3574 Da, 3570 Da (def 2), 3487 Da, 3480 Da (def 3),3450 Da (def 1),3444 Da, 3408 Da, 3372 Da, 3280, 3275 Da, Da, 3160, Da, 2960 Da, 2955 Da, 2933 Da, 2878 Da, 2850 Da, 2840 Da, 2799 Da, 2693 Da, 2462 Da, 2450 Da, 2364 Da, 2330 Da, 2275 Da, 2230 Da, 2210 Da, 1945 Da,1930 Da, 1688 Da, 1536 Da, 1365 Da, 1256 Da, 1042 Da, 1026 Da, and 1005 Da,

for the prediction of the clinical outcome, complications and mortality of an individual diagnosed with colorectal cancer.

In the present context, the term "diagnosing" includes determining whether a person has colorectal cancer as well as indicating the stage or prognosis of a cancer in a patient.

As will be evident to a person of skill in the art, it is not always possible to diagnose with certainity whether a person has colorectal cancer by use of a method of the invention. Within the broad term "diagnosing" is thus also included determining a diagnosis by use of at least one of the markers disclosed herein with a certain specificity i.e. 50% or 60% and preferably with a higher specificity, such as 70%, 75%, 80%, 83%, 84%, 85%, 86%, 87%, 88%, 89%, 90%, 91%, 92%, 93%, 94%, 95%, 96%, 97%, 98%, 99%, or most preferably 100%.

The sensitivity of the method of diagnosing is also of importance. The sensitivity that the diagnosis provided by use of at least one of the markers disclosed herein is correct should be 50% or 60%, preferably higher such as 62%, 70%, 72%, 74%, 77%, 80%, 83%, 84%, 85%, 86%, 87%, 88%, 89%, 90%, 91%, 92%, 93%, 94%, 95%, 96%, 97%, 98%, 99%, or most preferably 100%.

The experimental part of the application provides a number of examples of preferred markers and combination of markers and the combination of specificity and sensitivity obtained when using said markers. These markers and combinations of markers are presently preferred embodiments of the invention.

In the context of the present invention, the term "prognosis" relates to an opinion (professional or non-professional, preferably a professional) on how an illness or a disease will develop and how the illness or disease will influence on other health conditions and death/survival of the mammal.

It is contemplated that by use of at least one of the markers of the invention or a combination of markers it will be possible to determine the prognosis or clinical outcome for an individual patient.

The present invention provides the means for giving a prognosis of the clinical outcome, complications and mortality of said mammal. In the context of the present invention, the term "clinical outcome" relates to the 'final result' or the 'final situation' or the condition of the patient after the patient has experienced a disease, e.g. a colorectal cancer or related diseases of the gastrointestinal tract. Thus, the clinical outcome may be death within a year or survival, and survival can be everything from poor health condition (moribund) to a healthy period for a period of 1, 2, 3, 4, 5, 6, 7, 8, 9, or 10 years.

In the context of the present invention, the term "complications" relates to symptoms of anything arising after the diagnosis of the disease, e.g the cancer spreading to other organs or tissues (metastasis), recurrence of carcinoma within the colon or development of a second primary colorectal cancer.

It should be understood that any feature and/or aspect discussed above in connection with the determination according to the invention apply by analogy to the "diagnosis", "prognosis" and "clinical outcome" according to the invention.

The term "colorectal cancer" relates to diseases such as colon cancer, familial adenomatous polyposis (FAP), rectal cancer and inflammatory bowel disease (IBD). It also relates to the non-invasive pre-cancerous lesions such as adenomatous polyps.

In the present context, the term "phases of colorectal cancer" relates to the progressive stage of the disease. This diagnosis of the severity of colorectal cancer is most often based on pathological observations after surgery. This currently used diagnostic model describes colorectal cancer progression from normal epithelia to metastasis through the phases of dysplasia, adenoma (early, intermediate and late) and carcinoma.

In the present context, the term mammal refers to a primate, preferably a human.

In order to detect the presence of a gene product in a biological sample, one can measure either DNA/RNA or protein or both using quantitative detection assay(s). Such detection assay can be selected from the group consisting of immunoassay, kinetic/real-time PCR, 2D gel, protein array, gene array and other nano-technology methods.

In the present context, the term "immunoassay" refers to assays such as ELISA (Enzyme-Linked Immunosorbent Assay), RIA (Radioimmunoassay) and FIA (Fluoroimmunoassay), which are based on the ELISA sandwich concept of catching antibody and detection antibody with different specificity to the same molecule. The detection antibody is then labelled with an enzyme, fluorochrome or a radioactive substance or the like, to quantify the desired molecule (protein), and the sensitivity of the assay depends partially on the label of the detection antibody.

The term" 2D-Gel" (two-dimensional electrophoresis) relates in the present context to the electrophoresis technique where a protein extract is subjected to an electrophoresis in one dimension and then directly afterwards to a second electrophoresis in a second dimension. The conditions during the separate steps are different, in terms of time of separation, voltage, buffer and agents present during the separation.

In a preferred embodiment of the present invention mass spectrometry is used to detect the protein markers. Furthermore the mass spectrometry method used is preferably a SELDI-TOF (Surface Enhanced Laser Desorption Ionization)-TOF (Time of Flight) technique, where the protein extract is bound to a protein chip. The chips have an active surface chemistry, which can be modified to retain proteins with certain properties. Thereby, proteins with different properties can be retained by different set of conditions and measured by MALDI-TOF or the like.

The SELDI-TOF/MS technique:

General description:

SELDI-TOF/MS (Surface Enhanced Laser Desorption/Ionisation-Time Of Flight/Mass Spectrometry) (Ciphergen) is mass spectrometry where the samples are purified on Protein Chips (Ciphergen) prior to analysis. In this purification step the majority of proteins (and salts & lipids) are removed and only a relatively small number of proteins remains on the chip surface. This chip is then analysed by mass spectrometry.

Protein chips and buffer solutions:

Different chips are available, and by varying the buffer solutions used in the binding and washing steps, different protein profiles are observed when analysing the chips in the PBS II instrument (Ciphergen). Thus, a person skilled in the art normally would test different chips and buffer solutions.

The chips are composed of common chromatographic materials, also used in HPLC techniques (anion-, cation, and hydrophobic-/reverse phase- surfaces) and the buffer solutions are also commonly used in other purification techniques. There is basically no difference between purification on a protein chip, as described here, and purification on a chromatographic column or by precipitating proteins by chromatographic pearls.

Analysis on the SELDI instrument:

The chips are analysed on the PBS II instrument (Ciphergen), which is an MALDI-TOF/MS (Matrix assisted Laser Desorption/Ionisation-Time Of Flight/Mass Spectrometry) instrument. The PBS II has a special loading device that allows analysis of protein chips, but is otherwise a normal MALDI-TOF/MS instrument. Instead of using specific chips, with specific binding abilities, a gold chip (Au Chip (Ciphergen)) can be used. In this case the protein solution is not purified on the chip but applied directly on to the gold surface and left to dry up together with the crystallisation solution; this is MALDI-TOF/MS.

Some proteins are present at very low concentrations in serum and can therefore only be detected after they have been in-concentrated on the protein chip (which is the initial step in the SELDI technique) and not directly by MALDI.

The only difference between SELDI and MALDI is that in SELDI the samples are treated in a special way before analysis. This allows for homogenous comparison of samples, which allows for sensitive identification of proteins that are differentially expressed. But the way the proteins are detected is exactly the same.

Basically, there is no difference between purifying proteins by standard purification techniques ((nano)-HPLC, gel-filtration, precipitation) and subsequently analysing the samples by MALDI, and by purifying the proteins on protein chips and analysing them by MALDI. The latter combination is called SELDI.

MALDI-TOF/MS is a technique that is highly sensitive in measuring the mass of molecules, especially proteins. The PBS II instrument has an accuracy of below +/- 0.2%, and in most cases around +/- 0.1%. Thus the mass value of a protein with m/z: 5000 is in most case m/z 5000 +/- 5. Therefore the measured masses are all defined as +/- maximum 0.2% and +/- minimum 0.1%.

Protein chips of the invention can be chips with an immobilized metal affinity capture array with a nitriloacetic acid (NTA) surface. An example of such a chip is the IMAC30 ProteinChip Array, which is activated with transition metals prior to use.

Other protein chips of the invention are arrays comprising a carboxylate chemistry (negatively charged) acting as a weak cation exchanger. The CM10 ProteinChip Array is an example of such an array.

Protein chips of the present invention may further be arrays, which bind proteins through reversed phase or hydrophobic interaction chromatography and have binding characteristics similar to that of a C6 to C12 alkyl chromatographic resin. The H50 ProteinChip array is an example of such an protein chip.

The protein chips of the present invention can also be arrays being strong anion exchange array comprising quaternary amine functionality such as the SAX2 ProteinChip Array.

Furthermore, the protein chips of the present invention can be mimic normal phase chromatography with silicate functionality such as the NP20 ProteinChip.

In the present context, the term "gene microarray" relates to low density nucleotide arrays, where nucleotide probes are attached or synthesised onto a surface and used as probes to retain nucleotides, mostly mRNA. This is usually referred to as transcription profiling, i.e. detection of the mRNA transcripts currently being used in a tissue at a certain time. Examples of such arrays are oligonucleotide arrays, where oligonucleotides are printed on glass slides and cDNA arrays, where cDNA (complementary DNA) is spotted on glass slide.

In a preferred embodiment of the present invention, the intensity signal detected in the quantitative detection assays is selected from the group consisting of fluorescence signal, mass spectrometry images, radioactivity, enzyme activity, and antibody detection.

The reference value can be calculated from a pool of samples from individuals with cancer and by comparison with a pool of samples from healthy individuals, a range for positive and negative calls can be made. Another possibility is to set a reference value based on a pool of samples from various phases or stages of the cancer to determine the progression or a stage of the disease. It may even be desirable to set reference values for prognosis of the disease. The reference value can be calculated as a mean or a median value of each intensity signal value(s) calculated from data from one or many of the markers, wherein the negative values are made positive. The reference value could even be the area under the curve (AUC) of at least one of the protein markers.

In one embodiment of the present invention the reference value is indicative of the stage of colorectal cancer. This may be accomplished by collecting a number of samples from several patients and after the samples have been diagnosed by the stage of the disease, the samples from the same stage are assayed.

In the present context, the reference value can be based on data calculated from intensity signal value(s) of said marker(s) obtained from a sample without colorectal cancer from the same mammal. The reference value can also comprise data calculated from intensity signal value(s) of said marker(s) obtained from samples from normal and colorectal cancer tissue from the same mammal. Samples can furthermore be obtained from both a healthy control population and a population having said cancer which samples are used to determine the reference value. After the reference value is determined with a statistical significance, such as but not limited to p-values of levels below 0.1. By assaying a significant number of patients and healthy individuals, the specificity of the method can be determined, obtaining a specified sensitivity. Thereby, it can be determined whether a person is likely to have colorectal cancer or not with a predetermined specificity and/or a predetermined sensitivity.

In the present context the term "data" relates to any calculation made using the intensity signal(s) as data input. The intensity signal(s) may be fluorescence signal, mass spectrometry images, radioactivity, spectrometry values, etc. The data can be obtained using any kind of mathematical formula or algorithm.

Samples for setting the reference value will vary depending on the purpose of the assay. For diagnosis tissue samples may be taken from a "normal" tissue section and a cancer from the same individual, but reference samples may also be taken from healthy individuals in this context. It is also possible to collect blood samples from healthy individuals together with blood samples from individuals, which are known to be suffering from colorectal cancer.

The prognosis of cancer patients is usually determined by the stage of the disease. The classification or the staging of the disease can be made using more than one model, but the most commonly used classification of colon cancer is based on the tumour morphology. This is the so-called Dukes' classification (referring to the original classification described by Lockhardt-Mummery & Dukes in the 1930'ies) classifying the disease into three stages using the terms Dukes' A-C. Dukes A describes a cancer, where the cancer is limited to the lining (mucosa or sub-mucosa) of the colon and has not penetrated the colon. At the Dukes' B stage, the cancer has penetrated the muscularis propria and invaded nearby organs. Dukes' C is characterised in that a regional metastasis of lymph nodes has occurred. Later, a commonly used stage "Dukes' D", referring to colorectal cancer with distant metastasis to organs like liver, lungs and brain was added to the classification. The 5-year survival prognosis for colorectal cancer is 80-90% at the Duke's A stage. Patients with Duke's B colorectal cancer have 60-70% 5-year survival rate whereas patients with Duke's C colorectal cancer are down to 20-30%. The 5-year survival rate for patients with Duke's D colorectal cancer is practically zero (Arends JW. et al.).

In a preferred embodiment of the present invention the reference value is indicative of the stage of colorectal cancer, wherein the stage is selected from the group consisting of Duke's A, Duke's B, Duke's C and Duke's D.

In the present context, the sample is a biological sample. The sample can be selected from the group consisting of blood, serum, plasma, faeces, saliva, urine, a cell lysate, a tissue sample, a biopsy, a tissue lysate, a cell culture, semen, seminal plasma, seminal fluid and cerebrospinal fluid.

In a preferred embodiment of the present invention a protein extract is made from the biological sample containing the total protein content including membrane proteins, nuclear proteins, cytosolic proteins and blood/serum proteins. When the protein extract has been established, the protein concentration of the extract is made constant. In the present context the term constant refers to that the protein concentration of the sample to be analysed should be standardised to a value being the same between different samples in order to be able to quantify the signal of the protein markers. Such standardisation could be made using photometry, spectrometry and gel electrophoresis.

In a presently preferred embodiment of the present invention, the intensity signal for markers 2850 Da, 3570 Da (def 2), 3450 Da (def 1), 3480 Da (def 3), 4270 Da, and/or 6850 Da, is preferably increased, whereas the intensity signal for markers 9090 Da and/or 12000 Da is preferably decreased. These markers are preferably selected for evaluation of the presence of the disease from tissue samples or biopsies. Furthermore, for evaluation of the presence of the disease from blood samples, the intensity signal for 5900 Da, 3882 Da, and/or 5906 Da, is preferably raised and the intensity signal for 3816 Da, 6436 Da, 13265 Da, 11133 Da, and/or 13331 is preferably decreased.

In a presently most preferred embodiment of the present invention, the intensity signal for markers 1945 Da and 2210 Da is decreased and the intensity signal for 5906 is increased. These markers are preferably selected for evaluation of the presence of the disease from blood samples.

In another presently preferred embodiment of the present invention, the intensity signal for markers 1945 Da, 2210 Da, 2230 Da, 2250 Da, 2275 Da, 4300 Da, 4480 Da, and/or 4500 Da is decreased. These markers are preferably selected for evaluation of the presence of the disease from blood samples.

In a further presently preferred embodiment of the present invention, the intensity signal for marker 5906 Da is raised. This marker is preferably selected for evaluation of the presence of the disease from blood samples.

Also in a presently preferred embodiment of the present invention, the intensity signal for marker 1945 Da is decreased. This marker is preferably selected for evaluation of the presence of the disease from blood samples.

Also in a presently preferred embodiment of the present invention, the intensity signal for marker 2210 Da is decreased. This marker is preferably selected for evaluation of the presence of the disease from blood samples.

One aspect of the present invention provides the use of degradation products of Human Serum Albumin as marker for cancer. The degradation products are selected from the group consisting of the polypeptides having apparent molecular weights of 60500 Da, 6187 Da, 6090 Da, 5920 Da, 5906 Da, 5901 Da, 5900 Da, and 5333 Da.

In an embodiment of the present invention the use of at least one polypeptide having apparent molecular weight of 6187 Da, 5901 Da, or 5333 Da as a marker for cancer is provided, wherein at least one of the polypeptides is alpha-fibrinogen protein. In the present context the cancer is colorectal cancer.

In a presently preferred embodiment of the invention, the intensity signal for markers 66800 Da, 66500 Da, 66300 Da, 64860 Da, 46000 Da, 45500 Da, 44300 Da, 28040 Da, 28025 Da, 28010 Da, 28000 Da, 27700 Da, 15580 Da, 15140 Da, 13700 Da, 13331 Da 13265 Da, 12000 Da, 11989 Da, 11133 Da, 9700 Da, 9600 Da, 9197 Da, 9090 Da, 9079 Da, 8971 Da, 8940 Da, 8931 Da, 8652 Da, 8580 Da, 8230 Da, 7324 Da, 7023 Da, 6880 Da, 6660 Da, 6650 Da, 6635 Da, 6450 Da, 6436 Da, 6435 Da,6430 Da, 5360 Da, 5075 Da, 4749 Da, 4660 Da, 4640 Da, 4634 Da, 4500 Da, 4480 Da, 4330 Da, 4300 Da, 4290 Da, 4168 Da, 4000 Da, 3984 Da, 3980 Da , 3960 Da, 3816 Da, 3777 Da, 3680 Da, 3280 Da, 3160 Da, 2450 Da, 2330 Da, 2275 Da, 2230 Da, 2210, 1945 Da, 1930 Da 1536 Da, 1365 Da, 1256 Da, 1042 Da, 1026 Da, and 1005 Da is increased and the intensity signal for markers 66500 Da, 46000 Da, 45500 Da, 44300 Da, 28040 Da, 27700 Da, 15580 Da, 15140 Da, 13700 Da, 13331 Da 13265 Da, 12000 Da, 11989 Da, 11133 Da, 9700 Da, 9600 Da, 9197 Da, 9090 Da, 9079 Da, 8971 Da, 8940 Da, 8652 Da, 8580 Da, 8230 Da, 7324 Da, 7023 Da, 6880 Da, 6660 Da, 6650 Da, 6450 Da, 6436 Da, 6430 Da, 5360 Da, 5075 Da, 4749 Da, 4660 Da, 4640 Da, 4634 Da, 4500 Da, 4480 Da, 4330 Da, 4300 Da, 4290 Da, 4168 Da, 4000 Da, 3984 Da, 3980 Da , 3960 Da, 3816 Da, 3777 Da, 3680 Da, 3280 Da, 3160 Da, 2450 Da, 2330 Da, 2275 Da, 2230 Da, 2210, 1945 Da, 1930 Da 1536 Da, 1365 Da, 1256 Da, 1042 Da, 1026 Da, and 1005 Da is decreased.

In an embodiment of the present invention the intensity signal for markers 60500 Da, 19900 Da, 11080 Da, 10830 Da, 9140 Da, 8930 Da, 6110 Da, 6090 Da, 5920 Da, 5900 Da, 5540 Da, 5330 Da, 5260 Da, 4460 Da, and 2960 Da is increased and the intensity signal for markers 66500 Da, 44300 Da, 28040 Da, 27700 Da, 15580 Da, 13700 Da, 6880 Da, 6660 Da, 6430 Da, 4660 Da, 4640 Da, 4330 Da, 4300 Da, 4290 Da, 4000 Da, 3980 Da , 3960 Da, 3680 Da, 3280 Da, and 3160 Da is decreased when assaying a serum sample on IMAC30 chip (Ciphergen).

In an embodiment of the present invention the intensity signal for markers 11900 Da, 11700 Da, 11650 Da, 11550 Da, and 11500 Da is increased and the intensity signal for markers 46000 Da, 45500 Da, 8940 Da, 8230 Da, 6650 Da, and 6450 Da is decreased when assaying a serum sample on H50 protein chip.

In an embodiment of the present invention the intensity signal for markers 15200 Da, 6125 Da, 5900 Da, 3275 Da, and 2955 Da is increased and the intensity signal for markers 4290 Da, 2450 Da, 1536 Da is decreased when assaying a serum sample on CM10 protein chip.

In an embodiment of the present invention the intensity signal for markers 33000 Da, 16150 Da, 15935 Da, and 15200 Da is increased when assaying a serum sample on Sax2protein chip.

In an embodiment of the present invention the intensity signal for markers 5857 Da, 4264 Da, 3878 Da, 3712 Da, 3651 Da, 3574 Da, 3487 Da, 3444 Da, 3372 Da, and 1688 Da is increased and the intensity signal for markers 9700 Da, 8652 Da, 8652 Da, 8580 Da, 7023 Da, 5360 Da, 4168 Da, 1365 Da, 1256 Da, 1042 Da, 1026 Da, and 1005 Da is decreased when assaying a tissue sample on NP20 protein chip.

In an embodiment of the present invention the intensity signal for markers 11987 Da, 5871 Da, 5234 Da, 4281 Da, 4266 Da, 4039 Da, 4024 Da, 3408 Da, 2933 Da, 2878 Da, 2840 Da, 2799 Da, 2693 Da, 2462 Da, and 2364 Da is increased and the intensity signal for 15140 Da, 11989 Da, 9600 Da, 9197 Da, 9079 Da, 8971 Da, 7324 Da, 5075 Da, 4749 Da, 4634 Da, 3984 Da, 3777 Da, 2330 Da, and 1930 Da is decreased when assaying a tissue sample on Sax2protein chip.

In a presently preferred embodiment of the invention the intensity signal for markers 5340 Da and 5906 Da is increased and the intensity signal for 3980 Da, 6880 Da, and 28010 is decreased when assaying a serum sample on IMac30 chip.

In the present context, the term "plasma sample" relates to a sample wherein a blood sample is tapped into "EDTA-liquid-glass", centrifuged and where the supernatant is optionally frozen immediately at -80˚C.

In the present context, the term "serum sample" relates to a sample wherein a blood sample is tapped into a dry-glass, left to coagulate at room temperature for one hour, after which they are centrifuged and the supernatant is optionally frozen immediately at - 80˚C.

In the present context, the term "increased" in relation to the term "intensity signal" for a marker, refers to a comparison of an intensity signal from a sample to a reference value, wherein the samples have been normalized to ion noise or "housekeeping genes". The intensity signal for a specific marker, having a certain size, weight, number of nucleotides or amino acids, is "increased" if it is higher in the sample as compared to the reference value. If the term "raised" is used this is to be interpreted to also mean "increased".

In the present context, the term "decreased" in relation to the term "intensity signal" for a marker, refers to a comparison of an intensity signal from a sample to a reference value, wherein the samples have been normalized to ion noise or "housekeeping genes". The intensity signal for a specific marker, having a certain size, weight, number of nucleotides or amino acids, is "decreased" if it is lower in the sample as compared to the reference value.

In one aspect of the present invention a method is provided for determining the presence of colorectal cancer on the basis of a sample from a mammal. The method comprises selecting a normalized protein expression data set from the sample, wherein the expression data set comprises a plurality of expression intensities of proteins on at least one protein chip. Thereafter, at least one marker is selected from the normalized protein expression data set from the group consisting of the polypeptides having apparent molecular weight of:

66500 Da, 60500 Da, 46000 Da, 45500 Da, 44300 Da, 33000 Da, 28040 Da, 27700 Da, 19900 Da, 16150 Da, 15935 Da, 15580 Da, 15200 Da, 15140 Da, 13700 Da, 13331 Da, 13265 Da, 12000 Da 11989 Da, 11987 Da, 11900 Da, 11700 Da, 11650 Da, 11550 Da, 11500 Da, 11133 Da, 11080 Da, 10830 Da, 9700 Da, 9600 Da, 9197 Da, 9140 Da, 9090 Da, 9079 Da, 8971 Da, 8940 Da, 8930 Da, 8652 Da, 8580 Da, 8230 Da, 7324 Da, 7023 Da, 6880 Da, 6850 Da, 6660 Da, 6650 Da, 6450 Da, 6436 Da, 6430 Da, 6125 Da, 6110 Da, 6090 Da, 5920 Da, 5906 Da, 5900 Da, 5871 Da, 5857 Da, 5540 Da, 5360 Da, 5330 Da, 5260 Da, 5234 Da, 5075 Da, 4749 Da, 4660 Da, 4640 Da, 4634 Da, 4500 Da, 4480 Da, 4460 Da, 4330 Da, 4300 Da, 4290 Da, 4281 Da, 4270 Da, 4266 Da, 4264 Da, 4168 Da, 4039 Da, 4024 Da, 4000 Da, 3984 Da, 3980 Da, 3960 Da, 3882 Da, 3878 Da, 3816 Da, 3777 Da, 3712 Da, 3680 Da, 3651 Da, 3574 Da, 3570 Da (def 2), 3487 Da, 3480 Da (def 3),3450 Da (def 1),3444 Da, 3408 Da, 3372 Da, 3280, 3275 Da, Da, 3160, Da, 2960 Da, 2955 Da, 2933 Da, 2878 Da, 2850 Da, 2840 Da, 2799 Da, 2693 Da, 2462 Da, 2450 Da, 2364 Da, 2330 Da, 2275 Da, 2230 Da, 2210 Da, 1945 Da,1930 Da, 1688 Da, 1536 Da, 1365 Da, 1256 Da, 1042 Da, 1026 Da, and 1005 Da. Thereafter the weight for said at least one marker is set and the intensities of said at least one marker is/are multiplied with the weight of said at least one marker. If the markers are more than one the sum of the multiplication obtained above is calculated and that sum value is compared with a cut off value (as explained in example 7).

In the present context the weight for each marker is set by assigning a number between - 0.9 and +0.9 to each marker. The exact number (between -0.9 and +0.9) is selected as the number that results in the highest combination of a sensitivity and specificity value. This can be tested as shown in table 15 in example 7.

In a presently preferred embodiment the determination is based on the following algorithm:

Give the selected markers weights between -0.9 and 0.9, i.e. marker A, weight a, marker B, weight b, marker C, weight c, marker D, weight d and marker N, weight n;
get intensities of A, B, C, D,..N markers in the following order: A m/z, B m/z, C m/z, D m/z, N .. m/z;
multiply the first intensity with weight a;
multiply the second intensity with weight b;
multiply the third intensity with weight c;
multiply the fourth intensity with weight d;
multiply the n intensities with weight n; and
calculate the sum of the above multiplications.

If sum lower than cutoff value => sample is negative for colon cancer.

If sum higher than cutoff value => sample is positive for colon cancer.

In another aspect of the present invention a computer system for monitoring colorectal cancer in a mammal is provided. The computer system comprises a storage means for electronically storing data, processing means for storing input data from a mass spectrometer, input means for interfacing between an mass spectrometer and the computer system, and an interface between a user and the computer system, wherein the processing means determines the likelihood of colorectal cancer by applying the following algorithm:

Give the selected markers weights between -0.9 and 0.9, i.e. marker A, weight a, marker B, weight b, marker C, weight c, marker D, weight d and marker N, weight n;
get intensities of A, B, C, D,..N markers in the following order: A m/z, B m/z, C m/z, D m/z, N .. m/z;
multiply the first intensity with weight a;
multiply the second intensity with weight b;
multiply the third intensity with weight c;
multiply the fourth intensity with weight d;
multiply the n intensities with weight n; and
calculate the sum of the above multiplications.

If sum lower than cutoff value => sample is negative for colon cancer.

If sum higher than cutoff value => sample is positive for colon cancer.

In the present context, the term "cutoff" in relation to the program refers to a value for classification. The predicted grouping of a sample is classified as positive for colon cancer if it is above the cutoff value and negative for colon cancer if it is below the cutoff value.

In mass spectrometry the measured mass is given i Daltons (Da) or m/z. Dalton is a weight unit, wherein m/z relates to mass over charge (mass/charge). In the present context there is no difference between Daltons (Da) or m/z.

In the present context, the term "storage means" relates to hard disk, DVD disk, CD disk or floppy diskettes for storing digital data.

In the present context, the term "processing means" relates to a computer comprising a processor, RAM memory, etc...

In the present context, the term "interface between a user and the computer system" relates to keyboard, computer mouse, and a monitor.

In one aspect of the present invention a kit for diagnosis of colorectal cancer is provided, the kit comprising: a first antibody including a portion bound to a solid phase and a region which specifically binds to alpha-fetoprotein, a second antibody including a region which specifically binds to alpha-fetoprotein and a portion which has a label, and optionally a reference protein.

In another aspect of the present invention a kit for diagnosis of colorectal cancer is provided, the kit comprising: a first antibody including a portion bound to a solid phase and a region which specifically binds to alpha-fibrinogen, a second antibody including a region which specifically binds to alpha-fibrinogen and a portion which has a label, and optionally a reference protein.

In yet another aspect of the present invention a kit for diagnosis of colorectal cancer is provided, the kit comprising: a first antibody including a portion bound to a solid phase and a region which specifically binds to human serum albumin (HSA) or fragments of HSA, a second antibody including a region which specifically binds to human serum albumin (HSA) or fragments of HSA and a portion which has a label, and optionally a reference protein.

In an embodiment of the present invention the kit for diagnosis of colorectal cancer may comprise components to detect one or more of the proteins alpha-fetoprotein, alpha-fibrinogen and human serum albumin (HSA). The antibodies may recognise epitopes which are only exposed when the protein is degraded.

In the present context the term "epitope" relates to a certain area on the surface of the protein comprising a number of amino acids.

Several mutations in oncogenes and tumour-suppresser genes have been identified in colorectal cancer. The majority of these genes are associated with certain phases of the disease. A mutation in the tumour-suppresser gene Adenomatous Polyposis Coli gene (APC), is considered to be a molecular "gatekeeper" for development of adenomas and it has been estimated that over 80% of all colorectal cancers have a somatic mutation in the APC gene. There are actually very few oncogenes, which have been shown to be involved with colorectal cancers apart from k-ras, but a small percentage of colorectal cancers show mutations in the myc, myb and neu oncogenes. A mutation in k-ras is considered to be an intermediate event in colorectal carcinogenesis advancing the disease from early adenoma to intermediate adenoma. Several other products of tumour-suppresser genes have also been associated with colorectal cancer, many of those genes are located on the long arm of chromosome 18. Allelic loss on 18q has been associated with the DCC gene (deleted in colorectal cancer), MADR2 gene (also known as JV18) and DPC4 gene (deleted in pancreatic cancer), the last two are players in the TGF-beta signalling pathway. It has been proposed that DCC, DPC4 and MADR2 play a role in the progression over to late adenoma (Gryfe R et al.).

One of the best known and studied tumour-suppresser genes, p53, is associated with driving the disease towards carcinoma. The product of the gene, which is located on chromosome 17, is a nuclear protein and has a function in cell cycle regulation, but a loss of heterozygocity on 17p has been demonstrated in over 70% of all colorectal cancers.

In a preferred embodiment of the present invention, the detection method using at least one of the novel protein markers for the detection of colorectal cancer could be supplemented with the detection of one or more protein markers selected from the group consisting of APC, k-ras, myc, myb, neu, DCC, DPC4, MADR2, p53, BCMP, CJA8, CZA8, BCX2, CBC2, CBC1, CBC3, CJA9, CGA7, BCN5, CQA1, BCN7, CQA2, CGA8, CAA7, CAA9, PKC isozyme, bcl-2, bax, TIMP-1 and c-myc.

**Figure legends**

*Figure 1.*
Average intensity values of markers of colorectal cancer. Tissue samples from 12 cancer patients including a normal tissue sample and cancer tissue sample from the same individual were homogenised and protein extracts were analysed by mass-spectrometry using SAX2 chips and the SELDI-TOF technique. The figure shows the intensity levels of the markers selected based on highest sensitivity and specificity.
*Figure 2.*
Discriminating values calculated for 8 markers. The average intensity value for each marker was calculated for normal and cancer tissue sample sets, after removing the highest and lowest values. The discriminating value for each marker was found by dividing the average intensities from each of the sample sets.
*Figure 3.*
Average intensity values of possible markers in serum. Serum samples from 10 cancer patients and 10 healthy individuals were analysed by mass-spectrometry using IMAC3 chips and the SELDI-TOF technique. The figure shows the intensity levels of the markers selected based on highest intensity.

*Figure 4*

| Serum marker: 1945 Da. | | |
|---|---|---|
| Signal intensity | Cancer | Normal |
| middle | 2.39339 | 24.94229 |
| Max | 8.899157 | 77.64356 |
| Min | 0.211373 | 2.690569 |

Threshold value: 8.9 (maximum value for cancer serum)
12 out of 78 normal serum samples fall below threshold, producing a specificity of 85%.

*Figure 5*

| Serum marker 2210 Da | | |
|---|---|---|
| Signal intensity | Cancer | Normal |
| middle | 2.902108887 | 23.80824 |
| Max | 12.68954992 | 44.71738 |
| Min | 0.113351842 | 0.988566 |

Threshold value: 12.7 (maximum value for cancer serum)
18 out of 78 normal serum samples fall below threshold, producing a specificity of 77%.

*Figure 6*

| Serum marker 2230 Da | | |
|---|---|---|
| Signal intensity | Cancer | Normal |
| mid | 1.302903945 | 13.56049 |

(continued)

| Serum marker 2230 Da | | |
| --- | --- | --- |
| Signal intensity | Cancer | Normal |
| max | 5.682529669 | 31.203 |
| min | 0.012316878 | 0.637036 |

Threshold value: 5.6 (maximum value for cancer serum)
18 out of 78 normal serum samples fall below threshold, producing a specificity of 77%.

*Figure 7*

| Serum marker 2250 Da | | |
| --- | --- | --- |
| Signal intensity | Cancer | Normal |
| mid | 1.204193541 | 7.006661 |
| max | 3.640628662 | 20.46203 |
| min | 0.234108032 | 0.550792 |

Threshold value: 3.6 (maximum value for cancer serum)
22 out of 78 normal serum samples fall below threshold, producing a specificity of 72%.

*Figure 8*

| Serum marker 2275 Da | | |
| --- | --- | --- |
| Signal intensity | Cancer | Normal |
| mid | 0.821724872 | 4.189622 |
| max | 3.090245007 | 14.90973 |
| min | 0.125868733 | 0.245692 |

Threshold value: 3.1 (maximum value for cancer serum)
30 out of 78 cancer serum samples fall below threshold, producing a specificity of 62%.

*Figure 9*

| Serum marker 4300 Da | | |
| --- | --- | --- |
| Signal intensity | Cancer | Normal |
| mid | 0.358838372 | 2.662629 |
| max | 1.082232326 | 10.52571 |
| min | 0.029092626 | 0.225152 |

Threshold value: 1.1 (maximum value for cancer serum)
20 out of 78 cancer serum samples fall below threshold, producing a specificity of 74%.

*Figure 10*

| Serum marker 4475 Da | | |
| --- | --- | --- |
| Signal intensity | Cancer | Normal |
| mid | 0.828595247 | 3.363255 |
| max | 2.067939342 | 7.826388 |
| min | 0.035968835 | 0.900171 |

Threshold value: 2.1 (maximum value for cancer serum)
20 out of 78 cancer serum samples are below threshold, producing a specificity of 74%.

*Figure 11*

| Serum marker 4500 Da | | |
|---|---|---|
| Signal intensity | Cancer | Normal |
| mid | 0.821256006 | 3.360526 |
| max | 2.067939342 | 7.826388 |
| min | 0.035968835 | 0.889889 |

Threshold value: 2.1 (maximum value for cancer serum)
20 out of 78 cancer serum samples are below threshold, producing a specificity of 74%.

*Figure 12*

| Serum marker 5.9 Da. | | |
|---|---|---|
| Signal intensity | Cancer | Normal |
| middle | 5.088206618 | 1.413438 |
| max | 13.43115416 | 5.412548 |
| min | 0.638267678 | 0.182963 |

Threshold value: 5.4 (maximum value for normal serum)
49 out of 78 cancer serum samples fall below threshold, producing a specificity of 37%.
*Figure 13*
Peptide pattern in the region from 1900 to 2500 Da.
*Figure 14*
Mass spectra from a same sample analysed by the SELDI TOF technique (A) and the MALDI-TOF technique (B)
*Figure 15*
A scatter-plot of the sample scores and variable loading of a data set comprising data from healthy individuals and individuals diagnosed with colon cancer.
*Figure 16*
A and B. Representative SELDI-TOF/MS spectra of normal colon tissue (A) on NP20 chip and normal serum (B) on iMAC30 chip. The two spectra differ significantly and each produce a total of 40 to 60 peaks, the majority of which lie in the specified range from 2 to 10 kDa.
C. Comparison of typical colon tumour spectrum (above) and normal colon spectrum (below) in the range from 3 to 4 kDa. The arrows point to the three differentially expressed peptides, subsequently identified as HNP 1-3. The three peptides are expressed in both the normal colon samples and the colon tumour samples, but the expression is up-regulated in the cancer samples. The same observation was made in the serum screening, but here the average signal intensity was significantly lower.
*Figure 17*

A. HNP profiles of normal and colon tumour tissue. 40 colon tumour and 40 normal colon tissue samples were analysed on NP20 chips. Differences in mean intensities of HNP1-3 in normal and colon tumour tissue are statistical significant at 5% level (p<0.0005).
B. HNP profiles of normal and colon cancer serum. Serum samples (125 colon cancer and 100 normal) were analysed on iMAC30 chips. The mean intensities are significantly different at 5% level (p<2.2e-16). The box-plot shows the 25th quintile, median, 75th quantile, and whiskers extend to min. and max. values.

*Figure 18*
Protein extract from tumour tissue was separated on a peptide gel-filtration column. The elution volumes of forty (unidentified) peptides is plotted against their respective mass values and an approximate elution curve is calculated. The arrows point to HNP 1-3, which are eluted in two fractions: in the void volume (8ml) together with High Mass proteins (above 20 kDa) and after 14ml together with peptides of similar mass range (2-4kDa). We interpret this as evidence for binding between HNP 1-3 and High Mass proteins.

*Figure 19*

Normal microscopy (A&B) and fluorescence microscopy (C&D) of MDCK cells. MDCK cells were exposed to calcein with (A&C) and without HNP 1-3 (B&D). By fluorescence microscopy (C&D) the cells were observed to uptake calcein only when treated with fractions containing HNP 1-3/calcein(C). Fractions containing other peptides (unidentified peptides also purified from colon tumours) were used as negative controls together with calcein and did not stimulate the cells to uptake calcein (D) Also, cell islands treated with HNP 1-3 appeared diffuse and showed enlarged nuclei, indicating apoptosis (A).

*Figure 20*

A-E shows the average intensity spectra of healthy individuals (solid) and patients diagnosed with colon cancer (dashed). The standard errors of means (SEM) are shown with bars.

**Examples**

EXAMPLE 1 IDENTIFICATION OF BIOMARKERS FOR COLORECTAL CANCER BY TISSUE INVESTIGATIONS

The aim of the study was to identify protein markers indicative of colorectal cancer by comparison of normal and cancer tissue from colon and rectum.

*Method*

Sample preparation

Samples from 12 cancer patients were collected. Normal tissue samples and cancer tissue samples from the same colon were taken and frozen at -80˚C. Prior to analysis the samples were taken out of the freezer and placed into homogenisation/Lysis buffer.

Lysis buffer:

100 mM TRIS, pH 8.0
9.5 M UREA
1% CHAPS.

The samples were homogenised in a Wheaton Overhead Stirrer for 2 minutes at speed step 2.

Analysis

Protein extracts were analysed by mass-spectrometry using the SELDI-TOF technique.

SAX2 chips were pre-treated with 50 $\mu$l 100 mM TRIS pH 8.0 buffer.

10 $\mu$l homogenised sample + 60 $\mu$l TRIS pH 8.0 buffer were mixed and incubated on SAX2 Chip in a Bioprocessor for 30 minutes at room temperature. Thereafter spots were washed twice in 250 $\mu$l 100 mM TRIS pH 8.0 for 5 minutes.

2 times 0.5 $\mu$l Matrix (CHCA) was applied onto spot surface.

Instrument settings

Proteinchips were analysed at Laser intensities of 190, 210, and 230, and the sensitivity level was set at 8.

*Results*

Putative markers were identified by visual examination of the mass spectra from cancer and normal samples.

Table 1. Mass values of proteins showing increased expression in cancer tissue:

| Laser Intensity | | |
|---|---|---|
| **190** | **210** | **230** |
| 2305 Da | 2305 Da | 2305 Da |
| - | 2460 Da | 2460 Da |
| - | 2840 Da | - |
| 2850 Da | 2850 Da | 2850 Da |
| 2991 Da | - | - |
| 3370 Da | 3370 Da | - |
| 3440 Da | 3440 Da | - |
| 3480 Da | 3480 Da | - |
| - | 4275 Da | 4275 Da |
| - | - | 6850 Da |

Table 2. Mass values of proteins showing decreased expression in cancer tissue:

| Laser Intensity | | |
|---|---|---|
| **190** | **210** | **230** |
| 1925 Da | | |
| - | | 1940 Da |
| - | | 5000 Da |
| - | | 6190 Da |
| - | 6375 Da | |
| - | 6575 Da | |
| - | | 6590 Da |
| - | | 7570 Da |
| - | | 8410 Da |
| - | | 8700 Da |
| - | | 9090 Da |
| - | | 11670 Da |
| - | | 12000 Da |

Possible markers:

In order to the determine the specificity and sensitivity of the possible markers all spectres were normalised based on total ion current.

Table 3. Specificity and sensitivity of protein markers showing increased expression in cancer tissue:

| Size (Da) | Specificity(%) | Sensitivity (%) |
|---|---|---|
| 2300 | 83 | 66 |
| 2460 | 75 | 83 |
| 2850 | 100 | 92 |

(continued)

| Size (Da) | Specificity(%) | Sensitivity (%) |
|---|---|---|
| 2840 | 66 | 92 |
| 2990 | 75 | 50 |
| 3370 | 75 | 83 |
| 3450 | 83 | 83 |
| 3480 | 83 | 92 |
| 4270 | 92 | 92 |
| 6850 | 91 | 92 |

Table 4. Specificity and sensitivity of protein markers showing decreased expression in cancer tissue.

| Size (Da) | Specificity(%) | Sensitivity (%) |
|---|---|---|
| 1920 | 75 | 50 |
| 1940 | 67 | 25 |
| 5000 | 50 | 50 |
| 6190 | 83 | 75 |
| 6375 | 67 | 100 |
| 6575 | 58 | 58 |
| 7590 | 83 | 50 |
| 8410 | 58 | 42 |
| 8700 | 66 | 58 |
| 9090 | 83 | 83 |
| 11670 | 83 | 50 |
| 12000 | 83 | 83 |

*Possible multi-protein marker:*

Based on values of sensitivity and specificity the most promising single protein markers were selected:

Table 5. Protein markers showing increased expression in cancer tissue:

| Size (Da) | Specificity(%) | Sensitivity (%) | Identification |
|---|---|---|---|
| 2850 | 100 | 92 | N.D. |
| 3370 | 75 | 83 | Alfa-Defensin-2 |
| 3450 | 83 | 83 | Alfa-Defensin-1 |
| 3480 | 83 | 92 | Alfa Defensin-3 |
| 4270 | 92 | 92 | N.D. |
| 6850 | 92 | 92 | N.D. |

Table 6. Protein markers showing decreased expression in cancer tissue.

| Size (Da) | Specificity(%) | Sensitivity (%) | Identification |
|---|---|---|---|
| 9090 | 83 | 83 | N.D. |

(continued)

| Size (Da) | Specificity(%) | Sensitivity (%) | Identification |
|---|---|---|---|
| 12000 | 83 | 83 | N.D. |

*Conclusion*

Eight promising single protein markers were found using the SELDI-TOF mass-spectrometry technique and applying samples on protein-chips. Three of the markers have been fully identified as Alpha-Defensin 1, 2, and 3. A multi-protein marker based on a combination of one or more of the eight proteins shown above appears to be a very effective way of screening for colorectal cancer.

EXAMPLE 2 IDENTIFICATION OF BIOMARKERS FOR COLORECTAL CANCER IN SERUM

The aim of the study was to identify protein markers indicative of colorectal cancer by comparison of serum samples from normal and cancer patients.

*Method*

Sample preparation

Serum was isolated from blood of 10 patients diagnosed as having colorectal cancer and 10 healthy individuals.

Analysis

An IMAC3 chip was pre-treated with 2 times 5 $\mu$l 100mM NiSO4 followed by wash with 5 $\mu$l MQ water and equilibration with 2 times 5 $\mu$l binding buffer.

Binding buffer:

100mM TRIS HCl, pH 7.5
500mM NaCl
0.1% Triton X-100

2 $\mu$l of each serum sample was diluted in 48 $\mu$l binding buffer of which 4 $\mu$l was applied to the protein chip surface. The chip was left on shaker at room temperature for 40 minutes. The sample was removed from the chip surface and each spot was washed with 3 times 5 $\mu$l washing buffer (PBS, pH 7.4, 700mM NaCl). Finally the chip was air-dried and 2 times 0.6 $\mu$l CHCA (100%) was applied to each spot.
Protein extracts were analysed by mass-spectrometry using the SELDI-TOF technique.

Instrument settings

Protein-chips were analysed at varying laser intensities and sensitivity levels to obtain optimal spectra.

*Results*

Sensitivity and specificity of putative serum markers:

Table 7. Protein markers showing increased expression in serum samples of cancer patients:

| Size (Da) | Specificity(%) | Sensitivity (%) | Identification |
|---|---|---|---|
| 5905 | 70 | 70 | N.D. |
| 5899 | 70 | 70 | N.D. |
| 5928 | 70 | 70 | N.D. |

(continued)

| Size (Da) | Specificity(%) | Sensitivity (%) | Identification |
|---|---|---|---|
| 3882 | 60 | 60 | N.D. |

Table 8. Protein markers showing decreased expression in serum samples of cancer patients.

| Size (Da) | Specificity(%) | Sensitivity (%) | Identification |
|---|---|---|---|
| 3816 | 60 | 60 | N.D. |
| 6435 | 60 | 60 | N.D. |
| 13265 | 60 | 60 | N.D. |
| 11132 | 50 | 50 | N.D. |
| 13331 | 50 | 50 | N.D. |

*Conclusion*

Eight possible single protein markers were found using the SELDI-TOF mass-spectrometry technique and applying serum samples on protein-chips. None of the markers have been fully identified and annotated. A multi-protein marker based on a combination of one or more of the eight proteins shown above appears to be a very effective way for diagnosis of colorectal cancer.

EXAMPLE 3 SERUM SCREENING

*Materials and method*

Chip:

Serum samples were analysed on IMAC3 chip (Ciphergen).

Pre treatment:

Each spot is outlined with hydro pen.
5$\mu$l 100 mM NiSO4 is added, shake (150rpm) 1 min. Remove. Repeat once.
5$\mu$l MQ water is added shake 1 min. Remove.
5$\mu$l Bind buffer is added shake 1min. Remove.

Binding step:

Chip is placed in Bioprocessor.
50 $\mu$l binding buffer + 5$\mu$l serum is mixed in eppendorf tube, solution is loaded in bioprocessor. Leave on shaker (250 rpm) for 40 min. Remove.

Washing step:

200 $\mu$l washing buffer is added. Shake (250 rpm) 5min. Remove. Repeat once.

Dry step:

Chips are removed from bioprocessor and left to air dry for 20 minutes.

Crystallisation step:

0.6 ul matrix solution is added to each spot. Air dry chip for 5 min.
Repeat once.

Analysis:

Chips are analysed on PBS II instrument (Ciphergen) at laser intensity 210 and detector sensitivity 4.

*Results*

Biomarker Wizard analysis
78 colon cancer serum and 78 normal serum samples were analysed as described above.
All spectra were pooled and normalised based on total ion current.
Possible markers were identified by Biomarker Wizard (Ciphergen) analysis with the following parameter settings:

First pass: 5, Min peak threshold: 0%, Cluster mass window: 0.3 %, Second pass: 5. Based on the results from the Biomarker Wizard 9 peptides showed promising marker characteristics.

Mass values of possible serum marker peptides:

Down-regulated in colon cancer serum:

1945, 2210, 2230, 2250, 2275, 4300, 4480, 4500 Da.

Up-regulated in colon cancer serum:

5906 Da.

Threshold values for possible serum markers

Optimal threshold values for the 9 serum markers were selected in order to determine maximum specificity of individual markers:

| Marker (Da) | Specificity (%) |
|-------------|-----------------|
| 1945 | 85 |
| 2210 | 77 |
| 2230 | 77 |
| 2250 | 72 |
| 2275 | 62 |
| 4300 | 74 |
| 4480 | 74 |
| 4500 | 74 |
| 5906 | 37 |

*Principal component analysis*

Based on principal component analysis of a sample set of 38 cancer serum and 31 normal serum, it was shown that especially three markers were of high importance for discriminating between cancer and normal serum.

*Conclusion*

Especially important markers: 1945 Da, 2210 Da, and 5906 Da.

EXAMPLE 4 USE OF SELDI-TOF/MS OR MALDI-TOF/MS FOR DETECTION OF BIOMARKERS FOR COLORECTAL CANCER.

The aim of this study was to compare the outcome of markers detected with different expression of proteins in healthy individuals vs, patients diagnosed with colorectal cancer, using either SELDI-TOF/MS or an MALDI-TOF/MS.

*Method*

The PBS II instrument allows variation of three important parameters when analysing protein chips or MALDI-TOF/MS samples.
Laser intensity, detector sensitivity and optimisation range.
Laser intensity was permanently set at 220. However, since the laser source is constantly becoming weaker as the instrument is being used, and varies significantly from instrument to instrument, this is not a value that has any general meaning. Most often values from 190 to 230 are chosen.
Detector sensitivity was set at values of 3,4,5,6,7,8 depending on the signal. The intensity (and only the intensity, not the protein profile) of the sample is highly dependent on the matrix solution which is made immediately prior each screening. The detector sensitivity value is chosen such that none of the protein peaks will ever produce a signal that overrides the maximum limit. Thus the appropriate detector value will depend on the specific matrix solution, and thus has no general meaning.
Optimisation range, this range specifies the mass interval where the instrument will measure the signal with highest accuracy. For each screening we made two measurements. One with low optimisation range (m/z 2000-20000) and one with high (m/z 20000-150000) The identified markers below m/z 20000 were all measured in the low screening and the markers above m/z 20000 were all measured in the high screening
Protein chips were analysed on the PBS II SELDI instrument (Ciphergen). SPA (Sinapinic Acid) matrix was used in the crystallisation step in all screenings:
SPA (Ciphergen) was dissolved in 150 $\mu$l MQ + 150$\mu$l Acetonitrile + 1,5 $\mu$l TFA (tri-flouro-acetic-acid) and left on shaker for 10 minutes and centrifuged at 14.000 rpm for 15 minutes.

*Analysis*

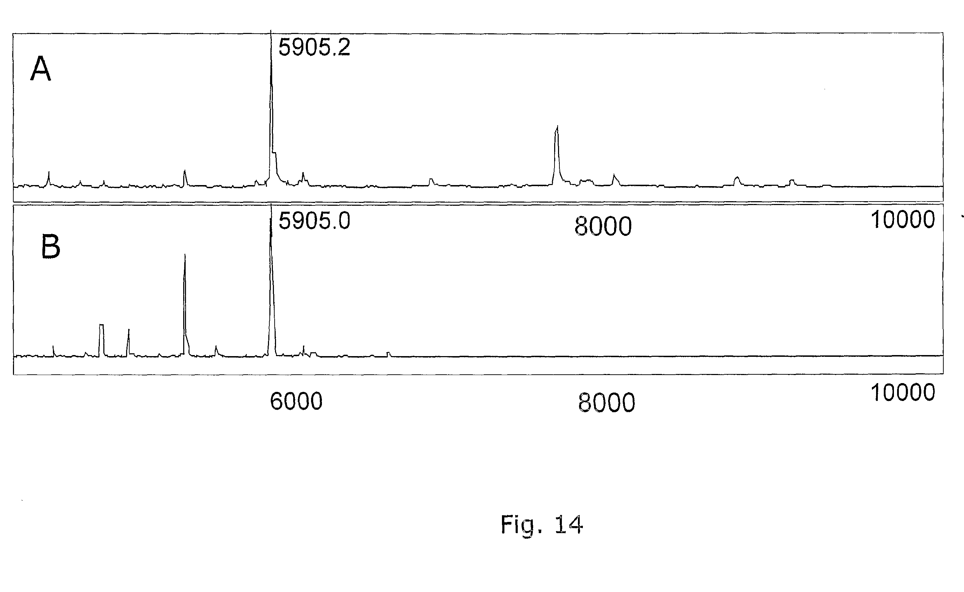

Mass spectra from serum samples of healthy individuals and patients diagnosed with colorectal cancer were analysed for potential markers.
An analysis of a serum sample by SELDI-TOF/MS indicated a protein marker of m/z 5900. The same sample was prepared for MALDI-TOF/MS analysis by removing salt and lipids from serum by gel-filtration. The results shown in figure 14 disclose this same protein with the same mass value as the SELDI-TOF/MS analysis did.

EXAMPLE 5 IDENTIFICATION OF SERUM MARKERS USING SEVERAL TYPES OF PROTEIN CHIPS

The aim of this study was to analyse the effect of using different protein chips in differential protein expression analysis using SELDI mass spectrometry.

*Materials and methods*

Samples

The IMAC study was based on analysis of serum from 12 cancer patients and 35 healthy individuals. The other studies (CM10, H50, and SAX2) were based on studies of analysis of serum from 8 cancer patients and 8 healthy individuals.
Cancer serum samples were obtained from cancer patients prior to surgery. Normal serum was obtained from a group of healthy individuals matched by age and gender to the cancer patients. Serum samples were stored at -80˚C until use. Samples were assayed by the SELDI-TOF/MS technique (Ciphergen).

Sample preparation

Samples were pre-treated by applying 5$\mu$l of pre-treatment solution to the chip surface and the chip was left on shaker for 5 minutes. The pre-treatment solution varies for different chip types. This process was repeated twice. The chip was washed in MQ-water twice and once in binding buffer.
Serum samples were thawed on ice and 5 $\mu$l serum was diluted in 50 $\mu$l binding buffer and left on shaker for 40 minutes. Next the samples were removed and chips were washed twice in washing buffer, followed by wash in MQ-water.

Chips were left to dry at room temp for 20 minutes. 0.6 µl crystallisation solution was applied twice.

Analysis

The PBS II instrument (Ciphergen) was calibrated prior to use and chips were analysed with detector sensitivity and laser intensity at suitable values.

*Data mining:*

All spectra were pooled into one experiment file and were normalised based on total ion current. Markers were identified by the Biomarker Wizard software (Ciphergen) and markers were compared and combined by principal component analysis

*Description of chips used for serum screening.*

As described, the protein chip surfaces are composed of common chromatographic resins commonly used in other purification techniques:

IMAC30 ProteinChip Array

The IMAC30 ProteinChip Array is an immobilised metal affinity capture array with a nitriloacetic acid (NTA) surface. The IMAC30 ProteinChip Array is activated with transition metals prior to use.

CM10 ProteinChip Array

The CM10 ProteinChip Arrays incorporate carboxylate chemistry (negatively charged) that acts as a weak cation exchanger.

H50 ProteinChip Array

H50 ProteinChip Arrays bind proteins through reversed phase or hydrophobic interaction chromatography and have binding characteristics similar to that of a C6 to C12 alkyl chromatographic resin.

SAX2 ProteinChip Array

The SAX2 ProteinChip Array is a strong anion exchange array with quaternary amine functionality.

*Description of buffers used for binding and washing steps in the serum screening*

The buffer solutions used, are common buffers used in other purification techniques:

IMAC30 screening
Pre-treatment :    100 mM NiSO4
Binding buffer :    100 mM TRIS, pH 7.5; 500 mM NaCl; 0.1% Triton X-100
Washing buffer:    PBS, pH 7.5; 700 mM NaCl


CM10 screening
Pre-treatment :    None
Binding buffer :    50 mM TRIS, pH 7.5
Washing buffer:    50 mM TRIS, pH 7.5

H50 screening
Pre-treatment :    100% acetonitrile
Binding buffer :   PBS, pH 7.4; 10% ACN; 250 mM NaCl
Washing buffer:    PBS, pH 7.4; 10% ACN; 250 mM NaCl


SAX2 screening
Pre-treatment :    None
Binding buffer :   50 mM TRIS, pH 8.0; 0.1% Triton X-100
Washing buffer:    50 mM TRIS, pH 8.0; 0.1% Triton X-100


*Results*

Table 10 Possible markers detected using different protein chips.

| Chip | Up-regulated | Down-regulated |
|---|---|---|
| **H50** | 11900 Da, 11700 Da, 11650 Da, 11550 Da, 11500 Da | 46000 Da, 45500 Da, 8940 Da, 8230 Da, 6650 Da, 6450 Da |
| **CM10** | 15200 Da, 6125 Da, 5900 Da, 3275 Da, 2955 Da | 4290 Da, 2450 Da, 1536 Da |
| **SAX2** | 33000 Da, 16150 Da 15935 Da, 15200 Da | |
| **IMAC30** | 60500 Da, 19900 Da, 11080 Da, 10830 Da, 9140 Da, 8930 Da , 6110 Da, 6090 Da, 5920 Da, 5900 Da, 5540 Da, 5330 Da, 5260 Da, 4460 Da, 2960 Da | 66500 Da, 44300 Da, 28121 Da, 28010 Da, 28315 Da, 27700 Da, 15580 Da, 13700 Da, 6880 Da, 6660 Da, 6430 Da, 4660 Da, 4640 Da, 4330 Da, 4300 Da, 4290 Da, 4000 Da, 3980 Da , 3960 Da, 3680 Da, 3280 Da, 3160 Da |

Only markers with above 70% sensitivity are shown.

*Conclusion*

We have compared the protein population of serum from colon cancer patients with serum from healthy individuals by different methods (different chips and different binding conditions). By the described procedure, we have identified a number of proteins that are differentially expressed (either up- or down-regulated) in serum from colon cancer patients compared to serum from normal individuals.
We find that the IMAC30 screening gives the prominent results, and the markers obtained from these screenings have been shown to have predictive power in discriminating between samples from healthy individuals and patients diagnosed with colorectal cancer.
The difference of markers detected in serum of this study as compared to the study described in example 1 is based on the state of the samples. The samples of this study were freshly frozen and thawed once prior to analysis, whereas the samples from example 1 have been thawed and refrozen several times.
The study further shows that some markers are detected on more than one type of chip, such as the up-regulation of 5900 as well as the down-regulation of 4290 on both CM10 and IMAC. Moreover, the study shows that by using more than one type of chip, the number of markers detected by using this technology can be increased considerably.

EXAMPLE 6 IDENTIFICATION OF BIOMARKERS FOR COLON CANCER BY DATA MINING OF MASS SPECTRA

The aim of this study was to separate healthy individuals from colorectal cancer patients using a Principal Component Analysis (PCA) on a normalised data set from mass spectra.

*Methods*

Samples

Serum samples were obtained from 12 healthy individuals and 35 patients diagnosed with colon cancer and the samples were assayed on IMAC30 chips according to the protocol described above in example 5.

Data mining

Raw data sets from mass spectra were normalised based on total ion current.
Data sets containing m/z, intensity and area of the peaks identified by "biomarker wizard" were generated as follows:

Data set 1: 4 healthy individuals and 4 patients diagnosed with colon cancer.
Data set 2: 8 healthy individuals and 8 patients diagnosed with colon cancer.
Data set 3: 12 healthy individuals and 35 patients diagnosed with colon cancer.

Computer programs:

Ciphergen ProteinChip Software with "biomarker wizard".
Multi Variate Statistical Program (MVSP), Kovack Computing.

*Parameters*

Biomarker wizard settings:

First pass: 5
Min peak threshold: 0
Cluster mass window: 0.3
Second pass: 2

Principal Component Analysis settings (MVSP):

Data standardised: Yes
Data centred: Yes

*Results:*

Principal component analysis of data set 1 resulted in two distinct groups, and identified as healthy individuals and patients with colon cancer. The separation was on the first principal component and all peaks irrelevant for the separation was removed from the analysis. Potential markers: 2960, 3170, 3980, 4650, 5340, 5906, 6120, 6840, 6880, 8940, 9140, and 28010 were identified.
Principal component analysis of data set 2 resulted in two distinct groups, and identified as healthy individuals and patients with colon cancer.
Potential markers: 1530, 3980, 4650, 5340, 5545, 5906, 6090, 6120, 6880, 11799, 13745, and 28010 were identified.
The most prominent combination of markers in both data set 1 and 2 were the following markers: 3980, 5340, 5906, 6880, and 28010 with 100% sensitivity and 100% specificity.
Data set 3 was used to verify the power of the selected markers.

Table 11: Sample scores of data set 2 using the following markers: 3980, 5340, 5906, 6880, and 28010.

| Sample ID | Sample scores on PC1 | Group |
|-----------|----------------------|-------|
| Chip17-A | -0.336 | cancer |
| Chip17-B | 0.64 | normal |
| Chip17-C | -0.592 | cancer |
| Chip17-D | 0.639 | normal |

(continued)

| Sample ID | Sample scores on PC1 | Group |
|---|---|---|
| Chip17-E | -0.248 | cancer |
| Chip17-F | 0.46 | normal |
| Chip17-G | -0.154 | cancer |
| Chip17-H | 0.148 | normal |
| Chip17-A(2) | -0.317 | cancer |
| Chip17-B(2) | 0.114 | normal |
| Chip17-C(2) | -0.442 | cancer |
| Chip17-D(2) | 0.591 | normal |
| Chip17-E(2) | -0.934 | cancer |
| Chip17-F(2) | 0.616 | normal |
| Chip17-G(2) | -0.541 | cancer |
| Chip17-H(2) | 0.356 | normal |

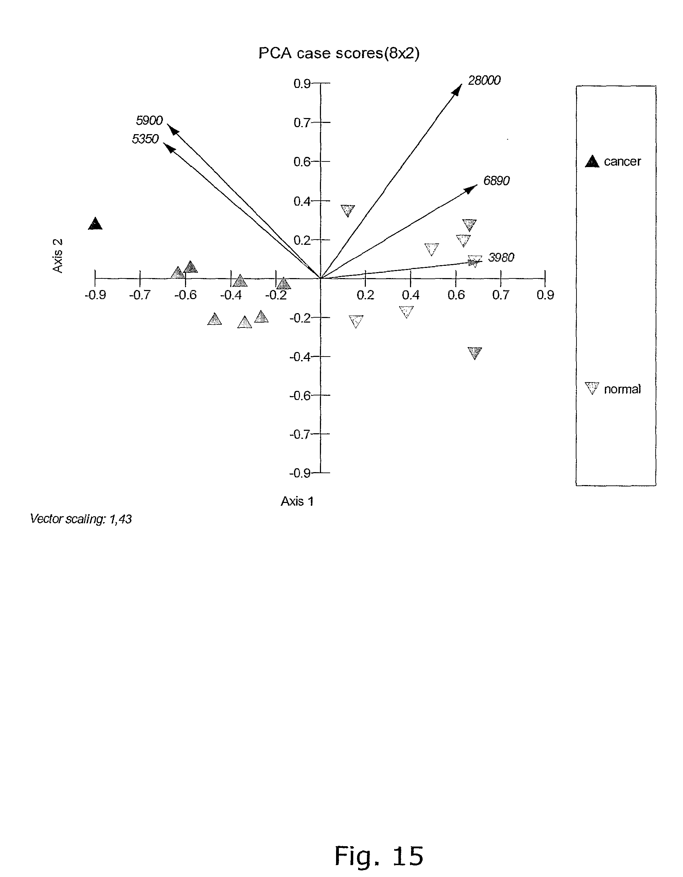

Figure 15 shows a scatter-plot of the sample scores and variable loading of data set 2. The figure demonstrates the power of the PCA.

Table 12 The sensitivity and specificity of data set 3.

| | Data set 3 |
|---|---|
| Sensitivity | 84 % |
| Specificity | 83 % |

The theoretical example shown here below demonstrates the power of the prediction model.
The intensity and m/z of the 5 markers (3980, 5340, 5906, 6880, and 28010) were then used on a data set comprising 2 healthy individuals, 2 patients diagnosed with colon cancer, and 4 unknown by applying PCA.

Table 13 Sample scores from PCA of samples form healthy individuals, cancer patients and samples from unknown subjects.

| Sample ID | Group | Sample score on PC1 |
|---|---|---|
| Chip25A | Cancer | -0.669 |
| Chip25B | Healthy | 0.995 |
| Chip25C | Cancer | -0.686 |
| Chip25D | Healthy | 0.520 |
| Chip25E | Unknown | 0.403 |
| Chip25F | Unknown | -0.425 |
| Chip25G | Unknown | -0.805 |
| Chip25H | Unknown | 0.666 |

Table 14 Numeric distance of sample scores from table 13.

| | | Numeric distance of sample scores on C1 | | | |
|---|---|---|---|---|---|
| Sample ID | Group | Chip25E | Chip25F | Chip25G | Chip25H |
| Chip25A | Cancer | 1.072 | 0.244 | 0.136 | 1.335 |

(continued)

| | | Numeric distance of sample scores on C1 | | | |
|---|---|---|---|---|---|
| Sample ID | Group | Chip25E | Chip25F | Chip25G | Chip25H |
| Chip25B | Healthy | 0.592 | 1.24 | 1.8 | 0.329 |
| Chip25C | Cancer | 1.089 | 0.261 | 0.119 | 1.352 |
| Chip25D | Healthy | 0.117 | 0.945 | 1.325 | 0.146 |
| Highest resemblance: | | Chip25D | Chip25A | Chip25C | Chip25D |
| Prediction: | | Healthy | Cancer | Cancer | Healthy |

*Conclusion:*

Principal Component Analysis can separate healthy individuals from patients with colon cancer using the intensity of the selected markers.

EXAMPLE 7 A METHOD FOR DISCRIMINATING BETWEEN HEALTHY INDIVIDUALS AND PATIENTS WITH COLON CANCER

The aim of the study was to develop a method for discriminating between healthy individuals and patients with colon cancer based on data from mass spectra generated using protein chips and the SELDI TOF mass spectrometry technique.

*Data mining*

Data sets:

Data set A: Intensities of the five serum markers from 24 patients diagnosed with colon cancer and 47 healthy individuals.
Data set B: Data set A minus the average of the intensity in healthy individuals.
The intensities were normalised based on total ion current.

Data format

The input data from each sample contained: Sample ID, intensity of 3980, 5340, 5906, 6880, 28010, and a grouping variable (1 = cancer, 0 = healthy).

Cut-off values

Three different cut-off values were analysed (0.4, 0.5, and 0.6).

Predicted grouping

If the predicted result is above cut-off, the sample is classified as positive for colon cancer (1).
If the predicted result is below cut-off, the sample is classified as negative for colon cancer (0).

Weights

The number of weights is 5 (one for each marker).
The weight is a number between -0.9 and 0.9.

Calculation

The program reads the data-file line by line, and stores them. For each combination of weights and each sample the predicted grouping is calculated:

$$\text{Predicted grouping} = a * \text{int}(3980) + b * \text{int}(5340) + c * \text{int}(5906) * 0.1 + d * \text{int}(6880) + e * \text{int}(28010)$$

$$\text{weights} = a,b,c,d,e \quad \text{int}(3980) = \text{intensity of marker 3980 Da, etc.}$$

Specificity and sensitivity is calculated, based on the predicted result, cut-off value, and grouping variable.

In order to identify the parameters for predicting cancer from a biological sample using selected markers, the following algorithm was used:

The input-file consists of intensities of the five markers and the desired result (if cancer = 1, if healthy = 0)

Place all lines from input-file in a list

A weight can take one of the following: -0.9, -0.8, -0.7, -0.6, -0.5, -0.4, -0.3, -0.2, -0.1, 0.1, 0.2, 0.3, 0.4, 0.5, 0.6, 0.7, 0.8, 0.9.

Make all possible weight combinations:

for each possible factor that the first weight can take and
for each possible factor that the second weight can take and
for each possible factor that the third weight can take and
for each possible factor that the fourth weight can take and
for each possible factor that the fifth weight can take.

for each cut-off (0.4, 0.5, 0.6)
for each possible combination of weights and each input line:

one = intensity of 3980 * first weight
two = intensity of 5340 * second weight
three = intensity of 5906 * third weight * 0.1
four = intensity of 6880 * fourth weight
five = intensity of 28010 * fifth weight

calculated result = one + two + three + four + five

if desired result = 1 and calculated result >= cutoff, true positive + 1
if desired result = 0 and calculated result < cutoff, true negative + 1
if desired result = 1 and calculated result < cutoff, false negative + 1
if desired result = 0 and calculated result >= cutoff, false positive + 1

calculate sensitivity and specificity for this combination of weights

sensitivity = ((true positive / (true positive + false negative)) * 100
specificity = ((true negative / (true negative + false positive)) * 100

change specificity and sensitivity into integers
if sensitivity > 70 and specificity > 70
join sensitivity, specificity, cutoff, and weights in one line place the line in an array

When all combinations of cut-off, weights, and input have been explored sort the array.

*Results*

Table 15: Different weight combinations and the corresponding specificity and sensitivity.

| No. | Data set | Cut-off value | Weights | | | | | Specificity % | Sensitivi % |
|---|---|---|---|---|---|---|---|---|---|
| | | | a | b | c | d | e | | |
| 1 | A | 0.4 | -0.9 | 0.3 | 0.1 | 0.3 | -0.6 | 87 | 95 |
| 2 | A | 0.4 | -0.9 | 0.4 | 0.2 | -0.1 | -0.9 | 80 | 95 |
| 3 | A | 0.5 | -0.9 | 0.4 | 0.2 | -0.6 | 0.2 | 85 | 91 |
| 4 | B | 0.5 | -0.4 | 0.3 | -0.5 | -0.6 | -0.1 | 91 | 95 |
| 5 | B | 0.5 | -0.9 | 0.2 | 0.1 | -0.2 | -0.2 | 85 | 95 |
| 6 | B | 0,6 | -0,4 | 0,3 | -0,5 | -0,7 | 0,1 | 91 | 95 |

The algorithm used for prediction is as follows:

Get intensities of the 5 markers for the sample from known healthy individuals in the following order: 3980, 5340, 5906, 6880, 28010 Da.

Calculate average intensity of the 5 markers.

Get intensities of the 5 markers of the test sample.
Subtract the average intensity calculated above.

Multiply the first intensity with weight a
Multiply the second intensity with weight b
Multiply the third intensity with weight c
Multiply the fourth intensity with weight d
Multiply the fifth intensity with weight e

Calculate the sum of the above multiplications.

If sum < cutoff value => sample is negative for colon cancer.
If sum > cutoff value => sample is positive for colon cancer.

*Conclusions*

The program found equations, which had sensitivity and specificity above 90 %. The intensity of the marker 5906 is approximately 10 times higher than the other markers. Therefore, in order to prevent the 5906 marker to carry more weight than the other markers it is multiplied by 0.1. The best performing equations were number 1, 4, and 6. This shows that computer algorithms are able to discriminate between healthy individuals and patients with colon cancer. With a larger number of samples it would be possible to use artificial neural network or other computer algorithms to be trained on the data. This might result in increased sensitivity and specificity of the markers.

EXAMPLE 8 IDENTIFICATION OF BIOMARKERS FOR COLORECTAL CANCER IN TISSUE SAMPLES

*Samples*

Tissue samples were obtained from cancer patients after surgery. Tissue samples were obtained from the removed fragment of the patient's colon following surgical treatment for colon cancer and were stored at -80˚C until use.

Sample preparation

100 mg tissue sample was thawed on ice and homogenised on a Wheaton Overhead Stirrer for 2 minutes at speed step 2, in 500 μl Lysis buffer (100mM TRIS-HCl, pH 8.0, 9.5 M UREA, 2% CHAPS). The samples were centrifuged at 14,000 rpm for 10 minutes and the pellet was discarded (repeated twice). The tissue protein extracts were stored at -80˚C until use. Samples were compared by the SELDI-TOF/MS technique (Ciphergen).
Samples were pre-treated by applying 5μl of pre-treatment solution to the chip surface and the chip was left on shaker for 5 minutes. This process was repeated twice. The solution was removed by washing the chip twice in MQ-water and once in binding buffer.

Tissue samples were thawed on ice and 10µl tissue sample was diluted in 50 µl binding buffer and left on shaker for 40 minutes. Next the samples were removed and the chips were washed twice in washing buffer, followed by wash in MQ-water. The chips were left to dry at room temp for 20 minutes and 0.6 µl of crystallisation solution was applied twice.

Analysis

The PBS II instrument (Ciphergen) was calibrated prior to use and chips were analysed with detector sensitivity and laser intensity at suitable values.

Data mining

All spectra were pooled into one experiment file and were normalised based on total ion current. Markers were identified by the Biomarker Wizard software (Ciphergen).

*Description of chips used in tissue screening*

As described, the protein chip surfaces are composed of common chromatographic resins commonly used in other purification techniques:

SAX2 ProteinChip Array

The SAX2 ProteinChip Array is a strong anion exchange array with quaternary amine functionality.

NP20 ProteinChip Array

NP20 ProteinChip Arrays, mimic normal phase chromatography with silicate functionality.

*Description of buffers used for binding and washing steps in the tissue screening*

The buffer solutions used, are common buffers used in other purification techniques:

```
                         SAX2 screening
                         Pre-treatment    : 100 mM TRIS-HCl, pH 8.0
                         Binding step     : 100 mM TRIS-HCl, pH 8.0
                         Washing step     : 100 mM TRIS-HCl, pH 8.0


                         NP20 screening
                         Pre-treatment    : 50 mM TRIS-HCl, pH 8.0
                         Binding step     : 50 mM TRIS-HCl, pH 8.0
                         Washing step     : 50 mM TRIS-HCl, pH 8.0
```

Table 16 Possible tumour markers with the following mass values for each protein chip type

| Chip | Up-regulated | Down-regulated |
|------|--------------|----------------|
| SAX2 | 11987 Da, 5871 Da, 5234 Da, 4281 Da, 4266 Da, 4039 Da, 4024 Da, 3408 Da, 2933 Da, 2878 Da, 2840 Da, 2799 Da, 2693 Da, 2462 Da, 2364 Da | 15140 Da, 11989 Da, 9600 Da, 9197 Da, 9079 Da, 8971 Da, 7324 Da, 5075 Da, 4749 Da, 4634 Da, 3984 Da, 3777 Da, 2330 Da, 1930 Da |
| NP20 | 5857 Da, 4264 Da, 3878 Da, 3712 Da, 3651 Da, 3574 Da, 3487 Da, 3444 Da, 3372 Da, 1688 Da | 9700 Da, 8652 Da, 8652 Da, 8580 Da, 7023 Da, 5360 Da, 4168 Da, 1365 Da, 1256 Da, 1042 Da, 1026 Da, 1005 Da |

Table 16 shows a number of putative markers for colon cancer using more than one type of chip. Although some markers may be detected using different chip with various surface characteristics, most of the markers detected by the different chip types do not overlap. This allows for detection of a larger number of markers in the same sample.

EXAMPLE 9 POSSIBLE TUMOUR MARKERS WITH MOLECULAR WEIGHT CORRESPONDING TO IDENTIFIED BIOMARKERS

The aim of this study was to use bioinformatics to associate the identified markers with annotated genes with a known function.

Many of the possible tumour markers have masses that correspond to specific peptides in the database. The mass values of the individual tumour markers may in some cases correspond to the mass values of specific human proteins in the database. By searching with the mass value of each tumour marker, a number of possible hits occur. These hits are possible identifications of the proteins.

Data bases and search engines
Database :                           Swiss-Prot (Human)
Search tool :                        TagIdent (Expasy)
Allowed deviation: Up to 0.5% deviation from noted mass accepted

Table 17 Possible identification of peptides up-regulated in tumour tissue on SAX2 Chip.

| Biomarker | Entry Name (primary accession number) |
|---|---|
| 2364: | Fragment of human serum albumin/ alpha-fetoprotein (seq: FLGMFLYEYARRHPDYSVV) (SEQ ID NO 1) |
| 2462: | ADML HUMAN (P35318) POLG HRV14 (P03303) REL3 HUMAN (Q8WXF3) |
| 2693: | MOTI HUMAN (P12872) |
| 2799: | HEPC HUMAN (P81172) |
| 2839: | No hits |
| 2878: | No hits |
| 2933: | TERA HUMAN (P55072) |
| 3112: | No hits |
| 3408: | CALO HUMAN (P01258) |
| 4024: | COPA HUMAN (P53621) NEU2 HUMAN (P01185) |
| 4039: | COPA HUMAN (P53621) DEF6 HUMAN (Q01524) NEU2 HUMAN (P01185) PYY HUMAN (P10082) |
| 4266: | NEUY HUMAN (P01303) RFRP HUMAN (Q9HCQ7) |
| 4281: | NEUY HUMAN (P01303) |
| 5234: | CART HUMAN (Q16568) CATB HUMAN (P07858) STAT HUMAN (P02808) |
| 5871: | GAG HV1A2 (P03349) GAG HV1C4 (P05887) GAG HV1J3 (P12494) |

Table 18 Possible identification of peptides down-regulated in tumour tissue on SAX2 chip

| Biomarker | Entry Name (primary accession number) |
|---|---|
| 1930: | No hits |
| 2330: | POLG_HRV16 (Q82122) POLG_HRV89 (P07210) |
| 3777: | APP1_HUMAN (P51693) CAL1_HUMAN (P06881) GLUC_HUMAN (P01275) SARL_HUMAN (000631) |

(continued)

| Biomarker | Entry Name (primary accession number) |
|---|---|
| 3984: | TKN1_HUMAN (P20366) |
| 4634: | CCKN_HUMAN (P06307) |
| 4749: | CRF_HUMAN (P06850) |
| 5075: | CMGA_HUMAN (P10645) CMGA_HUMAN (P10645) TYBN_HUMAN (Q99406) |
| 7324: | POLG_HRV1B (P12916) YU01_HUMAN (Q9BTD5) (+5 other hits) |
| 8971: | >10 hits |
| 9079: | >10 hits |
| 9197: | >10 hits |
| 9600: | >10 hits |
| 15140: | > 10 hits |

Table 19 Possible identification of peptides up-regulated in tumour tissue on NP20 chip

| Biomarker | Entry Name (primary accession number) |
|---|---|
| 1688: | NEUT HUMAN (P30990) S112 HUMAN (P80511) |
| 3372: | CU89 HUMAN (P59042) DEF1 HUMAN (P59665) DEF3 HUMAN (P59666) GLUC HUMAN (P01275) |
| 3444: | DEF1 HUMAN (P59665) (+6 other hits) |
| 3487: | DEF3 HUMAN (P59666) GLUC HUMAN (P01275) NEUY HUMAN (P01303) PTHR HUMAN (P12272) |
| 3574: | OREX HUMAN (043612) PISD HUMAN (Q9UG56) PNOC HUMAN (Q13519) SEM1 HUMAN (P04279) |
| 3651: | CMGA HUMAN (P10645) PRRP HUMAN (P81277) |
| 3712: | A4 HUMAN (P05067) DEF4 HUMAN (P12838) PSPC HUMAN (P11686) |
| 3878: | CCKN HUMAN (P06307) EXXK ADE02 (P03242) GAST HUMAN (P01350) |
| 4264: | NEUY HUMAN (P01303) RFRP HUMAN (Q9HCQ7) |
| 5857: | GAG HV1C4 (P05887) GAG HV1J3 (P12494) PRP2 HUMAN (P02812) |

Table 20 Possible identification of peptides down-regulated in tumour tissue on NP20 chip

| Biomarker | Entry Name (primary accession number) |
|---|---|
| 1005: | GAJU HUMAN (P01358) |
| 1026: | No hits |
| 1042: | No hits |
| 1256: | GON2 HUMAN (043555) |
| 1365: | NPFF HUMAN (015130) PIV6 ADE12 (P35988) |
| 4168: | PAHO HUMAN (P01298) POLN LORDV (P54634) |
| 5360: | COXO HUMAN (P15954) |
| 7023: | RPCX HUMAN (P53803) |

(continued)

| Biomarker | Entry Name (primary accession number) |
|---|---|
| 8580: | >10 hits |
| 8652: | SZ10 HUMAN (P02778) (+5 other hits) |
| 9700: | >10 hits |

*Results and conclusion*

It should be noted that the hits may not necessarily refer to the full length protein encoded by the specified gene, but in many cases to a specific peptide produced by alternative splicing or post-translational processing, hence one mass value may produce more than one hit within one gene.

The results show that some of the markers identified in the examples listed above can be linked to proteins, which have been associated with tumour initiation, tumour growth or tumour progression, such as Def 1 and 3 as well as Cathepsin B.

Furthermore, it should be noted that some of the markers detected by the mass spectrometry might reflect degradation products of larger proteins.

EXAMPLE 10 USE OF DEFINSINS AS SERUM MARKERS FOR CANCER DETECTED BY MASS SPECTROMETRY

SELDI-TOF/MS (Surface Enhanced Laser Desorption/Ionisation-Time Of Flight/Mass Spectrometry) protein profiling was used to demonstrate that the expression of human neutrophil peptides -1, -2 and -3 (HNP 1-3), also known as alfa-defensin-1, -2 and -3, is up-regulated in colon tumour tissue relative to normal colon tissue. Further, by comparing serum from colon cancer patients with serum from a group of healthy individuals, we show that this abnormal HNP 1-3 expression is reflected in colon cancer serum.

The tissue screening was performed on NP20 chip, whereas the serum screening was performed on SAX2 chip.

NP20 ProteinChip Array

NP20 ProteinChip Arrays, mimic normal phase chromatography with silicate functionality.
| | |
|---|---|
| Pre-treatment : | 50 mM TRIS-HCl, pH 8.0 |
| Binding step : | 50 mM TRIS-HCl, pH 8.0 |
| Washing step : | 50 mM TRIS-HCl, pH 8.0 |

SAX2 ProteinChip Array

The SAX2 ProteinChip Array is a strong anion exchange array with quaternary amine functionality.
| | |
|---|---|
| Pre-treatment : | 100 mM TRIS-HCl, pH 8.0 |
| Binding step : | 100 mM TRIS-HCl, pH 8.0 |
| Washing step : | 100 mM TRIS-HCl, pH 8.0 |

The Defensin screening was performed by as described for the general serum / tissue screenings. The expression of three peptides with mass/charge ratio (m/z) values of 3372, 3443 and 3486 (+/- 0.1%) were found to be up-regulated in the tumour samples compared to the samples and up-regulated in serum from patients with colon cancer when compared with serum from healthy individual. The three peptides were subsequently identified as HNP 2, 1 and 3, respectively. This was done by peptide mapping (trypsin digest) and reduction with DTT.

EXAMPLE 11 EXPRESSION OF ALFA-DEFENSIN-1, -2 AND -3 IN SERUM AND TISSUE OF COLON CANCER PATIENTS

The aim of this study was to define the relationship of the expression of human neutrophil peptides -1, -2 and -3 (HNP 1 -3) and colon cancer.

*Materials and methods*

Tissue screening

Tissue samples were obtained from the removed fragment of the patient's colon following surgical treatment for colon cancer and were stored at -80˚C until use. 100 mg tissue sample was thawed on ice and homogenised on a Wheaton Overhead Stirrer for 2 minutes at speed step 2, in 500 μl Lysis buffer (100mM TRIS-HCl, pH 8.0, 9.5 M UREA, 2% CHAPS). The samples were centrifuged at 14,000 rpm for 10 minutes and the pellet was discarded (repeated twice). The tissue protein extracts were stored at 80˚C until use. Minor pilot studies were performed on different chips (data not shown) and the NP20 (Normal Phase) (Ciphergen) chip was chosen for the tissue screening. NP20 chips was placed in bioprocessor and pre-treated with 50 μl tissue binding buffer (50 mM TRIS-HCl, pH 8.0) for 5 minutes on shaker (250 rpm) (repeated twice). 5 μl tissue protein extract was diluted in 50 μl tissue binding buffer and incubated in bioprocessor on NP20 chips for 40 minutes at room temperature on shaker (250 rpm). Spots were washed twice in 250 μl tissue washing buffer (50 mM TRIS-HCl, pH 8.0) for 5 minutes. The chips were air-dried for 20 minutes, followed by treatment with two times 0.6 μl 100% SPA matrix solution.

Serum screening

Cancer serum samples were obtained from cancer patients prior to surgery. Normal serum was obtained from a group of healthy individuals matched by age and gender to the cancer patients. Serum samples were stored at -80˚C until use. Serum pilot studies were performed on different chips to monitor the presence of HNP 1-3 in serum (data not shown). The immobilised metal affinity capture (iMAC30) chip was chosen for the actual screening and pre-treated with nickel before analysis: 5 μl 100 mM NiSO4 were added to each spot and left on shaker (150 rpm) for 5 minutes (repeated twice). The chips were placed in bioprocessor and incubated with 100μl MQ for 5 minutes on shaker (250 rpm). Each spot was treated with 50 μl serum binding buffer (100 mM TRIS-HCl, pH 7.5, 500 mM NaCl, 0,1% Triton X-100) and left on shaker for 5 minutes (250 rpm). Serum samples were thawed on ice and 5 μl serum was diluted in 50 μl serum binding buffer and applied to spots and left on shaker (250 rpm) at room temperature for 40 minutes. Samples were removed and spots washed twice in 200 μl serum washing buffer (100 mM PBS, pH 7.4, 700 mM NaCl), followed by one wash in 200 μl MQ-water. The chips were removed from the bioprocessor and left to air dry for 20 minutes followed by treatment with two times 0.6 μl SPA (100%). Only freshly made matrix solutions were used and the instrument was calibrated prior to use. Cancer and normal samples were run side by side. The chips were analysed on a PBSII instrument (Ciphergen). All spectra in each screening were normalised based on total ion current.

Purification and identification of HNP 1-3

100 μl protein extract from cancer tissue in tissue lysis buffer was loaded unto a RP-HPLC column (uRPC C2/C18 ST 4.6/100, Pharmacia Biotech, Flow rate: 0.5 ml/min, Fraction size: 0.5 ml) in buffer A (0.065% Tri-flouro-aceticacid (TFA) in MQ-water) and proteins were eluted in a gradient of 0-100% buffer B (0.05% TFA in acetonitrile (ACN)). Elution of peptides was monitored by absorption spectrometry (OD280). All protein containing fractions were analysed by MALDI-TOF (Matrix Assisted Laser Desorption/Ionization-Time of flight) on the PBS II instrument: 1.5 μl fraction was incubated with 0.6 μl SPA (100%) on a Gold array (Ciphergen) and left to crystallise on chip, followed by an additional 0.6 μl SPA (100%) and the Gold array was analysed by MALDI-TOF. The HNP 1-3 containing fraction (32% buffer B) was further purified on a peptide gel-filtration column (Superdex Peptide HR 10/30, Pharmacia Biotech, Flow rate 0.9 ml/min, Fraction size: 0.5 ml, Buffer: 50% ACN, 0.1 % TFA). Elution of peptides was monitored by absorption spectrometry (OD280) and protein containing fractions were again analysed by MALDI-TOF on the PBS II instrument as described. Purified HNP 1-3 was subjected to on-chip trypsin digestion. 10 μl HNP 1-3 fraction was applied to NP20-chip and left on shaker (250 rpm) at room temperature for 40 minutes. Sample was removed and spot was washed twice with 10 μl water (on-chip purification step). In order to denature peptides prior to digestion, the chip was left on heating block (80 C) for 5 minutes. The chip was cooled on ice for 2 minutes. 10 μl trypsin digestion solution (0.01μg/μl trypsin in 50 mM NH4HCO3, pH 8.0) was added, and the chip was left for 10 hours at 40˚C in humidity chamber after which the chip was left to air dry for 20 minutes. 1 μl CHCA (100%) was added and the peptide map was analysed on PBS II instrument. Identification was done by the use of PepIdent on the Expasy server.

Size exclusion chromatography of HNP 1-3

50 um colon cancer serum was loaded unto a peptide gel-filtration column (optimal separation range: 1 to 7 kDa, flow rate: 0.5 ml/min, fraction size: 0.5 ml, buffer: 10 mM Ammonium carbonate, pH: 8.0). Elution of peptides was followed by absorption spectrometry (OD280). All protein-containing fractions were analysed by MALDI-TOF on PBSII (Ciphergen) as described above. Maximum signal intensity of 40 individual peaks was plotted as a function of elution volume and an approximate elution curve was calculated.

Functional study of HNP 1-3 by microflow

For micro flow experiments, MDCK cells were plated onto poly-d-lysine coated cover slips at a concentration 3000cells/well, grown in DMEM with 10% FBS for five days with the result of confluent islands. Microflow was performed in an Eppendorf micromanipulator 5171 and transjector 5246 system mounted on a Leica DMIRBE inverted research microscope. Micro capillaries (borosilicate with filament, Sutter Instruments Company, Novato, California, USA) were pulled to an outer diameter of 0.85 nm on a Sutter P-97 Micropipette Puller. The dye-loaded cells were visualised by excitation at 470 nm and recorded at 509-nm emission using Haupage version 3.3.18038 software and Kappa CF 15/4 MC-S camera (Leica). The MDCK cells were recorded (in $CO_2$ independent media) on the inverted DMIRBE inverted research microscope. The capillary was placed 20 nm over the confluent cells with a constant flow (1300 hPa) of calcein (20mM). The MDCK cells were exposed to peptide fractions purified from colon tumours by size-exclusion chromatography. *Results*

HNP1-3 expression in tissue and serum

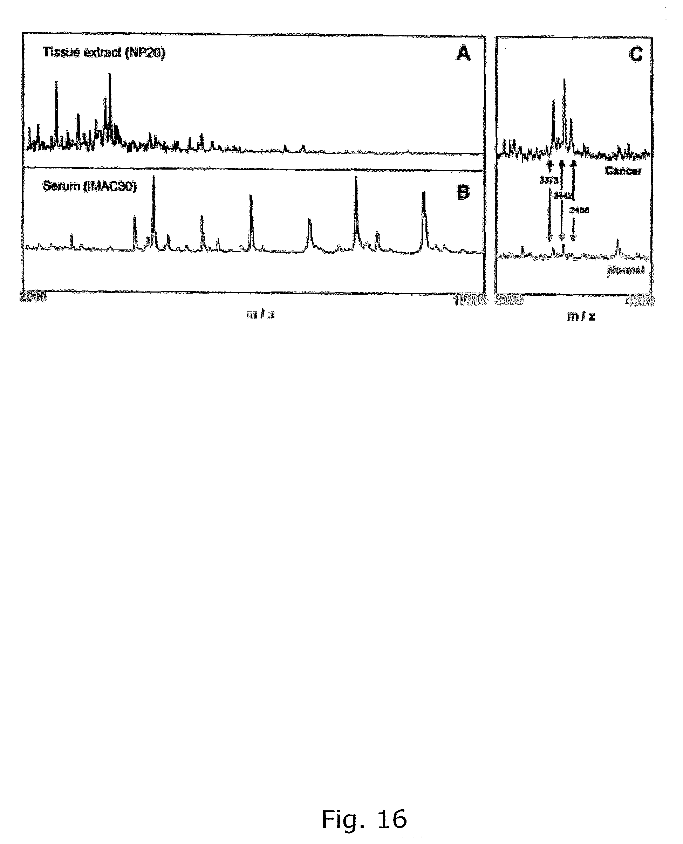

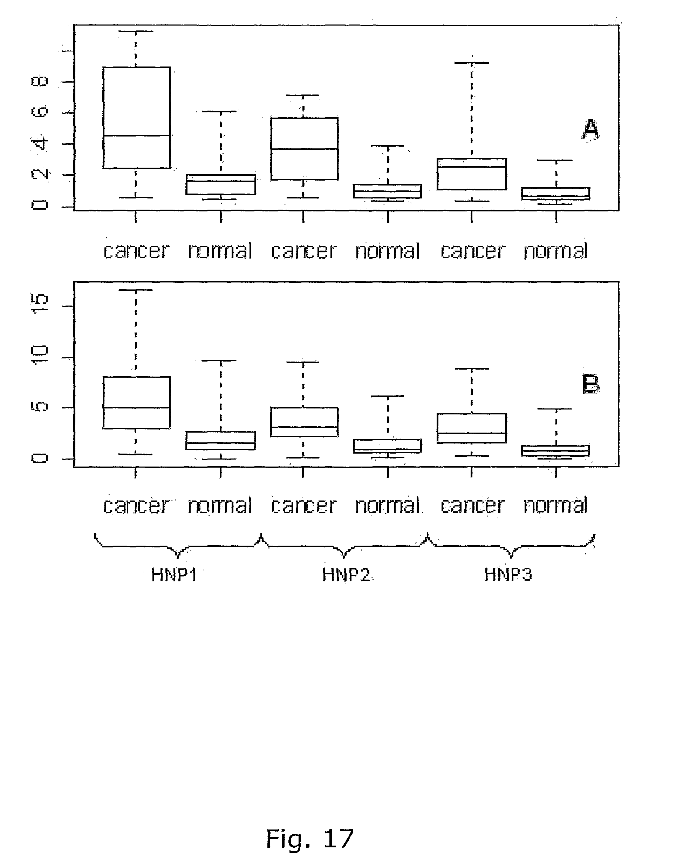

Pilot studies of colon tumour and normal colon tissue was performed on a variety of chips with different chemical properties and under different binding and washing conditions. Based on these preliminary studies, the expression of three peptides with mass/charge ratio (m/z) values of 3372, 3443 and 3486 (+/- 0.1%) (subsequently identified as HNP 2, 1 and 3, respectively), were found to be up-regulated in the tumour samples. The three peptides were visible on different chips and under different binding conditions (data not shown). However the strongest signals of HNP 1-3 in tissue extract were obtained on the NP20 (Normal Phase) chip, whereas the strongest signal of HNP 1-3 in serum was observed on the iMAC30 (immobilised metal affinity capture) chip activated with nickel, and these conditions were chosen for the actual screenings. Protein extract from 40 colon tumour and 40 normal colon tissue samples were analysed on NP20 chips and 125 colon cancer serum samples and 100 normal serum samples were analysed on iMAC30 chips. All spectra in each screening were pooled and normalised based on overall ion current. Each spectrum produced approximately 40 to 60 protein peaks in the range from 2 to 80 kDa (FIG. 16A-C). Statistical analysis of the intensity values of HNP 1-3 in the tissue screening (FIG. 17A showed) that HNP 1-3 were significantly up-regulated in tumours ($p < 0.0005$). Similarly, statistical analysis of HNP 1-3 expression in the serum screening (FIG. 17B.) showed that HNP 1-3 were significantly up-regulated in cancer serum also ($p < 2.2e\text{-}16$). Compared to other peptides in the same range, HNP 1-3 showed average signal intensity in most normal colon tissue extract, whereas the HNP 1-3 signal was extremely high in most tumour samples (in some tumour samples the HNP 1-3 was the most prominent of all detected peptides). On the contrary, in the normal serum samples the HNP 1-3 signals were relatively low, and only slightly, but still significantly, higher in the cancer serum. This difference between the HNP 1-3 signal in the tissue screening performed on the NP20 chip and serum screening performed on the iMAC30 chip was not due to the different chips used in the screenings, since the HNP 1-3 signal in serum was relatively low on the NP20 chip also (data not shown). Thus, even though most tissue samples were "contaminated" with blood, the vast majority of the HNP 1-3 signal originated from the tumour microenvironment. This was verified by gel-filtration analysis of tissue extract versus serum. HNP containing fractions from tissue analysis were far more concentrated (approximately x10) than the same fractions in serum analysis, as seen by MALDI-TOF analysis (data not shown).

Identification of HNP 1-3

The three possible markers were purified by RP-HPLC, peptide gel-filtration and on-chip purification, after which they were identified by peptide mapping as HNP-2 (3372 Da), HNP-1 (3442 Da) and HNP-3 (3486 Da) (Table 1A.). The measured masses correspond to the peptides in their oxidised states, with three disulphide bridges. After heat denaturation (10 minutes, 80˚C) and treatment with DTT (200mM DTT, room temperature, 30 minutes), HNP-1 and HNP-2 increased 6 Dalton in mass, due to reduction of the six cysteines (Table 1B). We were not able to reduce HNP-3, due to degradation during the reduction process.

Size exclusion chromatography of HNP 1-3

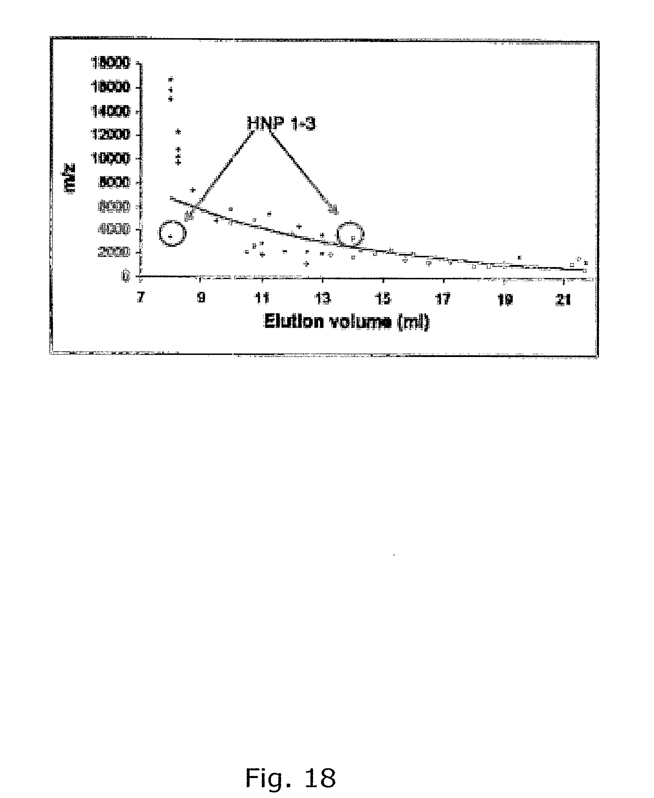

50 $\mu$l colon tumour extract in Lysis buffer was applied to a peptide gel-filtration column. Elution of peptides was followed by absorption spectrometry (OD280). All fractions were analysed by MALDI-TOF on PBSII (Ciphergen). Maximum signal intensity of 40 individual peaks was plotted as a function of elution volume and an approximate elution curve was calculated (FIG. 18). HNP 1-3 peptides were found to be eluted primarily together with high mass proteins above 20 kDa and to a lesser degree in fractions together with other peptides of the same mass interval (FIG. 18).

Cytoxic assay

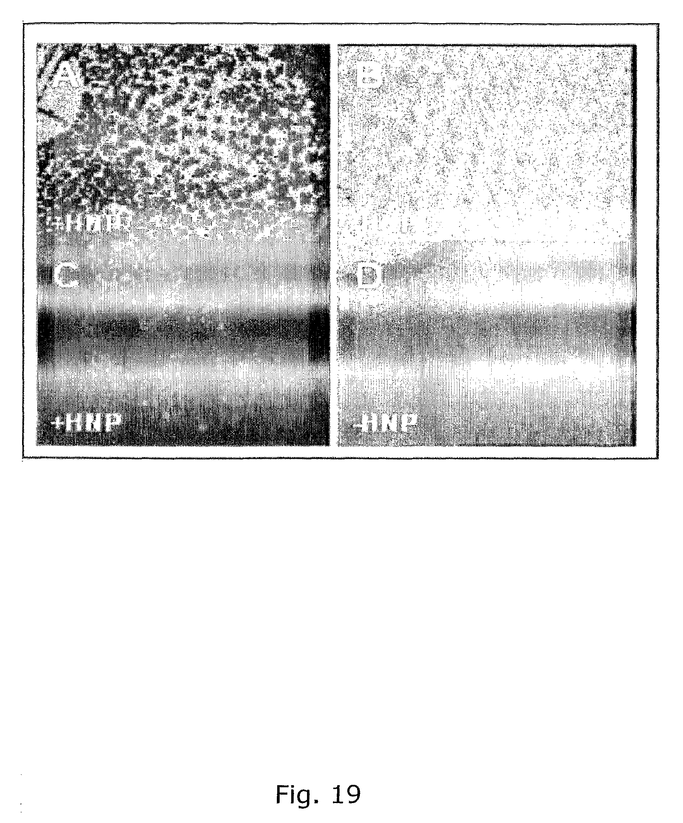

The cytotoxicity of HNP 1-3 purified from colon tumours was tested by exposing MDCK cells to different fractions purified from colon tumours. Calcein were added to the fractions and the solutions were left to overflow the cells for one hour. By fluorescence microscopy calcein was observed to accumulate only in cells exposed to HNP 1-3/calcein fractions, whereas cells treated with fractions containing other (unidentified) tumour peptides did not uptake calcein (FIG. 19 C&D). Further, by normal microscopy, we observed that cells exposed to HNP 1-3 appeared more diffuse and had enlarged nuclei, indicating apoptosis (FIG. 19 A&B).

*Discussion*

Elevated concentrations of HNP 1-3 in colon cancer serum

Abnormal concentration of HNP 1-3 in body fluids has previously been demonstrated. Elevated concentrations of HNP 1-3 following infection (bacterial-/non-bacterial- infection and pulmonary tuberculosis) has been found in plasma, blood and a number of body fluids and plasma HNP 1-3 concentrations have been shown to be elevated in patients with septicaemia or bacterial meningitis. HNP 1-3 have been found in urine from patients with transitional cell carcinoma of the bladder and in salvia of patients with oral carcinomas.
Our study is the first that demonstrate elevated concentrations of HNP 1-3 in serum following tumour growth.

Elevated concentrations of HNP 1-3 in colon tumours

HNP expression has previously been linked to different types of tumours and cell lines.
HNP-1 has been detected in lung tumours and in the submandibular glands of patients with oral carcinomas. By RT-PCR, mass spectrometry and flow cytometric analysis, HNP 1-3 have been shown to be expressed by cell lines deriving from renal cell carcinomas and the expression of a specific HNP precursor peptide has been shown to be up-regulated in human leukaemia cells. In a study of squamous cell carcinomas of the human tongue it was suggested that the tumour expressed HNP 1-3 originated from tumour invading neutrophils. Since our tissue screening is based on comparison of whole tissue samples, the up-regulated expression of HNP 1-3 may not necessarily originate from the colon cancer cells, but could originate from tumour infiltrating neutrophils. HNP 1-3 are known to stimulate bronchial epithelial cells to up-regulate interleukin-8 production, a potent neutrophil chemotactic factor and HNP 1-3 are also capable of regulating the systemic immune response (discussed below). Thus, the up-regulated expression of HNP 1-3 in colon tumours may primarily originate from invading neutrophils, but could be initiated by HNP 1-3 produced by cancer cells. Even though the signal intensity in mass-spectrometry can not directly be interpreted as a measure of protein concentration, our results suggests that HNP 1-3 are very abundant in colon tumours. This is in agreement with the study of HNP-1 in lung tumours, where the maximum observed level was 26 nano-moles per gram wet tissue. It follows, that in order for these excessive amounts of peptide to be detectable in serum, the peptides must be released from the cells. This is in agreement with studies of HNP 1-3 expression in kidney and brain.

Size exclusion chromatography of HNP 1-3

We explain the elevated concentrations of HNP 1-3 in colon cancer serum by unspecific binding between HNP 1-3 and high mass serum proteins. We believe the peptides attach to serum proteins in the tumour area and are carried into the bloodstream. Even though the HNP 1-3 we observe in high mass fractions from size exclusion, could also be explained by multimerisation, we interpret the size exclusion results as evidence for interaction between HNP 1-3 and unidentified high mass proteins through unspecific interactions. In one study, it was demonstrated that Defensins form voltage dependent channels in lipid bi-layer membranes, supported by further conductance investigations, suggested that the channels were formed by multimers containing 2-4 molecules and a crystal structure study of HNP-3 revealed an amphiphilic dimer. We add to the growing realisation that common plasma proteins bind disease specific peptides and therefore should not be ignored in marker research. Our size-exclusion results are in agreement with a number of previous studies that show that HNP's are bound to plasma protein in vitro and that high concentrations of HNP's causes precipitation of plasma proteins, specifically 2-macroglubulin and C1 complement has been shown to bind Defensin. Another study showed that HNP-1 bind to various serum proteins, notably serum albumin, and it was found that serum, or serum albumin, was able to inhibit the anti-viral activity of HNP-1. This ability to bind to serum proteins could also explain why HNP 1-3 lysis of mammalian cells is hindered in the presence of serum.
Common to beta-Defensin 2, another member of the Defensin family, and HNP 1-3 is an uneven distribution of surface charges. Beta-Defensin 2 has been shown to bind to a chemokine receptor and it has been suggested that the positively charged cluster, which is also shared by chemokines, may play a common role in binding to receptors in general, but is not important for determining receptor specificity. The same surface charge could also explain the binding of HNP 1-3 to plasma proteins. The observation that Defensins are localised to lymphocyte nuclei could similarly be explained by unspecific binding to shuttle proteins.

HNP 1-3 - cytotoxic peptides

The exact concentration of HNP's in the tumour microenvironment may have profound influence on the in vivo function of HNP 1-3. One study shows that HNP 1-3 mediates lysis of tumours in a concentration dependent manner. This is in agreement with another study that show that only relatively high concentrations of HNP-1 (10-4 M) are cytotoxic for human monocytes, whereas lower concentration of HNP-1 (10-8 to 10-9 M) increases TNF-alpha production by monocytes. In a study of renal cell carcinoma lines it was shown that HNP 1-3 were cytotoxic to all tested cell lines when present in high concentrations (above 25 ug/ml), but at lower concentration HNP 1-3 stimulated growth of a subset of tumour cell lines. We add to the established theory that HNP 1-3 are cytotoxic to mammalian cells, by demonstrating that HNP 1-3 purified from colon tumours are capable of lysing MDCK cells. Our study was based on a 30 minutes microflow study and did not allow us to investigate the minimum concentration of HNP 1-3 necessary for lysis.

*Conclusion*

The high concentration of HNP 1-3 observed in tumours and the observation that HNP 1-3 are capable of lysing mammalian cells leads to the immediate conclusion that the peptides serve to the benefit of the host by primarily killing tumour cells. However, HNP 1-3 bind to HLA-Class II molecules and are capable of reducing the proliferation of a HLA-DR-restricted T-cell line after stimulation and could in this way help the tumour avoid immune recognition. Defensins also regulate the systemic immune response. Through interaction with the chemokine receptor CCR6, beta-Defensins recruit dendritic cells and T cells and HNP 1-3 are capable of recruiting leukocytes to sites of infection in mice. Up-regulated immune responses are known to stimulate tumour proliferation: immune cells are actively recruited by tumours to exploit their pro-angiogenic and pro-metastatic effects. Whether the high concentrations of HNP 1-3 in the tumour limits the tumour growth or on the contrary stimulate tumour proliferation is not clarified. Recently, it was found that the excess amounts of HNP 1-3 observed in urine from bladder cancer patients was produced by the actual bladder cancer cells, (and not by tumour infiltrating neutrophils), and that highly invasive bladder cancer cells produced more HNP 1-3 than less invasive ones. We suggest that the prominent surface charge on Defensins, their ability to bind to high mass proteins and the observed excess amounts of peptides seen in tumours, could provide the peptides with broad antagonising effects, that may influence numerous receptors in the tumour microenvironment.

EXAMPLE 12 IDENTIFICATION OF BIOMARKERS FOR COLORECTAL CANCER BY PLASMA INVESTIGATIONS

The plasma screening was performed on IMAC30 chips according to the protocol used for serum screening described above on IMAC30 chips, with the exception of adding 5 µl plasma instead of 5 µl serum to the binding buffer.

Table 21 Possible tumour markers with the following mass values from plasma samples of colon cancer patients

| Up-regulated in cancer plasma (m/z) | Down-regulated in cancer plasma (m/z) |
| --- | --- |
| 3895 | 6435 |
| 4136 | 6635 |
| 4480 | 8931 |
| 4977 | 64860 |
| 5266 | 66300 |
| 5905 | 66500 |
| 7469 | 66800 |
| 9950 | |
| 11723 | |
| 13747 | |

(continued)

| Up-regulated in cancer plasma (m/z) | Down-regulated in cancer plasma (m/z) |
|---|---|
| 13870 | |
| 14030 | |
| 14100 | |
| 14300 | |
| 14470 | |
| 19865 | |
| 19966 | |
| 60475 | |
| 60730 | |

EXAMPLE 13 IDENTIFICATION OF BIOMARKERS FOR COLON CANCER BY DATA MINING OF MASS SPECTRA FROM PLASMA

The aim of this study was to separate healthy individuals from colorectal cancer patients using a Principal Component Analysis (PCA) on a normalised data set from mass spectra.

*Methods*

Samples

Plasma samples were obtained from 16 healthy individuals and 16 patients diagnosed with colon cancer and the samples were analysed on IMAC30 chips according to the protocol described above in Example 12.

Data

Two data sets containing m/z and intensity of the peaks identified by "biomarker wizard" were generated. The first data set contained half of the spectra. The second data set contained all spectra. Spectra were pooled and normalised based on total ion current in the two data sets.

Computer programs:

Ciphergen ProteinChip Software with "biomarker wizard".
Multi Variate Statistical Program (MVSP), Kovack Computing.

*Parameters*

Biomarker wizard settings:

First pass: 5
Min peak threshold: 0
Cluster mass window: 0.3
Second pass: 2

Principal Component Analysis settings (MVSP):

Data standardised: Yes
Data centred: Yes

*Results:*

Potential markers from a principal component analysis of the first data set: 1455, 1500, 1532, 1573, 1704, 1725, 3445, 3545, 3895, 4136, 4480, 4977, 5266, 5910, 6110, 6435, 6635, 6673, 8931, 9015, 9173, 9950, 10838, 11723, 13747, 13870, 19865, 28028, 32490, 33233, 50820, 60638, 65706, 66213, and 79155 Da.
The following combinations of markers yielded 100 % sensitivity and 100 % specificity:
3895, 6110, 8931, and 6635 Da.
6110, 8931, and 6635 Da.
19865, 13747, 8931, and 9015 Da.
8931, 9015, 33233, and 13747 Da.
19865, 13747, 8931, 9015, and 33233 Da.
Principal component analysis on the second data set yielded the following potential markers: 1573, 1704, 1725, 6435, 6673, 9015, 9173, 10838, 11341, 11723, 13747, 13880, 28028, and 50825 Da.
The most prominent combination of markers was the following: 9173, 11728, and 13880 Da with 100 % specificity and 100 % sensitivity.

*Conclusion:*

Principal Component Analysis can separate healthy individuals from patients with colon cancer using the intensity of the selected markers.
As presented in Example 9, a peptide of mass 2364 is up-regulated in tumour tissue when analysed on SAX2 Chip (table 17, line 1). This peptide was purified (by RP-HPLC and peptide-gel-filtration) and subsequently identified by ESI-MS/MS. The peptide was found to consist of the following sequence: FLGMFLYEYARRHPDYSW (m/z 2363.7) SEQ ID NO 1. This sequence corresponds to a fragment of human serum albumin, demonstrating that human serum albumin is excessively degraded in colon tumour samples compared to normal colon tissue samples and thus supports the results that show that there is an abnormal degradation of serum albumin in serum from cancer patients

EXAMPLE 14 PROTEOLYTIC DEGRATION OF COMMON BLOOD PROTEINS AS A MARKER FOR CANCER

Abnormal protease activity in colon cancer serum

When serum is analysed on the IMAC30 chip (as described in the procedure for the serum screening) two high mass proteins are found to be differentially expressed (as described in the results of the serum screening): a protein with m/z: 66500 is down-regulated in cancer serum whereas a protein with m/z: 60500 is up-regulated in cancer serum (see table 10).
The protein of 66500 is human serum albumin (HSA) (ALBU_HUMAN (P02768))
The theoretical mass of HSA is 66472 Da, well within 0.1% of the observed mass of 66500 Da. The peak at 66500 is an easily identifiable and prominent peak of high intensity, often observed in mass spectrometry analysis of biological samples and any person familiar with mass spectrometry would immediately identify the prominent peak at 66500 as serum albumin.
Therefore, we show that HSA is present in lower amounts in serum from cancer patients than in serum from normal individuals.
The protein at 60500 appears in a reverse proportional manner to HSA: in the normal serum where there is high amounts of HSA, there is only little amount of 60500, and in the cancer serum where there is relatively low amounts of HSA, there is relatively high amount of 60500.
From this we conclude that 60500 is a degradation product of HSA, that is produced when a fragment of approximately 6000 Da is lost from HSA.
HSA is produced in the liver which is not influenced by tumour growth in the colon, at least not at this stage in the disease, and the observation, that there is relatively more HSA in serum from normal individuals than in serum from cancer patients, can therefore not be explained by an altered expression of HSA by liver cells. The only meaningful explanation for this abnormality is altered proteolytic degradation of HSA in serum from cancer patients. Since the proteolytic product, in this case the HSA fragment at 60500, is also present in serum from normal individuals, albeit at lower amounts than in serum from cancer patients, the exact proteolytic mechanism responsible for the specific degradation of HSA leading to the production of 60500 is not unique to serum from cancer patients.
Therefore, our results show direct evidence for altered proteolytic activity in cancer serum.
Finally, as presented in Example 9, a peptide of mass 2364 is up-regulated in tumor tissue when analysed on SAX2 Chip (table 17, line 1). This peptide was purified (by RP-HPLC and peptide-gelfiltration) and subsequently identified by ESI-MS/MS (as described in example 15). The peptide was found to consist of the following sequence:FLGMFLYEYARRHPDYSVV (m/z 2363.7). This sequence corresponds to a fragment of human serum albumin, demonstrating that human serum albumin is excessively degraded in colon tumor samples compared to normal colon tissue samples. This supports the results that show that there is an abnorm degradation of serum albumin in serum from cancer patients.

Identification of serum/plasma marker 28040/28025/28010

By HPLC, gel purification and trypsin peptide mapping we positively identify 28040/28025/28010 as apolipoprotein (P02647).

*Results:*

Best mathch:

| Score | # peptide matches | AC | ID | Description | pI | Mw |
|---|---|---|---|---|---|---|
| 0.43 | 3 | P02647 | APA1_HUMAN_1 | CHAIN 1: Apolipoprotein A-I. - Homo sapiens (Human). | 5.27 | 28078.62 |

Peptide map:

| user mass | matching mass | Δmass (Dalton) | #MC | modification | position | peptide |
|---|---|---|---|---|---|---|
| 1301.6 | 1301.4216 | -0.1783 | 0 | | 185-195 | THLAPYSDELR (SEQ ID NO 2) |
| 1301.6 | 1302.4681 | 0.8681 | 1 | | 165-175 | LSPLGEEMRDR (SEQ ID NO 3) |
| 1723.87 | 1723.9499 | 0.0799 | 2 | | 141-155 | QKVEPLRAELQEGAR (SEQ ID NO 4) |
| 3032.97 | 3033.3418 | 0.3718 | 2 | | 37-64 | DLATVYVDVLKDSGR DYVSQFEGSALGK(SEQ ID NO 5) |

Apolipoprotein information:

*Function:* Participates in the reverse transport of cholesterol from tissues to the liver for excretion by promoting cholesterol efflux from tissues and by acting as a cofactor for the lecithin cholesterol acyltransferase (LCAT).

*Subcellular location:* Secreted.

*Tissue specificity:* Major protein of plasma HDL, also found in chylomicrons. Synthesized in the liver and small intestine.

As discussed above, abnormal concentrations of common plasma/serum proteins produced by the liver will probably not be due to altered transcription/translation of the relevant gene, but instead a consequence of abnormal proteolytic activity.

EXAMPLE 15 IDENTIFICATION OF PEPTIDES FROM COLON CANCER MARKERS

The purpose of this project is to identify a number of peptides which have been found in blood serum and which are identified as markers for colon cancer.

*Analysis*

Two samples were purified, wherein one sample contained two peaks. Each sample was initially analysed by MALDI-TOF to establish the molecular weight of the components and to have an estimate on the amount of peptide present in the sample.

The peptides of interest, found during MALDI analysis, were fragmented by both MALDI-TOF/TOF and ESI-MS/MS analysis.

Sample 1 (containing the 5901 Da peptide) was purified by reversed phase HPLC and each fraction was analysed by MALDI-TOF to locate the fractions containing the 5901 Da peptide. The fractions containing the peptide were pooled and analysed both directly by MS/MS analysis and further purified by 1D SDS gel electrophoresis. The band at 6000 Da was cut out, digested with trypsin and analysed by MALDI-TOF and TOF/TOF.

*Instruments*

Bruker Reflex IV (MALDI-TOF)
Bruker Ultraflex (MALDI-TOF/TOF)
Micromass Ultima (nanoLC-MS/MS)
Applied Biosystems Vision Workstation (HPLC)

*Results*

Human serum sample (300 µl) was purified by reversed phase HPLC. The three fractions containing the 5900 Da peptide were pooled and analysed by MALDI-TOF. The final fraction contains 4 major peaks; MH$^+$ at 4961.8 Da, 5333.5 Da, 5901.1 Da and 6187.05 Da.

The pooled fractions were dried down and loaded on a SDS PAGE gel. The gel band of interest was cut out of the gel, reduced and alkylated, and digested with trypsin.

The digest sample was micro-purified over a graphite/carbon column. A peptide fingerprint was made. One peptide (MH$^+$ 1190.5) was selected for MALDI-TOF/TOF analysis. Database search of the fragmented peptide gave a Mascot search score of 69 and an ion score of 47. The peptide is part of alpha-fibrinogen.

The sequence from gi|1706799|sp|P02671 was used to search for the masses found in the pooled fraction. The m/z 5901.9 Da peptide can be a part of alpha-fibrinogen, and the tryptic peptide (MH$^+$ 1190,5) can be included in the m/z 5901.9 Da peptide. The sequence is:

*Peptide Sequences*

*5901.9 Peptide*
SSSYSK**QFTSSTSYNR**GDSTFESKSYKMADEAGSEADHEGTHSTKRG HAKSRPV (SEQ ID NO 6)
The bold underlined part of the sequence shows the tryptic peptide (MH$^+$ 1190.5 Da).
The tryptic peptide does unfortunately also fit to the masses 5333.5 and 6187.05 Da found in the fraction.
*5333.5 Peptide:*
GIFTNTKESSSHHPGIAEFPSRGKSSSYSK**OFTSSTSYNR**GDSTFESKS (SEQ ID NO 7)
or
SGIFTNTKESSSHHPGIAEFPSRGKSSSYSK**OFTSSTSYNR**GDSTFESK (SEQ ID NO 8)
*6187.05 Peptide*
GSESGIFTNTKESSSHHPGIAEFPSRGKSSSYSK**OFTSSTSYNR**GDSTF ESKSYKMA (SEQ ID NO 9)

*Conclusion*

One peptide was found after digest of the gel band containing the "5900 Da peptide". Fragmentation of the peptide (MH$^+$ 1190.5) by MALDI-TOF/TOF gave the sequence (QFTSSTSYNR). This is part of alpha-fibrinogen. Searching the alpha-fibrinogen sequence for the mass m/z 5901.9 gave a hit where the sequence from the tryptic peptide also is included. The sequence does also fit to the masses 5333.5 and 6187.05, respectively.

Sample 2.

*Results:*

From the MALDI-TOF spectra, the peaks m/z 2363.05 and 1686.84 Da were found to corresponds to the masses from the SELDI approach.
It was only possible to make ESI-MS/MS on 2363.05. The peptide was seen a triply charged ion. Attempts were also made to make MALDI-TOF/TOF on these peaks, but without success.

Peptide Sequences

De-Novo sequencing gave the tag: **FLGMFLYE** (SEQ ID NO 10). This was searched as a sequence tag together with the mass MH $^{3+}$ 788.3 Da. This matched the peptide **FLGMFLYEYARRHPDYSVV** (SEQ ID NO 11).

A similarity search of the sequence shown in table 22, resulted in the following hit:

Table 22 Similarity search for the 6187.05 peptide

| Hit number | Annotation |
| --- | --- |
| 1 | (AF116645) PRO1708 [Homo sapiens] |
| 2 | (AF119890) PRO2675 [Homo sapiens] |
| 3 | Similar to alpha-fetoprotein [Homo sapiens] |
| 5 | (AF130077) PRO2619 [Homo sapiens] |
| 6 | Human Serum Albumin In A Complex |

*Conclusion*

Direct analysis of the sample with MALDI-TOF showed the peptides of interest. ESI-MS/MS was only possible on mass MH$^{3+}$788.3 Da. This matched the peptide:

FLGMFLYEYARRHPDYSVV. This can be a part of alpha-fetoprotein/human serum albumin.

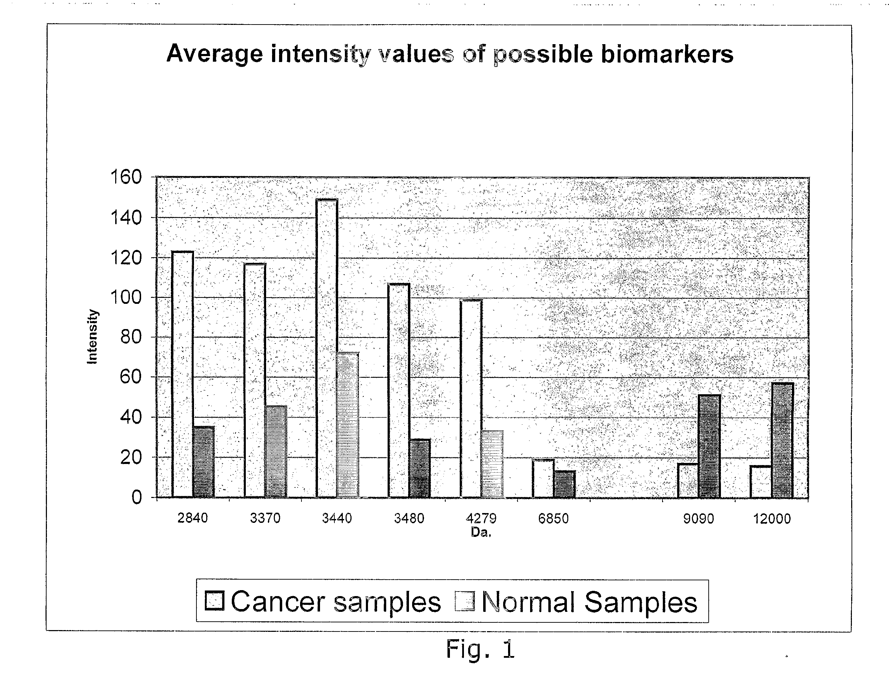

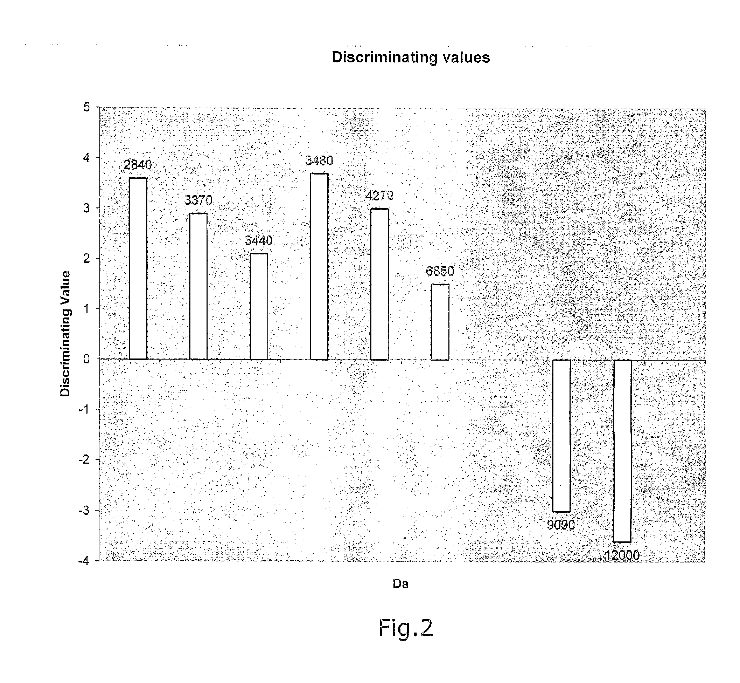

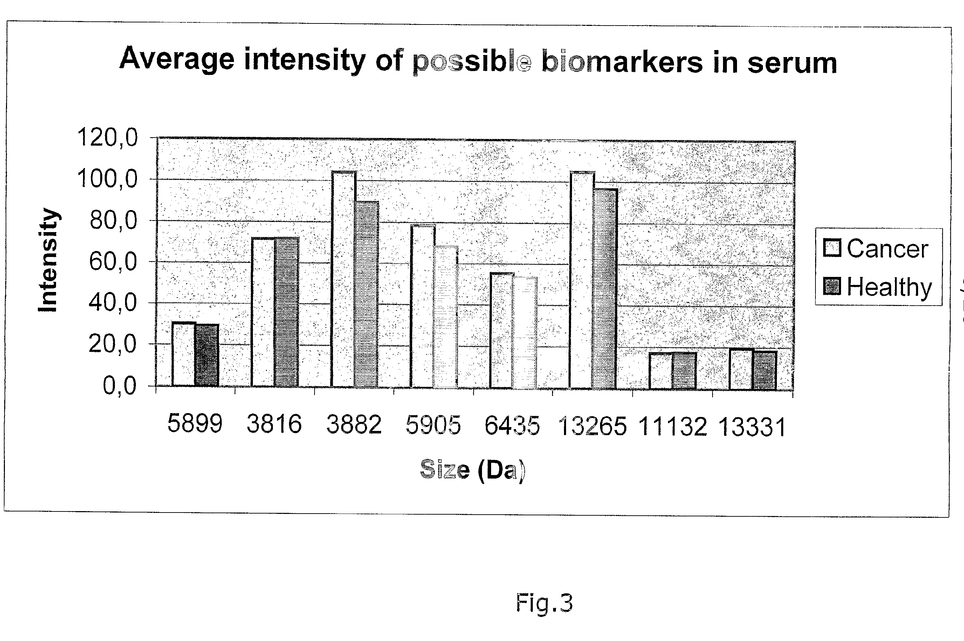

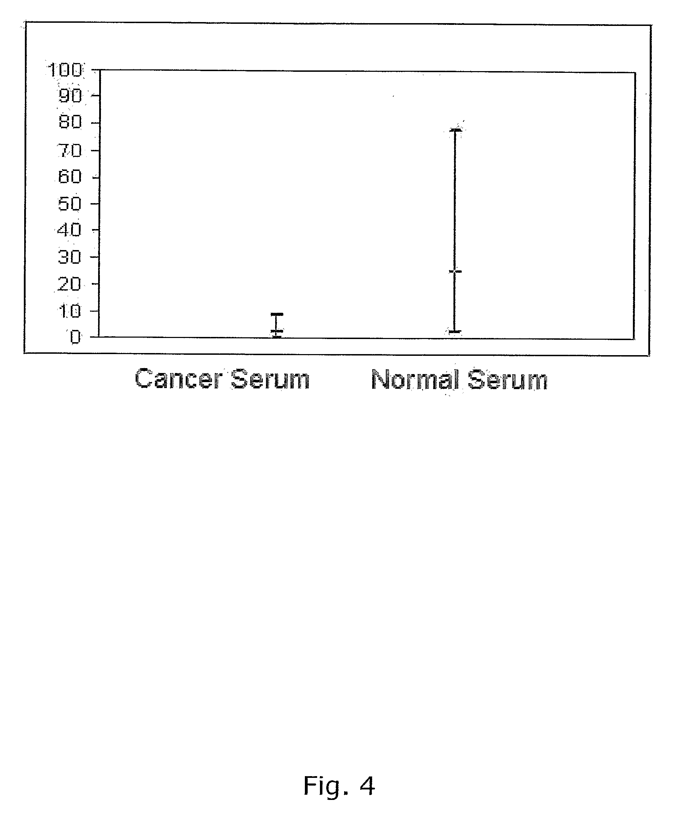

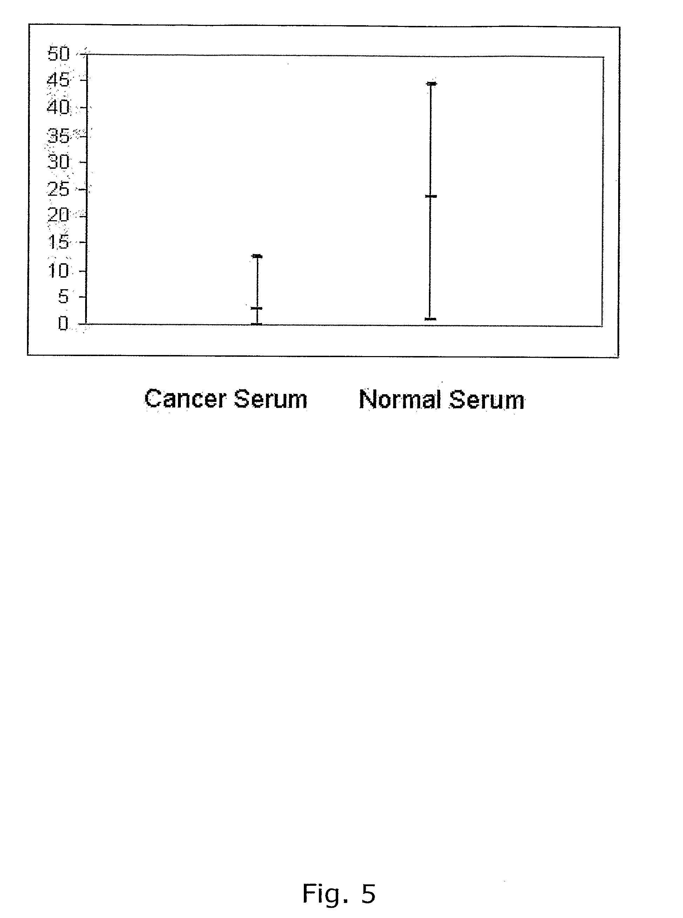

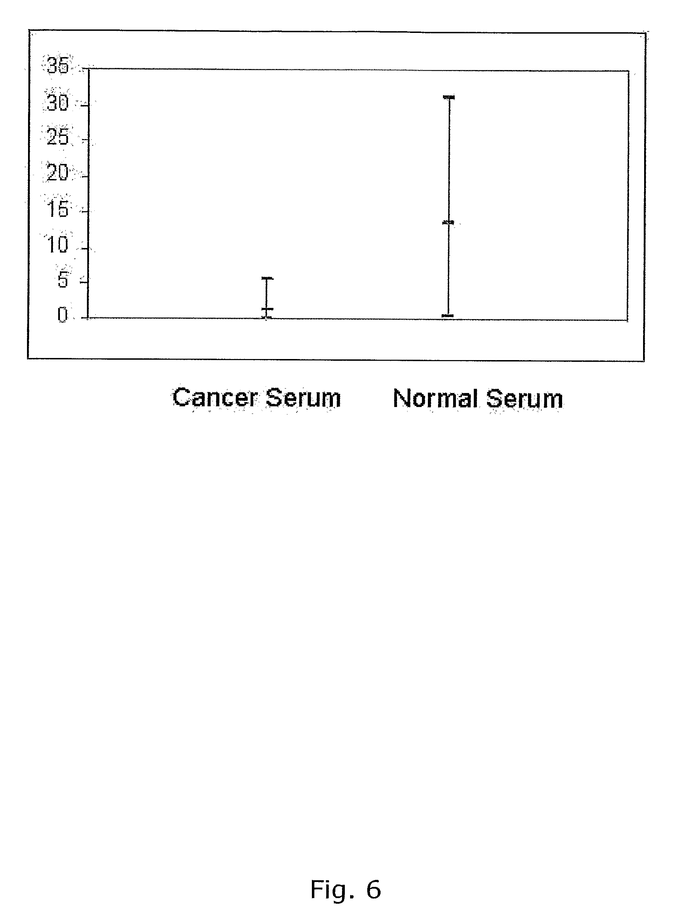

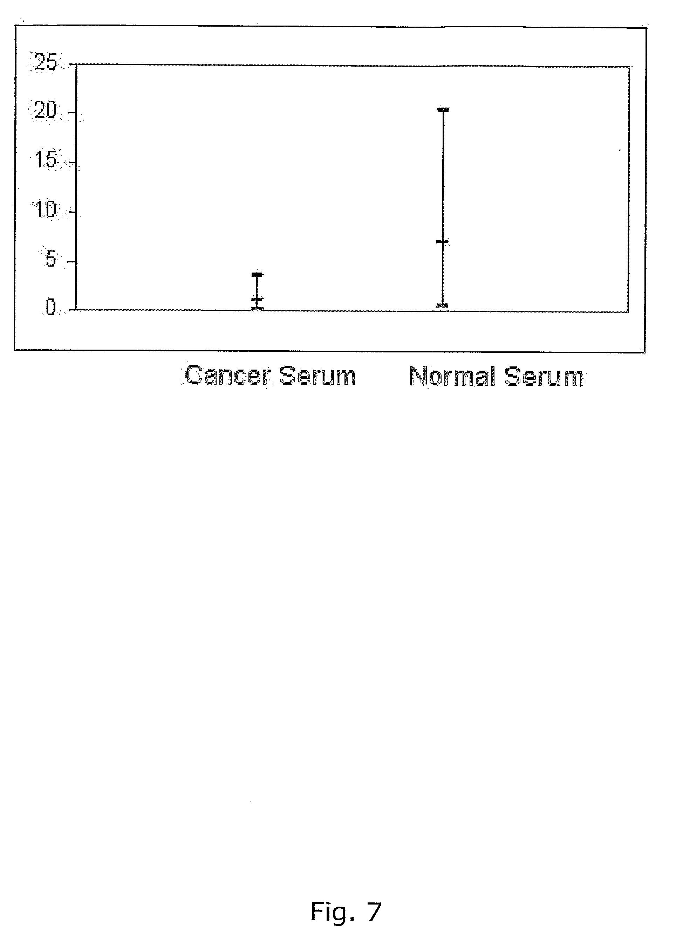

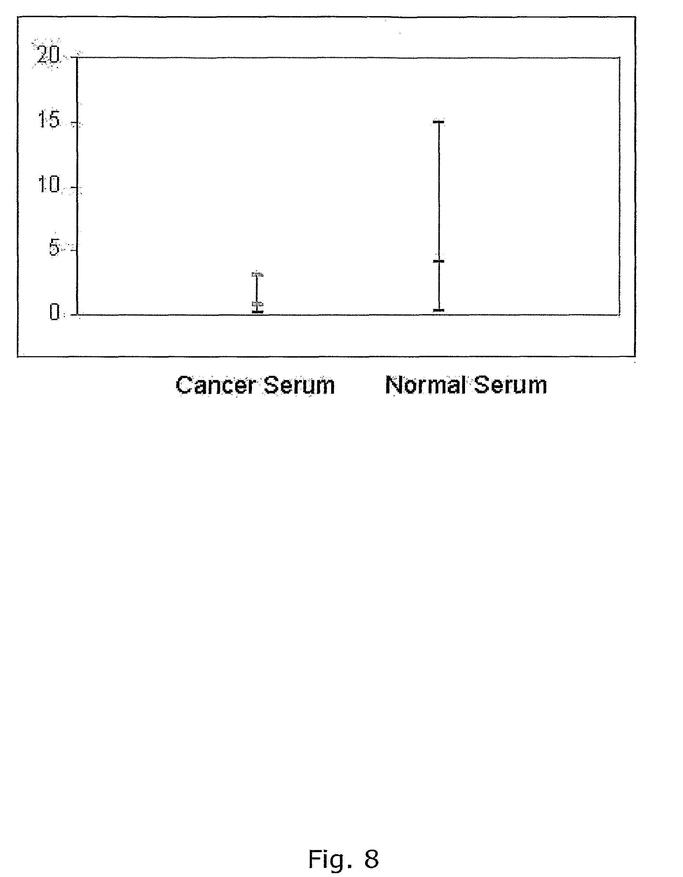

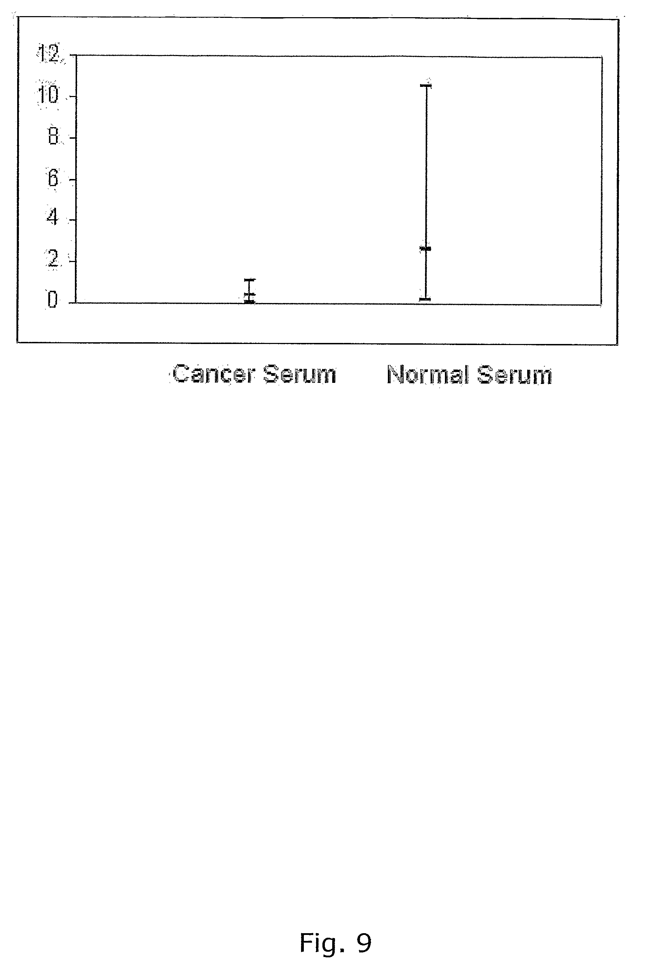

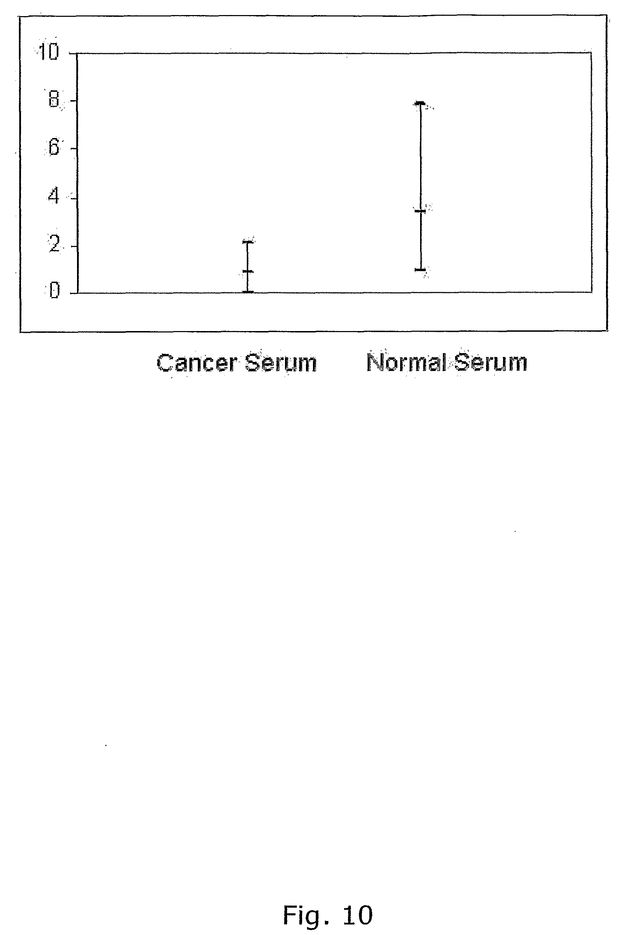

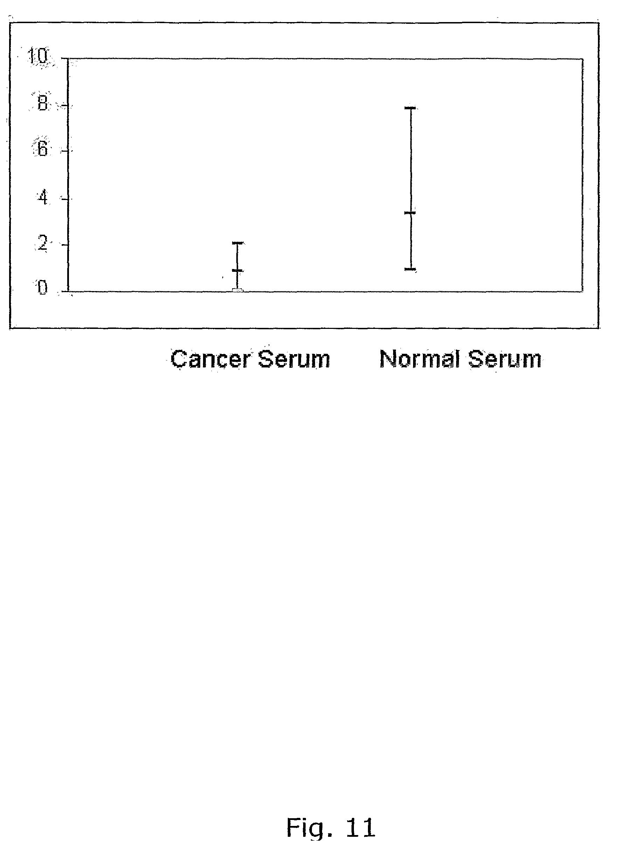

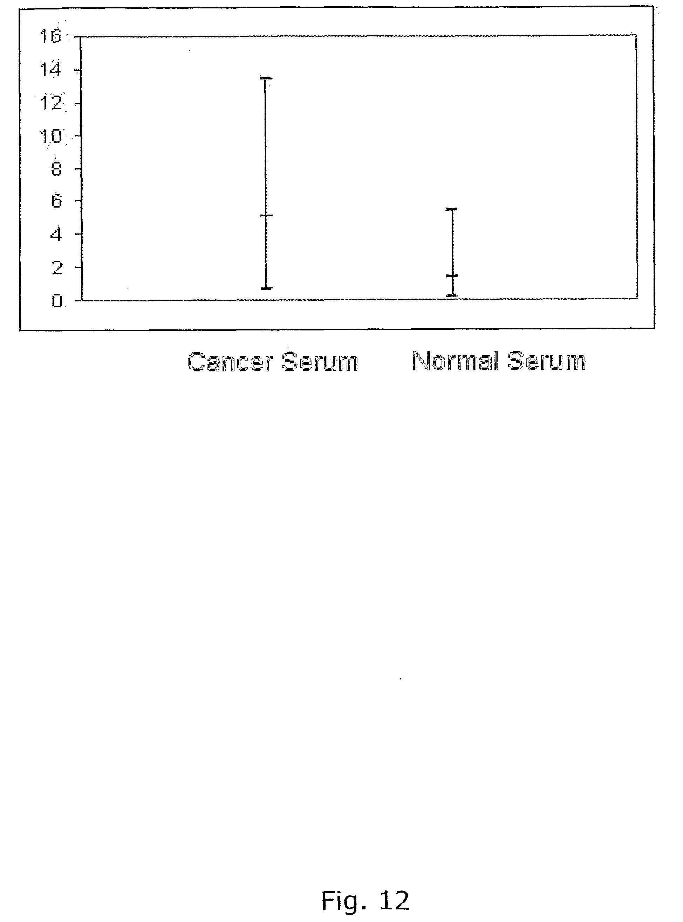

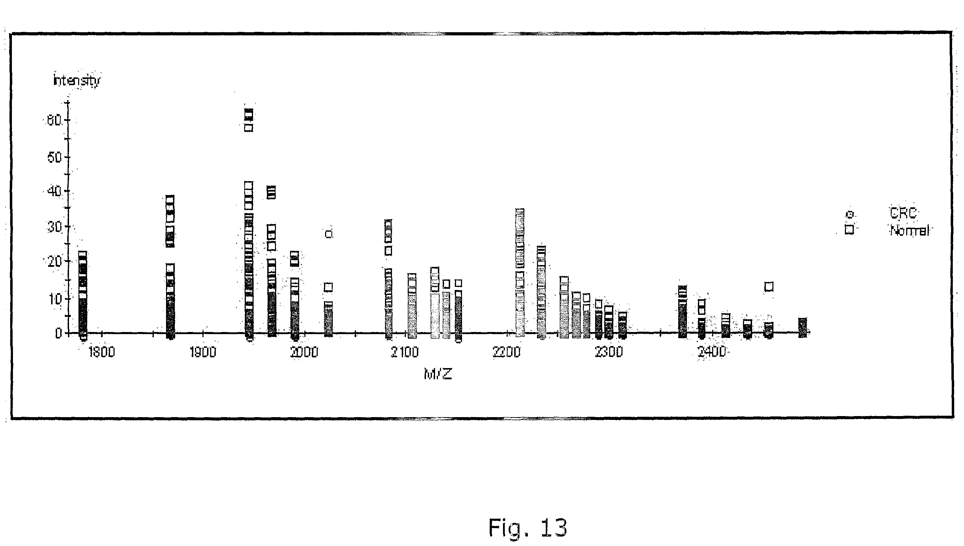

Figure 13 presents the observed pattern of peptides in the region form 1900 to 2500 Da, the present inventors propose that the possible markers of values 1945, 2210, 2230, 2250 and 2275 Da are somehow related. The pattern could indicate:

1) Fragmentation of a larger protein
2) Ligand binding peptides
3) Proteolytic processing of peptides
4) Translational / transcriptional regulation of peptides.

EXAMPLE.16 A METHOD FOR DISCRIMINATING BETWEEN HEALTHY INDIVIDUALS AND PATIENTS WITH COLON CANCER

The aim of the study was to determine if visual inspection of mass spectra is a method for discriminating between healthy individuals and patients with colon cancer.

*Computer programs:*

Ciphergen ProteinChip Software
Excel

*Data:*

Serum samples from 47 healthy individuals and 24 patients diagnosed with colon cancer were assayed on IMAC30 chips and analysed as described above.
Intensities were normalised based on total ion current.

*Method:*

Raw data was exported from Ciphergen ProteinChip Software to Excel, mean and standard error of means (SEM) was calculated for each m/z value.
Mean and SEM was imported in R. Plots for specific regions of the spectra were designed. The specific regions were chosen to include the 5 markers used for Principal component analysis as described above.

*Results:*

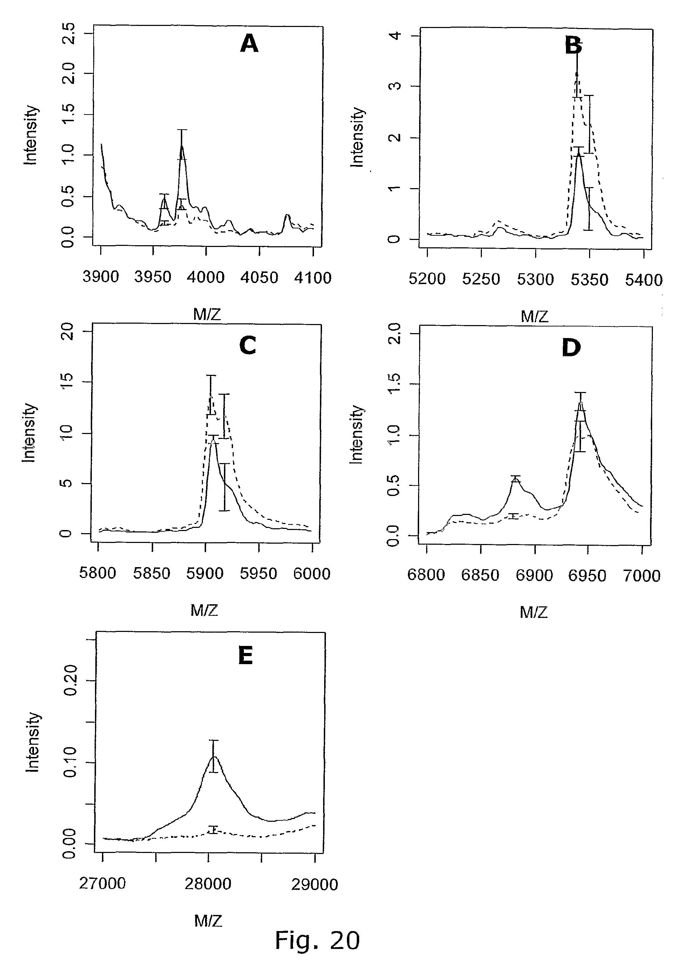

Figure 20 A-E shows the average intensity spectra of healthy individuals (solid) and patients diagnosed with colon cancer (dashed). The standard errors of means (SEM) are shown with bars. A: The area from 3900 to 4100 Da, SEM shown for 3960 and 3980 Da. B: The area from 5200 to 5400 Da, SEM shown for 5340 and 5350 Da. C: The area from 5800 to 6000 Da, SEM shown for 5906 and 5920 Da. D: The area from 6800 to 7000 Da, SEM shown for 6880 and 6940 Da. E: The area from 27000 to 29000 Da, SEM shown for 28025 Da.

*Conclusion*

Visual inspection of specific regions can be used for discriminating healthy individuals from patients with colon cancer.

EXAMPLE 17 POSSIBLE IDENTIFICATION OF SERUM MARKERS BY DIRECT MASS SEARCH.

The aim of this study was to search a database for proteins with known mass corresponding to the measured mass value of the markers identified. This may constitute a possible identification.

*Methods*

The measured mass value is analysed on the "TagIdent Tool" on the ExPASy server. With the following parameters:

| | |
|---|---|
| Mass value range: | 0.2% |
| pI: | Complete range |
| Organism: | Human |
| Database(s) on which the scan should be performed: | Swiss-Prot |

*Results*

By searching the database with the mass value of each of the markers a number of possible identifications occur (hits):

Table 22 Up-regulated serum markers

| Marker 11900, up-regulated on H50 | | |
|---|---|---|
| Gene symbol | Accession No. | Annotation |
| ANFB_HUMAN | (P16860) | Gamma-brain natriuretic peptide. |
| ICE2_HUMAN | (P42575) | Caspase-2 subunit p12. |
| ICE3_HUMAN | (P42574) | Apopain p12 subunit. |
| ICEA_HUMAN | (Q92851) | Caspase-10 subunit p12. |
| LRP2_HHV1F | (P17589) | Latency-related protein 2. |
| VE4_HPV41 | (P27553) | Probable E4 protein. |
| VE7_HPV56 | (P36833) | E7 protein. |
| VE7_HPV66 | (Q80956) | E7 protein. |
| YG49_HUMAN | (Q9BY77) | Hypothetical protein KIAA1649 |
| | | |
| Marker 11700, up-regulated on H50 | | |
| Gene symbol | Accession No. | Annotation |
| GPA2_HUMAN | (Q96T91) | Glycoprotein hormone alpha 2. |

(continued)

| Marker 11700, up-regulated on H50 | | |
|---|---|---|
| Gene symbol | Accession No. | Annotation |
| LSM3_HUMAN | (Q9Y4Z1) | U6 snRNA-associated Sm-like protein LSm3 (MDS017). |
| MIR2_HUMAN | (Q9Y6H6) | Potassium voltage-gated channel subfamily E member 3 |
| NRTN_HUMAN | (Q99748) | Neurturin. |
| S103_HUMAN | (P33764) | S100 calcium-binding protein A3 (S-100E protein). |
| SAA_HUMAN | (P02735) | Serum amyloid A protein. |
| ULA9_HCMVA | (P16738) | Hypothetical protein UL109. |
| VE7_HPV05 | (P06932) | E7 protein. |
| VE7_HPV5B | (P26559) | E7 protein. |
| E311_ADE02 | (P24935) | Early E3A 11.6 kDa glycoprotein. |
| FKBB_HUMAN | (Q16645) | FK506-binding protein 1B |
| GLRX_HUMAN | (P35754) | Glutaredoxin (Thioltransferase) (TTase). |
| RLA2_HUMAN | (P05387) | 60S acidic ribosomal protein P2. |
| S114_HUMAN | (Q9HCY8) | S100 calcium-binding protein A14 (S114). |
| SM31_HUMAN | (P55854) | Ubiquitin-like protein SMT3A. |
| TAT_HV1MN | (P05905) | TAT protein (Transactivating regulatory protein). |
| VE7_HPV08 | (P06430) | E7 protein. |
| Y116_ADE02 | (P03287) | Hypothetical 11.6 kDa early protein |

| Marker 11550, up-regulated on H50 | | |
|---|---|---|
| Gene symbol | Accession No. | Annotation |
| CF53_HUMAN | (Q9POS9) | Protein C6orf53 (Protein HSPC194). |
| HMGI_HUMAN | (P17096) | High mobility group protein |
| INI7_HUMAN | (P40305) | Interferon-alpha induced 11.5 kDa protein (p27). |
| K413_HUMAN | (Q9BYU7) | Keratin associated protein |
| KV1W_HUMAN | (P04431) | Ig kappa chain V-I region Walker precursor |
| TAT_HV1A2 | (P04614) | TAT protein (Transactivating regulatory protein). |
| TAT_HV1OY | (P20893) | TAT protein (Transactivating regulatory protein). |
| TAT_HV1RH | (P05908) | TAT protein (Transactivating regulatory protein). |
| ULB1_HCMVA | (P16831) | Hypothetical protein UL111. |
| VE7_HPV19 | (P36822) | E7 protein. |
| VE7_HPV21 | (P50779) | E7 protein. |
| VE7_HPV47 | (P22423) | E7 protein. |
| VPR_HV1A2 | (P05952) | VPR protein (R ORF protein). |
| Y115_ADE07 | (P03288) | Hypothetical 11.5 kDa early protein. |

(continued)

| Marker 11500, up-regulated on H50 | | |
|---|---|---|
| Gene symbol | Accession No. | Annotation |
| LV1G_HUMAN | (P06316) | Ig lambda chain V-I region BL2. |
| PRP1_HUMAN | (P04280) | Salivary proline-rich protein precursor |
| RLA1_HUMAN | (P05386) | 60S acidic ribosomal protein P1. |
| RT16_HUMAN | (Q9Y3D3) | 28S ribosomal protein S16. |
| S11Y_HUMAN | (Q9UDP3) | Putative S100 calcium-binding protein H_NH0456N16.1. |
| TAT_HV1JR | (P20879) | TAT protein (Transactivating regulatory protein). |
| TAT_HV1S1 | (P19553) | TAT protein (Transactivating regulatory protein). |
| TAT_HV1S3 | (P19552) | TAT protein (Transactivating regulatory protein). |
| VE7_HPV12 | (P36819) | E7 protein. |
| VE7_HPV25 | (P36823) | E7 protein. |
| VPR3_HUMAN | (Q9UKI3) | Pre-B lymphocyte protein 3. |
| VPR_HV10Y | (P20891) | VPR protein (R ORF protein). |

| Marker 15200, up-regulated on CM10 | | |
|---|---|---|
| Gene symbol | Accession No. | Annotation |
| CYB5_HUMAN | (P00167) | Cytochrome b5. |
| ENR1_HUMAN | (Q14264) | Transmembrane protein (By similarity). |
| H33_HUMAN | (P06351) | Histone H3.3 |
| H3B_HUMAN | (Q93081) | Histone H3/b. |
| LSM1_HUMAN | (015116) | U6 snRNA-associated Sm-like protein LSm1 |
| SSB_HUMAN | (Q04837) | Single-stranded DNA-binding protein. |

| Marker 6125, up-regulated on CM10 | | |
|---|---|---|
| Gene symbol | Accession No. | Annotation |
| MT1A_HUMAN | (P04731) | Metallothionein-IA (MT-1A). |
| MT1B_HUMAN | (P07438) | Metallothionein-IB (MT-1B). |

| Marker 5900, up-regulated on CM10 | | |
|---|---|---|
| Gene symbol | Accession No. | Annotation |
| A4_HUMAN | (P05067) | Gamma-CTF(50) (By similarity). |

| Marker 33000, up-regulated on SAX2 | | |
|---|---|---|
| Gene symbol | Accession No. | Annotation |
| ADT1_HUMAN | (P12235) | ADP,ATP carrier protein |
| CAMG_HUMAN | (P49069) | Calcium-signal modulating cyclophilin ligand (CAML). |
| DSR3_HUMAN | (014972) | Down syndrome critical region protein 3 |
| LECH_HUMAN | (P07306) | Asialoglycoprotein receptor 1 |

(continued)

| Marker 33000, up-regulated on SAX2 | | |
|---|---|---|
| MC33_HUMAN | (Q14805) | Metaphase chromosomal protein 1 |
| MCAT_HUMAN | (043772) | Mitochondrial carnitine/acylcarnitine carrier protein |
| MDHM_HUMAN | (P40926) | Malate dehydrogenase. |
| MIOX_HUMAN | (Q9UGB7) | Inositol oxygenase |
| MSLN_HUMAN | (Q13421) | Mesothelin. |
| PCTL_HUMAN | (Q9Y365) | PCTP-like protein |
| R1AB_CVH22 | (Q05002) | Replicase polyprotein lab |
| R1AB_CVHSA | (P59641) | NSP3 (By similarity). |
| REM_HUMAN | (075628) | GTP-binding protein REM |
| SGCZ_HUMAN | (Q96LD1) | Zeta-sarcoglycan (Zeta-SG) (ZSG1). |
| ST1A_HUMAN | (Q16623) | Syntaxin 1A (Neuron-specific antigen HPC-1). |
| T2EB_HUMAN | (P29084) | Transcription initiation factor IIE, beta subunit |
| THTM_HUMAN | (P25325) | 3-mercaptopyruvate sulfurtransferase (EC 2.8.1.2) MST |
| UCP1_HUMAN | (P25874) | Mitochondrial brown fat uncoupling protein 1 (UCP 1) |
| UL07_HHV11 | (P10191) | Protein UL7. |
| UL07_HHV2H | (P89430) | Protein UL7. |
| VE4_HPV47 | (P22421) | Probable E4 protein. |
| VP19_HCMVA | (P16783) | Capsid protein VP19C |
| CU87_HUMAN | (P59051) | Hypothetical protein C21orf87. |
| GGB1_HUMAN | (075459)+ | G antigen family B 1 protein |
| GGD2_HUMAN | (Q9HD64) | G antigen family D 2 protein |
| ID1_HUMAN | (P41134) | DNA-binding protein inhibitor ID-1 |
| POLG_HRV16 | (Q82122) | Core protein p2A. |
| POLG_HRV89 | (P07210) | Core protein p2A. |
| PP13_HUMAN | (Q9UHV8) | (Placenta protein 13) |

| Marker 15935, up-regulated on SAX2 | | |
|---|---|---|
| Gene symbol | Accession No. | Annotation |
| CAL5_HUMAN | (Q9NZT1) | Calmodulin-like protein 5 |
| COAC_HUMAN | (Q14019) | Coactosin-like protein. |
| GML_HUMAN | (Q99445) | Glycosyl-phosphatidylinositol-anchored molecule-like |
| HBD_HUMAN | (P02042) | Hemoglobin delta chain. |
| HPT_HUMAN | (P00738) | Haptoglobin alpha chain. |
| IR09_HCMVA | (P16807) | Hypothetical protein IRL9 (TRL9). |
| M46E_HUMAN | (Q96DS6) | Membrane-spanning 4-domains subfamily A member 6E. |
| RS19_HUMAN | (P39019) | 40S ribosomal protein S19 |
| SJ2B_HUMAN | (P57105) | Synaptojanin 2 binding protein |
| ULC6_HCMVA | (P16836) | Hypothetical protein UL126. |

(continued)

| Marker 15200, up-regulated on SAX2 | | |
|---|---|---|
| Gene symbol | Accession No. | Annotation |
| CYB5_HUMAN | (P00167) | Cytochrome b5. |
| ENR1_HUMAN | (Q14264) | Transmembrane protein (By similarity). |
| H33_HUMAN | (P06351) | Histone H3.3 |
| H3B_HUMAN | (Q93081) | Histone H3/b. |
| LSM1_HUMAN | (015116) | U6 snRNA-associated Sm-like protein LSm1 |
| SSB_HUMAN | (Q04837) | Single-stranded DNA-binding protein. |

| Marker 60500, up-regulated on IMAC30 | | |
|---|---|---|
| Gene symbol | Accession No. | Annotation |
| A1AD_HUMAN | (P25100) | Alpha-ID adrenergic receptor |
| CBS_HUMAN | (P35520) | Cystathionine beta-synthase |
| CDY1_HUMAN | (Q9Y6F8) | Testis-specific chromodomain protein Y 1. |
| CDY2_HUMAN | (Q9Y6F7) | Testis-specific chromodomain protein Y 2. |
| ELS_HUMAN | (P15502) | Elastin precursor (Tropoelastin). |
| EST1_HUMAN | (P23141) | Liver carboxylesterase. |
| FIB1_ADE41 | (P14267) | Fiber protein 1. |
| GKP2_HUMAN | (Q14410) | Glycerol kinase, testis specific 2 |
| GKP3_HUMAN | (Q14409) | Glycerol kinase, testis specific 1 |
| N4B3_HUMAN | (015049) | Nedd4-binding protein 3 (N4BP3). |
| SMA4_HUMAN | (Q13485) | (SMAD 4) |
| SUW1_HUMAN | (P59817) | Suppressor of hairy wing homolog 1 (3'OY11.1). |
| TCPG_HUMAN | (P49368) | (TCP-1-gamma) (CCT-gamma). |
| THAS_HUMAN | (P24557) | Thromboxane-A synthase |
| TTC8_HUMAN | (Q8TAM2) | Tetratricopeptide repeat protein 8 |
| Y469_HUMAN | (Q9UJP4) | Hypothetical protein KIAA0469. |
| Z306_HUMAN | (Q9BRRO) | Zinc finger protein 306 |
| Z479_HUMAN | (Q96JC4) | Zinc finger protein Kr19) (HKr19). |

| Marker 19900, up-regulated on IMAC30 | | |
|---|---|---|
| Gene symbol | Accession No. | Annotation |
| AMEX_HUMAN | (Q99217) | Amelogenin, X isoform. |
| CIT1_HUMAN | (Q99966) | Cbp/p300-interacting transactivator 1 |
| CLE1_HUMAN | (075596) | C-type lectin superfamily member 1. |
| CRAA_HUMAN | (P02489) | Alpha crystallin A chain. |
| FRIL_HUMAN | (P02792) | Ferritin light chain (Ferritin L subunit). |
| GILT_HUMAN | (P13284) | (Gamma-interferon-inducible protein IP-30). |

(continued)

| Marker 19900, up-regulated on IMAC30 | | |
|---|---|---|
| KR45_HUMAN | (Q9BYR2) | Keratin associated protein 4-5 |
| RB8A_HUMAN | (Q9Y5S9) | RNA-binding protein 8A |
| TD52_HUMAN | (P55327) | Tumor protein D52 (N8 protein). |
| TMG4_HUMAN | (Q9BZD6) | TMG4-prescursor |
| YAF2_HUMAN | (Q8IY57) | YY1-associated factor 2. |

| Marker 11080, up-regulated on IMAC30 | | |
|---|---|---|
| Gene symbol | Accession No. | Annotation |
| IDS_HUMAN | (P22304) | Iduronate 2-sulfatase 14 kDa chain. |
| S110_HUMAN | (P08206) | Calpactin I light chain |
| TAT_HV1EL | (P04611) | TAT protein |
| VE7_HPV65 | (Q07859) | E7 protein. |

| Marker 10830, up-regulated on IMAC30 | | |
|---|---|---|
| Gene symbol | Accession No. | Annotation |
| LSM2_HUMAN | (Q9Y333) | U6 snRNA-associated Sm-like protein LSm2 |
| LST1_HUMAN | (000453) | Leukocyte specific transcript 1 protein |
| POLG_HE701 | (P32537) | Core protein p2B. |
| POL_HV1ND | (P18802) | Protease. |
| POL_HV1OY | (P20892) | Protease. |
| POL_HV2BE | (P18096) | Protease. |
| S108_HUMAN | (P05109) | Calgranulin A |
| VE7_HPV33 | (P06429) | E7 protein. |
| VE7_HPV58 | (P26557) | E7 protein. |

| Marker 9140, up-regulated on IMAC30 | | |
|---|---|---|
| Gene symbol | Accession No. | Annotation |
| LSM6_HUMAN | (Q9Y4Y8) | U6 snRNA-associated Sm-like protein LSm6 |
| SAP_HUMAN | (P07602) | Saposin D. |
| VPU_HV1LW | (Q70625) | VPU protein (U ORF protein). |

| Marker 8930, up-regulated on IMAC30 | | |
|---|---|---|
| Gene symbol | Accession No. | Annotation |
| APC2_HUMAN | (P02655) | Apolipoprotein C-II. |
| IL8_HUMAN | (P10145) | Interleukin-8. |
| PLMN_HUMAN | (P00747) | Activation peptide. |
| SLUR_HUMAN | (P55000) | Secreted Ly-6/uPAR related protein 1. |
| SRG1_HUMAN | (075711) | Scrapie-responsive protein 1. |

(continued)

| Marker 8930, up-regulated on IMAC30 | | |
|---|---|---|
| SY08_HUMAN | (P80075) | Small inducible cytokine A8. |
| VGLF_PI2H | (P25467) | Fusion glycoprotein F2. |
| VGLF_PI2HG | (P27286) | Fusion glycoprotein F2. |
| VGLF_PI2HT | (P26629) | Fusion glycoprotein F2. |
| | | |
| Marker 6110, up-regulated on IMAC30 | | |
| Gene symbol | Accession No. | Annotation |
| MT1B_HUMAN | (P07438) | Metallothionein-IB (MT-1B). |
| PPLA_HUMAN | (P26678) | Cardiac phospholamban (PLB). |
| WFAB_HUMAN | (Q8IUB3) | Protein WFDC10B. |
| | | |
| Marker 6090, up-regulated on IMAC30 | | |
| Gene symbol | | Accession No. Metallothionein-IF |
| MT1F_HUMAN | (P04733) | (MT-1F) (HQP0376). |
| | | |
| Marker 5920, up-regulated on IMAC30 | | |
| Gene symbol | Accession No. (P05067) | Annotation Gamma-CTF(50) |
| A4_HUMAN | | (By similarity). |
| | | |
| Marker 5900, up-regulated on IMAC30 | | |
| Gene symbol | Accession No. | Annotation |
| A4_HUMAN | (P05067) | Gamma-CTF(50) (By similarity). |
| GAG_HV1A2 | (P03349) | Core protein p6. |
| | | |
| Marker 5330, up-regulated on IMAC30 | | |
| Gene symbol | Accession No. | Annotation Oculomedin |
| TISR_HUMAN | (Q9Y5M6) | |

Table 23 Down-regulated on IMAC30

| Marker 46000, down-regulated on H50 | | |
|---|---|---|
| Gene symbol | Accession No. | Annotation |
| AB3B_HUMAN | (Q9UH17) | Phorbolin 1-related protein) |
| B3G7_HUMAN | (Q9NY97) | Beta-1,3-galactosyltransferase 7 |
| BTB6_HUMAN | (Q96KE9) | BTB/POZ domain containing protein 6 |
| CRF2_HUMAN | (Q13324) | Corticotropin releasing factor receptor 2 precursor |
| EGL1_HUMAN | (Q9GZT9) | Egl nine homolog 1 |
| FXF2_HUMAN | (Q12947) | Forkhead box protein F2 |
| GPT_HUMAN | (Q9H3H5) | (EC 2.7.8.15) (GPT |

(continued)

| Marker 46000, down-regulated on H50 | | |
|---|---|---|
| K1HA_HUMAN | (076009) | Keratin, type I cuticular HA3-I |
| MCR1_HUMAN | (Q99705) | Melanin-concentrating hormone receptor 1 |
| NCAP_CVHSA | (P59595) | Nucleocapsid protein |
| OAS1_HUMAN | (P00973) | 2'-5'-oligoadenylate synthetase 1 |
| OST4_HUMAN | (P39656) | (Oligosaccharyl transferase 48 kDa subunit) |
| PI53_HUMAN | (P53807) | Phosphatidylinositol-4-phosphate 5-kinase type III |
| Marker 46000, down-regulated on H50 | | |
| Gene symbol | Accession No. | Annotation |
| PRD7_HUMAN | (Q9NQW5) | PR-domain zinc finger protein 7. |
| RL3_HUMAN | (P39023) | 60S ribosomal protein L3 |
| S143_HUMAN | (Q9UDX4) | SEC14-like protein 3 |
| SSXT_HUMAN | (Q15532) | SSXT protein |
| TDG_HUMAN | (Q13569) | G/T mismatch-specific thymine DNA glycosylase |
| TR1B_HUMAN | (P20333) | Tumor necrosis factor receptor superfamily |
| Z193_HUMAN | (015535) | Zinc finger protein 193 (PRD51). |
| Z514_HUMAN | (Q96K75) | Zinc finger protein 514. |
| ZDHB_HUMAN | (Q9H8X9) | Zinc finger protein 399 |
| | | |
| Marker 45500, down-regulated on H50 | | |
| Gene symbol | Accession No. | Annotation |
| AAAD_HUMAN | (P22760) | Arylacetamide deacetylase |
| BHB2_HUMAN | (014503) | Class B basic helix-loop-helix protein 2 |
| CL02_HUMAN | (Q8NHQ8) | Protein C12orf2 |
| COT2_HUMAN | (P24468) | COUP transcription factor 2 |
| CV05_HUMAN | (Q9Y519) | Putative MAP kinase activating protein |
| CXA7_HUMAN | (P36383) | Gap junction alpha-7 protein |
| DEMA_HUMAN | (Q08495) | Dematin |
| DOK2_HUMAN | (060496) | Docking protein 2 |
| FUT4_HUMAN | (P22083) | Fucosyltransferase 4 |
| GAG2_HUMAN | (P10264) | HERV-K10 putative GAG polyprotein 2. |
| IL5R_HUMAN | (Q01344) | Interleukin-5 receptor alpha chain precursor |
| MKK2_HUMAN | (P49137) | MAP kinase-activated protein kinase 2 |
| NTR2_HUMAN | (095665) | Neurotensin receptor type-2 |
| ODBA_HUMAN | (P12694) | 2-oxoisovalerate dehydrogenase alpha subunit, |
| PCO1_HUMAN | (Q15113) | Procollagen C-proteinase enhancer protein precursor |
| PLA1_HUMAN | (Q9HB21) | Pleckstrin homology domain-containing protein family A member 1 |
| PREB_HUMAN | (Q9HCU5) | Prolactin regulatory element-binding protein. |
| PSD6_HUMAN | (Q15008) | 26S proteasome non-ATPase regulatory subunit 6 |

(continued)

| Marker 45500, down-regulated on H50 | | |
|---|---|---|
| RHCE_HUMAN | (P18577) | Blood group Rh(CE) polypeptide |
| RT29_HUMAN | (P51398) | Mitochondrial 28S ribosomal protein |
| SYT7_HUMAN | (043581) | Synaptotagmin VII (SytVII). |
| TC10_HUMAN | (Q12799) | T-complex protein 10A homolog. |
| TCO1_HUMAN | P20061) | Transcobalamin I. |
| TCO2_HUMAN | (P20062) | Transcobafamin II. |

| Marker 45500, down-regulated on H50 | | |
|---|---|---|
| Gene symbol | Accession No. | Annotation |
| ULB7_HCMVA | (P16770) | Hypothetical protein UL117. |
| VE2_HPV1A | (P03118) | Regulatory protein E2. |
| VE2_HPV50 | (Q80930) | Regulatory protein E2. |
| VE2_HPV63 | (Q07850) | Regulatory protein E2. |
| VE2_HPV65 | (Q07851) | Regulatory protein E2. |
| VRK1_HUMAN | (Q99986) | Serine/threonine protein kinase VRK1 |
| WDR4_HUMAN | (P57081) | WD-repeat protein 4. |

| Marker 8940, down-regulated on H50 | | |
|---|---|---|
| Gene symbol | Accession No. | Annotation |
| SLUR_HUMAN | (P55000) | Secreted Ly-6/uPAR related protein 1. |
| SRG1_HUMAN | (075711) | Scrapie-responsive protein 1. |
| SY07_HUMAN | (P80098) | Small inducible cytokine A7. |
| VE5_HPV58 | (P26552) | Probable E5 protein. |

| Marker 8230, down-regulated on H50 | | |
|---|---|---|
| Gene symbol | Accession No. | Annotation |
| PSCA_HUMAN | (043653) | Prostate stem cell antigen. |
| UGR2_HUMAN | (Q96QR1) | Uteroglobin-related protein 2. |
| ULD1_HCMVA | (P16773) | Hypothetical protein UL131. |

| Marker 6650, down-regulated on H50 | | |
|---|---|---|
| Gene symbol | Accession No. | Annotation |
| 68MP_HUMAN | (P56378) | 6.8 kDa mitochondrial proteolipid |
| A4_HUMAN | (P05067) | Gamma-CTF(57). |
| CCKN_HUMAN | (P06307) | Cholecystokinin CCK58. |
| NRG4_HUMAN | (Q8WWG1) | Neuregulin-4. |
| PART_HUMAN | (Q9NPDO) | Prostate-specific and androgen regulated protein PART-1 |
| PE19_HUMAN | (P48539) | Brain-specific polypeptide PEP-19 |
| RS30_HUMAN | (Q05472) | 40S ribosomal protein S30. |

(continued)

| Marker 6450, down-regulated on H50 | | |
| --- | --- | --- |
| Gene symbol | Accession No. | Annotation |
| 3CL_HUMAN | (Q13412) | Pre-T/NK cell associated protein 3Cl. |
| E306_ADE35 | (P17591) | Early E3 6.4 kDa protein. |
| GAG_HV1A2 | (P03349) | Core protein p7. |
| GAG_HV1B1 | (P03347) | Core protein p7. |
| GAG_HV1JR | (P20873) | Core protein p7. |
| GAG_HV1MN | (P05888) | Core protein p7. |
| GAG_HV1PV | (P03350) | Core protein p7. |
| GLPE_HUMAN | (P15421) | Glycophorin E. |

| Marker 1536, down-regulated on CM10 | | |
| --- | --- | --- |
| Gene symbol | Accession No. | Annotation |
| CCKN_HUMAN | (P06307) | Cholecystokinin CCK12. |
| FIBA_HUMAN | (P02671) | Fibrinopeptide A. |

| Marker 66500, down-regulated on IMAC30 | | |
| --- | --- | --- |
| Gene symbol | Accession No. | Annotation |
| AFAM_HUMAN | (P43652) | Afamin. |
| ALBU_HUMAN | (P02768) | Serum albumin. |
| AN21_HUMAN | (Q86YR6) | Ankyrin repeat domain protein 21 |
| BRL1_EBV | (P03209) | Transcription activator BRLF1. |
| CALI_HUMAN | (Q13939) | Calicin. |
| CD93_HUMAN | (Q9NPY3) | Complement component C1q receptor. |
| CDYL_HUMAN | (Q9Y232) | Chromodomain Y-like protein |
| FETA_HUMAN | (P02771) | Alpha-fetoprotein precursor |
| FPGT_HUMAN | (014772) | Fucose-1-phosphate guanylyltransferase |
| FUT8_HUMAN | (Q9BYC5) | Alpha-(1,6)-fucosyltransferase |
| GBP5_HUMAN | (Q96PP8) | Interferon-induced guanylate-binding protein 5 |
| GDS1_HUMAN | (P52306) | Rap1 GTPase-GDP dissociation stimulator 1 |
| GRK4_HUMAN | (P32298) | G protein-coupled receptor kinase |
| MM09_HUMAN | (P14780) | type IV collagenase. |
| MOT8_HUMAN | (P36021) | Monocarboxylate transporter 8 |
| NR42_HUMAN | (P43354) | Orphan nuclear receptor NURR1 |
| SNX9_HUMAN | (Q9Y5X1) | Sorting nexin 9 |
| STB2_HUMAN | (Q15833) | Syntaxin binding protein 2 |
| VP40_HHV11 | (P10210) | Gene UL26 protein. |

(continued)

| Marker 66500, down-regulated on IMAC30 | | |
|---|---|---|
| Gene symbol | Accession No. | Annotation |
| VU47_HHV6U | (Q06093) | Glycoprotein U47. |

| Marker 44300, down-regulated on IMAC30 | | |
|---|---|---|
| Gene symbol | Accession No. | Annotation |
| A1AT_HUMAN | (P01009) | Alpha-1-antitrypsin. |
| ABA2_HUMAN | (Q96P71) | Amyloid beta A4 protein-binding family A |
| APL3_HUMAN | (095236) | Apolipoprotein L3 |
| CEA2_HUMAN | (Q9NPF8) | Centaurin alpha 2. |
| CK16_HUMAN | (Q9NQ32) | Protein C11orf16. |
| D3DR_HUMAN | (P35462) | D(3) dopamine receptor. |
| DCT2_HUMAN | (Q13561) | Dynactin complex 50 kDa subunit |
| ELK3_HUMAN | (P41970) | ETS-domain protein Elk-3 |
| GATM_HUMAN | (P50440) | Glycine amidinotransferase |
| GBAF_HUMAN | (P38405) | Guanine nucleotide-binding protein G(olf) |
| HXB3_HUMAN | (P14651) | Homeobox protein Hox-B3 |
| KLFC_HUMAN | (Q9Y4X4) | Krueppel-like factor 12 |
| LHX2_HUMAN | (P50458) | LIM/homeobox protein Lhx2 |
| MM11_HUMAN | (P24347) | Stromelysin-3. |
| MPK4_HUMAN | (P45985) | MAP kinase kinase 4 |
| OMGP_HUMAN | (P23515) | Oligodendrocyte-myelin glycoprotein. |
| P2X3_HUMAN | (P56373) | P2X purinoceptor 3 |
| PSG3_HUMAN | (Q16557) | Pregnancy-specific beta-1-glycoprotein 3 |
| RUN3_HUMAN | (Q13761) | Runt-related transcription factor 3 |
| S3B4_HUMAN | (Q15427) | Splicing factor 3B subunit 4 |
| SB13_HUMAN | (Q9UIV8) | Hurpin |
| SUT3_HUMAN | (075486) | Transcription initiation protein SPT3 homolog |
| TE2I_HUMAN | (Q9NYB0) | Telomeric repeat binding factor 2 interacting protein 1 |
| TFT1_HUMAN | (Q9NNX1) | Tuftelin. |
| TRUA_HUMAN | (Q9Y606) | tRNA pseudouridine synthase A |
| UL61_HCMVA | (P16818) | Hypothetical protein UL61. |
| VE2_HPV03 | (P36778) | Regulatory protein E2. |
| VE2_HPV29 | (P50772) | Regulatory protein E2. |
| VE2_HPV41 | (P27552) | Regulatory protein E2. |
| VU3_HHV7J | (P52520) | U3 protein. |

| Marker 28121, down-regulated on IMAC30 | | |
|---|---|---|
| Gene symbol | Accession No. | Annotation |

(continued)

| Marker 28121, down-regulated on IMAC30 | | |
|---|---|---|
| 143F_HUMAM | (Q04917) | 14-3-3 protein eta (Protein AS1). |
| 143G_HUMAN | (P35214) | 14-3-3 protein gamma |
| ABME_HUMAN | (P41238) | Apolipoprotein B |
| APA1_HUMAN | (P02647) | Apolipoprotein A-I precursor (Apo-AI). |
| CCG6_HUMAN | (Q9BXT2) | calcium channel gamma-6 subunit |
| CDX1_HUMAN | (P47902) | Homeobox protein CDX-1 |
| CNG6_HUMAN | (Q9Y224) | Protein C14orf166 (CGI-99). |
| CTX3_HUMAN | (Q9UJQ1) | Protein C20orf103 precursor. |
| DRN2_HUMAN | (000115) | Deoxyribonuclease II precursor |
| E1A_ADE04 | (P10407) | Early E1A 28 kDa protein. |
| EP34_HCMVA | (P16768) | Early phosphoprotein P34. |
| FA7_HUMAN | (P08709) | Factor VII heavy chain. |
| K247_HUMAN | (Q92537) | Protein KIAA0247 precursor. |
| M4AC_HUMAN | (Q9NXJO) | Membrane-spanning 4-domains subfamily A member 12. |
| MIP_HUMAN | (P30301) | Lens fiber major intrinsic protein |
| MLF2_HUMAN | (Q15773) | Myeloid leukemia factor 2 |
| ORC6_HUMAN | (Q9Y5N6) | Origin recognition complex subunit 6. |
| PMM2_HUMAN | (015305) | Phosphomannomutase 2 |
| PRPK_HUMAN | (Q96S44) | p53-related protein kinase |
| RFXK_HUMAN | (014593) | DNA-binding protein RFXANK |
| STXA_HUMAN | (060499) | Syntaxin 10 (Syn10). |
| TPA_HUMAN | (P00750) | Tissue-type plasminogen activator chain |
| WBP2_HUMAN | (Q969T9) | WW domain binding protein 2 |
| | | |
| Marker 28010, down-regulated on IMAC30 | | |
| Gene symbol | Accession No. | Annotation |
| 2DOB_HUMAN | (P13765) | HLA class II histocompatibility antigen |
| CATW_HUMAN | (P56202) | Cathepsin W |
| CRAR_HUMAN | (P48740) | Complement-activating component of Ra-reactive factor precursor |
| DB83_HUMAN | (P57088) | DB83 protein. |
| DGK_HUMAN | (Q16854) | Deoxyguanosine kinase. |
| GS2_HUMAN | (P41247) | GS2 protein (DXS1283E). |
| HXB9_HUMAN | (P17482) | Homeobox protein Hox-B9 |
| IF28_HUMAN | (Q96DX8) | 28 kDa interferon responsive protein. |
| MOX1_HUMAN | (P50221) | Homeobox protein MOX-1 |
| SHP_HUMAN | (Q15466) | Orphan nuclear receptor SHP |
| SPRE_HUMAN | (P35270) | Sepiapterin reductase |
| T4S8_HUMAN | (060637) | Transmembrane 4 superfamily |

(continued)

| Marker 28010, down-regulated on IMAC30 | | |
|---|---|---|
| VP40_HCMVA | (P16753) | Assemblin. |
| | | |

| Marker 28315, down-regulated on IMAC30 | | |
|---|---|---|
| Gene symbol | Accession No. | Annotation |
| AQP5_HUMAN | (P55064) | Aquaporin 5. |
| BA29_HUMAN | (Q9UHQ4) | B-cell receptor-associated protein 29 |
| C151_HUMAN | (P48509) | Platelet-endothelial tetraspan antigen 3 |
| CBX7_HUMAN | (095931) | Chromobox protein homolog 7. |
| CHOD_HUMAN | (Q9H9P2) | Chondrolectin. |
| CSS1_HUMAN | (P04632) | Calpain small subunit 1 |
| CU02_HUMAN | (043822) | Protein C21orf2 |
| ECHM_HUMAN | (P30084) | Enoyl-CoA hydratase. |
| EMX2_HUMAN | (Q04743) | Homeobox protein EMX2. |
| IFE3_HUMAN | (060573) | Eukaryotic translation initiation factor 4E type |
| NS3B_HUMAN | (Q9BS92) | NipSnap3B protein (SNAP1). |
| POLG_EC22H | (Q66578) | Coat protein VP3. |
| PSA3_HUMAN | (P25788) | Proteasome subunit alpha type 3 |
| THAA_HUMAN | (Q9P2Z0) | THAP domain protein 10. |
| UNG_HCMVA | (P16769) | Uracil-DNA glycosylase |
| VATD_HUMAN | (Q9Y5K8) | Vacuolar ATP synthase subunit D |
| | | |

| Marker 27700, down-regulated on IMAC30 | | |
|---|---|---|
| Gene symbol | Accession No. | Annotation |
| 143Z_HUMAN | (P29312) | 14-3-3 protein zeta/delta |
| AQPA_HUMAN | (Q96PS8) | Aquaporin 10 |
| C1S_HUMAN | (P09871) | Complement C1s component precursor |
| CSS2_HUMAN | (Q96L46) | Calpain small subunit 2 |
| FGFE_HUMAN | (Q92915) | Fibroblast growth factor-14 |
| HXC8_HUMAN | (P31273) | Homeobox protein Hox-C8 |
| NUCG_HUMAN | (Q14249) | Endonuclease G. |
| NXP2_HUMAN | (095156) | Neurexophilin 2. |
| POLG_HE71B | (Q66478) | Coat protein VP2. |
| SHH_HUMAN | (Q15465) | Sonic hedgehog protein C-product |
| SIX6_HUMAN | (095475) | Homeobox protein SIX6 |
| TMS2_HUMAN | (015393) | Transmembrane protease serine 2 non |
| TRYA_HUMAN | (P15157) | Alpha-tryptase. |
| | | |

(continued)

| Marker 15580, down-regulated on IMAC30 | | |
| --- | --- | --- |
| Gene symbol | Accession No. | Annotation |
| CND8_HUMAN | (Q9H867) | Protein C14orf138. |
| ECP_HUMAN | (P12724) | Eosinophil cationic protein. |
| IGJ_HUMAN | (P01591) | Immunoglobulin J chain. |
| POLG_HRV2 | (P04936) | Core protein p2A. |
| RET4_HUMAN | (P29373) | Retinoic acid-binding protein II, |
| SRB7_HUMAN | (Q13503) | RNA polymerase II holoenzyme component SRB7 |
| VNS1_HRSVA | (P04544) | Nonstructural protein 1 |
| | | |
| Marker 13700, down-regulated on IMAC30 | | |
| Gene symbol | Accession No. | Annotation |
| AOAH_HUMAN | (P28039) | Acyloxyacyl hydrolase small subunit. |
| ASAH_HUMAN | (Q13510) | Acid ceramidase alpha subunit. |
| C17_HUMAN | (Q9NRR1) | Cytokine-like protein C17. |
| CU77_HUMAN | (Q9NV44) | Protein C21orf77. |
| NEF_HV1H2 | (P04601) | Negative factor (F-protein) |
| | | |
| Marker 6680, down-regulated on IMAC30 | | |
| Gene symbol | Accession | No. Annotation |
| CU51_HUMAN | (P58511) | Protein C21orf51. |
| | | |
| Marker 6660, down-regulated on IMAC30 | | |
| Gene symbol | Accession No. | Annotation |
| 68MP_HUMAN | (P56378) | 6.8 kDa mitochondrial proteolipid |
| A4_HUMAN | (P05067) | Gamma-CTF(57). |
| GALA_HUMAN | (P22466) | Galanin message-associated peptide. |
| NRG4_HUMAN | (Q8WWG1) | Neuregulin-4. |
| PE19_HUMAN | (P48539) | Brain-specific polypeptide PEP-19 |
| RS30_HUMAN | (Q05472) | 40S ribosomal protein S30. |
| | | |
| Marker 6430, down-regulated on IMAC30 | | |
| Gene symbol | Accession No. | Annotation |
| E306_ADE35 | (P17591) | Early E3 6.4 kDa protein. |
| GAG_HV1BR | (P03348) | Core protein p7. |
| GAG_HV1H2 | (P04591) | Core protein p7. |
| GAG_HV1LW | (Q70622) | Core protein p7. |
| MT4_HUMAN | (P47944) | Metallothionein-IV (MT-IV). |
| YG02_HUMAN | (O60908) | Hypothetical 6.4 kDa protein A-363E6.1. |

EXAMPLE 18 POSSIBLE IDENTIFICATION OF PLASMA MARKERS BY DIRECT MASS SEARCH.

This identification of plasma markers was performed as described for the serum markers in Example 17.

Table 24 Up-regulated plasma markers

| Marker 14100, up-regulated on IMAC30 | | |
|---|---|---|
| Short name | Code | Annotation |
| BATF_HUMAN | (Q16520) | ATF-like basic leucine zipper transcriptional factor B-ATF |
| HEX9_ADE07 | (P03283) | Hexon-associated protein |
| IL9_HUMAN | (P15248) | Interleukin-9 |
| LCA_HUMAN | (P00709) | Alpha-lactalbumin. |
| LSMA_HUMAN | (Q969L4) | U7 snRNA-associated Sm-like protein LSm10. |
| RT06_HUMAN | (P82932) | Mitochondrial 28S ribosomal protein S6 |
| TNRB_HUMAN | (P28908) | Tumor necrosis factor receptor superfamily member 8 precursor |
| TX12_HUMAN | (Q9BXUO) | Testis expressed protein 12. |
| YYY3_HUMAN | (P20931) | Very very hypothetical B-cell growth factor |
| | | |
| Marker 14030, up-regulated on IMAC30 | | |
| Short name | Code | Annotation |
| CTRB_HUMAN | (P17538) | Chymotrypsin B chain B. |
| GRL1_HUMAN | (Q9H0R8) | Gamma-aminobutyric acid receptor-associated protein-like |
| H2AA_HUMAN | (P28001) | Histone H2A.a |
| H2AM_HUMAN | (P04908) | Histone H2A.m (H2A/m). |
| PRB4_HUMAN | (P10163) | Salivary proline-rich protein PO precursor |
| UL30_HCMVA | (P16765) | Hypothetical protein UL30. |
| | | |
| Marker 13870, up-regulated on IMAC30 | | |
| Short name | Code | Annotation |
| CST8_HUMAN | (060676) | Cystatin 8 |
| CYTD_HUMAN | (P28325) | Cystatin D. |
| H2BE_HUMAN | (Q99879) | Histone H2B.e (H2B/e). |
| | | |
| Marker 13747, up-regulated on IMAC30 | | |
| Short name | Code | Annotation |
| ASAH_HUMAN | (Q13510) | Acid ceramidase alpha subunit. |
| CHM1_HUMAN | (075829) | Chondromodulin-I. |
| H2BJ_HUMAN | (Q93079) | Histone H2B.j (H2B/j). |
| H2BR_HUMAN | (P06899) | Histone H2B.r (H2B/r) (H2B.1). |
| RS25_HUMAN | (P25111) | 40S ribosomal protein S25. |
| TTHY_HUMAN | (P02766) | Transthyretin. |
| VAG1_HUMAN | (075348) | Vacuolar ATP synthase subunit G 1 |

(continued)

| Marker 13747, up-regulated on IMAC30 | | |
|---|---|---|
| Short name | Code | Annotation |
| | | |

| Marker 11723, up-regulated on IMAC30 | | |
|---|---|---|
| Short name | Code | Annotation |
| ALK1_HUMAN | (P03973) | Antileukoproteinase 1. |
| B2MG_HUMAN | (PO1884) | Beta-2-microglobulin. |
| GPB5_HUMAN | (Q86YW7) | Glycoprotein hormone beta 5. |
| LSM3_HUMAN | (Q9Y4Z1) | U6 snRNA-associated Sm-like protein LSm3 |
| MIR2_HUMAN | (Q9Y6H6) | Potassium voltage-gated channel subfamily E member 3 |
| PRLS_HUMAN | (Q99954) | Proline-rich protein 5 |
| REV_HV2RO | (P04615) | Anti-repression transactivator protein |
| S103_HUMAN | (P33764) | S100 calcium-binding protein A3 |
| S104_HUMAN | (P26447) | Placental calcium-binding protein |
| S111_HUMAN | (P31949) | Calgizzarin |
| SZ09_HUMAN | (Q07325) | Small inducible cytokine B9 |
| ULA9_HCMVA | (P16738) | Hypothetical protein UL109. |

| Marker 9950, up-regulated on IMAC30 | | |
|---|---|---|
| Short name | Code | Annotation |
| CART_HUMAN | (Q16568) | Cocaine- and amphetamine-regulated transcript protein |
| K123_HUMAN | (P60328) | Keratin associated protein KAP12- |
| NUOS_HUMAN | (Q9NRX3) | NADH:ubiquinone oxidoreductase MLRQ subunit homolog |
| VE4_HPV51 | (P26548) | Probable E4 protein |
| | | |

| Marker 7469, up-regulated on IMAC30 | | |
|---|---|---|
| Short name | Code | Annotation |
| IGF2_HUMAN | (P01344) | Insulin-like growth factor II. |
| | | |

| Marker 5905, up-regulated on IMAC30 | | |
|---|---|---|
| Short name | Code | Annotation |
| A4_HUMAN | (P05067) | Gamma-CTF(50) (By similarity). |
| | | |

| Marker 4977, up-regulated on IMAC30 | | |
|---|---|---|
| Short name | Code | Annotation |
| GIP_HUMAN | (P09681) | Gastric inhibitory polypeptide. |
| | | |

| Marker 4136, up-regulated on IMAC30 | | |
|---|---|---|
| Short name | Code | Annotation |
| UCN3_HUMAN | (Q969E3) | Urocortin III. |

Table 25 Down-regulated plasma markers

| Marker 66800, down-regulated on IMAC30 | | |
|---|---|---|
| Short name | Code | Annotation |
| 3BP1_HUMAN | (Q9Y3L3) | SH3-domain binding protein 1 |
| DCE1_HUMAN | (Q99259) | Glutamate decarboxylase |
| IF3I_HUMAN | (Q9Y262) | Eukaryotic translation initiation factor 3 subunit 6 interacting protein |
| LIB3_HUMAN | (075022) | Leukocyte immunoglobulin-like receptor subfamily B member 3 precursor |
| MAG_HUMAN | (P20916) | Myelin-associated glycoprotein precursor (Siglec-4a). |
| ML1X_HUMAN | (Q13585) | Melatonin-related receptor (H9). |
| NKX3_HUMAN | (Q9HC58) | Sodium/potassium/calcium exchanger 3. |
| NRD1_HUMAN | (P20393) | Orphan nuclear receptor NR1D1 |
| P2CD_HUMAN | (015297) | Protein phosphatase 2C delta isoform |
| PEX5_HUMAN | (P50542) | Peroxisomal targeting signal 1 receptor |
| PRLR_HUMAN | (P16471) | Prolactin receptor precursor |
| PYRG_HUMAN | (P17812) | CTP synthase |
| R1AB_CVHSA | (P59641) | Helicase (By similarity). |
| S133_HUMAN | (Q8WWT9) | Solute carrier family 13, |
| SAH3_HUMAN | (Q96HN2) | Putative adenosylhomocysteinase 3 |
| VU47_HHV6G | (P30005) | Glycoprotein U47 |

| Marker 66500, down-regulated on IMAC30 | | |
|---|---|---|
| Short name | Code | Annotation |
| AFAM_HUMAN | (P43652) | Afamin. |
| ALBU_HUMAN | (P02768) | Serum albumin. |
| AN21_HUMAN | (Q86YR6) | Ankyrin repeat domain protein 21 |
| BRL1_EBV | (P03209) | Transcription activator BRLF1. |
| CALI_HUMAN | (Q13939) | Calicin. |
| CD93_HUMAN | (Q9NPY3) | Complement component C1q receptor. |
| CDYL_HUMAN | (Q9Y232) | Chromodomain Y-like protein (CDY-like). |
| FETA_HUMAN | (P02771) | Alpha-fetoprotein. |
| FPGT_HUMAN | (014772) | Fucose-1-phosphate guanylyltransferase |
| FUT8_HUMAN | (Q9BYC5) | Alpha-(1,6)-fucosyltransferase |
| GBP5_HUMAN | (Q96PP8) | Interferon-induced guanylate-binding protein |
| GDS1_HUMAN | (P52306) | Rap1 GTPase-GDP dissociation stimulator 1 |
| GRK4_HUMAN | (P32298) | G protein-coupled receptor kinase |
| MM09_HUMAN | (P14780) | type IV collagenase. |
| MOT8_HUMAN | (P36021) | Monocarboxylate transporter 8 |
| NR42_HUMAN | (P43354) | Orphan nuclear receptor NURR1 |

| Marker 66500, down-regulated on IMAC30 | | |
|---|---|---|
| Short name | Code | Annotation |
| SNX9_HUMAN | (Q9Y5X1 | Sorting nexin 9) |
| STB2_HUMAN | (Q15833) | Syntaxin binding protein 2 |
| VP40_HHV11 | (P10210) | Gene UL26 protein. |
| VU47_HHV6U | (Q06093) | Glycoprotein U47 precursor. |

| Marker 66300, down-regulated on IMAC30 | | |
|---|---|---|
| Short name | Code | Annotation |
| 2AAB_HUMAN | (P30154) | Serine/threonine protein phosphatase 2A |
| ACDV_HUMAN | (P49748) | Acyl-CoA dehydrogenase |
| AD30_HUMAN | (Q9UKF2) | ADAM 30. |
| AN21_HUMAN | (Q86YR6) | Ankyrin repeat domain protein |
| BS69_HUMAN | (Q15326) | Adenovirus 5 E1A-binding protein |
| CDYL_HUMAN | (Q9Y232) | Chromodomain Y-like protein |
| ESR1_HUMAN | (P03372) | Estrogen receptor |
| EXON_HHV2 | (P06489) | Alkaline exonuclease |
| GDS1_HUMAN | (P52306) | Rap1 GTPase-GDP dissociation stimulator 1 |
| LAM1_HUMAN | (P20700) | Lamin B1. |
| LCP1_HUMAN | (094842) | Epidermal Langerhans cell protein LCP1. |
| MOT8_HUMAN | (P36021) | Monocarboxylate transporter 8 |
| MPP3_HUMAN | (Q13368) | MAGUK p55 subfamily member 3 |
| NLFD_HUMAN | (Q8IXH7) | Negative elongation factor C/D |
| NO56_HUMAN | (000567) | Nucleolar protein Nop56 |
| PPO2_HUMAN | (Q9UGN5) | Poly [ADP-ribose] polymerase-2 |
| R1AB_CVH22 | (Q05002) | Helicase. |
| RIB1_HUMAN | (P04843) | Ribophorin I |
| TRI4_HUMAN | (Q15650) | Thyroid receptor interacting protein 4 |
| WDR1_HUMAN | (075083) | WD-repeat protein 1 |
| YHL1_EBV | (P03181) | Hypothetical BHLF1 protein. |
| Z430_HUMAN | (Q9H8G1) | Zinc finger protein 430 |

| Marker 64860, down-regulated on IMAC30 | | |
|---|---|---|
| Short name | Code | Annotation |
| 5NTC_HUMAN | (P49902) | Cytosolic purine 5'-nucleotidase |
| AD15_HUMAN | (Q13444) | ADAM 15. |
| ALU6_HUMAN | (P39193) | Alu subfamily SP sequence |

(continued)

| Marker 64860, down-regulated on IMAC30 | | |
|---|---|---|
| Short name | Code | Annotation |
| BNA2_HUMAN | (P78348) | Amiloride-sensitive brain sodium channel |
| COE3_HUMAN | (Q9H4W6) | Transcription factor COE3 |
| DAZ4_HUMAN | (Q86SG3) | Deleted in azoospermia protein 4. |
| DOPO_HUMAN | (P09172) | Dopamine beta-monooxygenase. |
| FL01_HUMAN | (P41440) | Folate transporter 1 |
| GLSL_HUMAN | (Q9UI32) | Glutaminase, liver isoform. |
| HAS1_HUMAN | (Q92839) | Hyaluronan synthase 1 |
| HEX3_ADE12 | (P36712) | Peripentonal hexon-associated protein |
| K2H4_HUMAN | (Q9NSB2) | Keratin, type II cuticular HB4 |
| KHL3_HUMAN | (Q9UH77) | Kelch-like protein 3. |
| KLC1_HUMAN | (Q07866) | Kinesin light chain 1 (KLC 1). |
| LIGA_HUMAN | (P41214) | Ligatin |
| MGD2_HUMAN | (Q9UNF1) | Melanoma-associated antigen D2 |
| MPI2_HUMAN | (P30305) | M-phase inducer phosphatase 2 |
| NAH8_HUMAN | (Q9Y2E8) | Sodium/hydrogen exchanger 8 |
| NKX4_HUMAN | (Q8NFF2) | Sodium/potassium/calcium exchanger 4 precursor |
| NMBL_HUMAN | (Q9Y6R0) | Numb-like protein |
| NOX1_HUMAN | (Q9Y5S8) | NADPH oxidase homolog 1 |
| SEN3_HUMAN | (Q9H4L4) | Sentrin-specific protease 3 |
| SHO2_HUMAN | (Q9UQ13) | Leucine-rich repeat protein SHOC-2 |
| SOA1_HUMAN | (P35610) | Sterol O-acyltransferase 1 |
| SVC1_HUMAN | (Q9UHI7) | Solute carrier family 23, member 1 |
| T9S3_HUMAN | (Q9HD45) | Transmembrane 9 superfamily protein member 3 |
| TAI2_HUMAN | (Q8WYN3) | TGF-beta induced apoptosis protein 2 |
| TIP_HUMAN | (Q8TB96) | T-cell immunomodulatory protein |
| | | |
| Marker 8931, down-regulated on IMAC30 | | |
| Short name | Code | Annotation |
| APC2_HUMAN | (P02655) | Apolipoprotein C-II. |
| IL8_HUMAN | (P10145) | Interleukin-8. |
| PLMN_HUMAN | (P00747) | Plasminogen precursor, Activation peptide. |
| SLUR_HUMAN | (P55000) | Secreted Ly-6/uPAR related protein 1. |
| SRG1_HUMAN | (075711) | Scrapie-responsive protein 1. |
| SY08_HUMAN | (P80075) | Small inducible cytokine 8 |
| | | |

(continued)

| Marker 6635, down-regulated on IMAC30 | | |
|---|---|---|
| Short name | Code | Annotation |
| APC1_HUMAN | (P02654) | Apolipoprotein C-I. |
| CCKN_HUMAN | (P06307) | Cholecystokinin CCK58. |
| CO7R_HUMAN | (014548) | Cytochrome c oxidase subunit VIIa-related protein |
| PART_HUMAN | (Q9NPD0) | Prostate-specific and androgen regulated protein |
| RS30_HUMAN | (Q05472) | 40S ribosomal protein S30 |

| Marker 6435, down-regulated on IMAC30 | | |
|---|---|---|
| Short name | Code | Annotation |
| E306_ADE35 | (P17591) | Early E3 6.4 kDa protein. |
| GAG_HV1B1 | (P03347) | Core protein p7. |
| GAG_HV1BR | (P03348) | Core protein p7. |
| GAG_HV1H2 | (P04591) | Core protein p7. |
| GAG_HV1LW | (Q70622) | Core protein p7. |
| GAG_HV1PV | (P03350) | Core protein p7. |
| YG02_HUMAN | (060908) | Hypothetical 6.4 kDa protein A-363E6.1. |

EXAMPLE 19 POSSIBLE IDENTIFICATION OF PLASMA AND SERUM MARKERS BY ARTIFICIAL DIGESTION

In some cases the measured markers correspond to the theoretical mass of a protein in the database (for example the Swiss-Prot database for human proteins) in other cases no significant hit can be obtained (there is no protein with a theoretical mass within for example 0.2% of the identified mass of the marker). There could be a number of reasons for this: the database is not complete (databases are continually being updated), the identified mass is a protein with post-translational modifications (these modifications add to the final mass, and are never accounted for in the database), the identified mass is not a mass of a full length protein, but a fragment of a protein (there is an almost infinite number of fragments for every protein and these are not accounted for in the database). If the identified mass corresponds to a fragment of a marker, a possible identification can be obtained by so called "artificial digest" or "in silico digest" of a protein of interest. In this procedure the sequence of a protein is pasted into a digestion program. This program then cleaves the sequence into specific fragments and calculates the mass values of these fragments. Some of these mass values may correspond to the measured mass values of the markers. This fragment may be an identification of the marker. However, there are more than one hundred thousand protein sequences in the database, which in theory each produces an infinite amount of fragments. Our screening was done on blood samples (serum or plasma), therefore we focused solely on a few common blood proteins.

*Methods*

The protein sequence was obtained from the NCBI Entrez Protein Bank in fasta format.
The sequence was digested by "PeptideMass" on the ExPASy server. The following parameters were chosen:

| | |
|---|---|
| Mass value: | [M], average. |
| Enzyme: | Trypsin (higher specificity) |
| Allowed missed cleavage sites: | 5 |

We have chosen Trypsin (higher specificity) based on the assumption that most proteases in blood are members of the trypsin-familiy of proteases.
The program allows for a maximum of 5 missed cleavage sites. This means that fragments of proteins that contain more than 5 cleavage sites will not be presented. Fragments containing more than 5 cleavage sites are however possible.

*Results:*

In some cases the measured markers correspond to the theoretical mass of a protein in the database (for example the Swiss-Prot database for human proteins). We have artificially digested the following common blood proteins: Human Serum Albumin (P02768), Haptoglobin (P00738), Alpha 2 Macroglobulin (P01023), C2 Complement (P06681), C3 complement (P01024)

In some cases the measured markers correspond to the theoretical mass of a protein in the database (for example the Swiss-Prot database for human proteins).

Table 26 Possible hits of up and down-regulated plasma markers

| Human Serum Albumin | | | |
|---|---|---|---|
| Possible hits of up-regulated markers: 5920, 5900, 5330, 4460 | | | |
| mass | position | #MC | peptide sequence |
| 59307162 | 21-73 | 3 | ALVLIAFAQYLQQCPFEDHV KLVNEVTEFAKTCVADESAE NCDKSLHTLFGDK (SEQ ID NO 12) |
| 59046970 | 18629 | 5 | DAHKSEVAHRFKDLGEENFK ALVLIAFAQYLQQCPFEDHV KLVNEVTEFAK (SEQ ID NO 13) |
| 53309633 | 476-521 | 3 | CCTESLVNRRPCFSALEVDE TYVPKEFNAETFTFHADICT LSEKER (SEQ ID NO 14) |
| 44591434 | 501-538 | 5 | EFNAETFTFHADICTLSEKE RQIKKQTALVELVKHKPK (SEQ ID NO 15) |
| | | | |
| Haptoglobin | | | |
| Possible hits of up-regulated markers: 9140, 5330, 2955 | | | |
| mass | position | #MC | [peptide sequence |
| 91321617 | 298-379 | 5 | YVMLPVADQDQCIRHYEGST VPEKKTPKSPVGVQPILNEH TFCAGMSKYQEDTCYGDAGS AFAVHDLEEDTWYATGILSF DK(SEQ ID NO 16) |
| 53311397 | 298-345 | 4 | YVMLPVADQDQCIRHYEGST VPEKKTPKSPVGVQPILNEH TFCAGMSK (SEQ ID NO 1) |
| 29605032 | 252-277 | 4 | LKQKVSVNERVMPICLPSKD YAEVGR (SEQ ID NO 18) |
| | | | |
| Alpha 2 macroglobulin | | | |
| Possible hits of up-regulated markers: 10830, 8930, 5900, 5330, 2960 | | | |
| 108473269 | 935-1031 | 5 | LPPNVVEESARASVSVLGDI LGSAMQNTQNLLQMPYGCGE QNMVLFAPNIYVLDYLNETQ QLTPEVKSKAIGYLNTGYQR QLNYKHYDGSYSTFGER (SEQ ID NO 19) |

EP 1 895 302 A2

EP 1 895 302 A2

(continued)

| Alpha 2 macroglobulin | | | |
|---|---|---|---|
| Possible hits of up-regulated markers: 10830, 8930, 5900, 5330, 2960 | | | |
| 89304246 | 429-507 | 4 | SPCYGYQWVSEEHEEAHHTA YLVFSPSKSFVHLEPMSHEL PCGHTQTVQAHYILNGGTLL GLKKLSFYYLIMAKGGIVR (SEQ ID NO 20) |
| 59060717 | 94-145 | 5 | SSSNEEVMFLTVQVKGPTQE FKKRTTVMVKNEDSLVFVQT DKSIYKPGQTVK (SEQ ID NO 21) |
| 53335611 | 688-733 | 4 | MCPQLQQYEMHGPEGLRVGF YESDVMGRGHARLVHVEEPH TETVRK (SEQ ID NO 22) |
| 29612771 | 1449-1474 | 1 | VYDYYETDEFAIAEYNAPCS KDLGNA (SEQ ID NO 23) |
| | | | |
| Possible hits of down-regulated markers: 4660, 4290, | | | |
| 46600966 | 272-312 | 3 | YSDASDCHGEDSQAFCEKFS GQLNSHGCFYQQVKTKVFQL K (SEQ ID NO 24) |
| 42903594 | 1082-1122 | 1 | SSGSLLNNAIKGGVEDEVTL SAYITIALLEIPLTVTHPVV R (SEQ ID NO 25) |
| | | | |
| C2 complement | | | |
| Possible hits of up-regulated markers: 8930. 2960 | | | |
| 89241288 | 78-162 | 5 | SLSKAVCKPVRCPAPVSFEN GIYTPRLGSYPVGGNVSFEC EDGFILRGSPVRQCRPNGMW DGETAVCDNGAGHCPNPGIS LGAVR (SEQ ID NO 26) |
| 29595139 | 717-740 | 4 | APRSKVPPPRDFHINLFRMQ PWLR (SEQ ID NO 27) |
| | | | |

| Possibel hits of down-regulated markers: 6660, 4290 | | | |
|---|---|---|---|
| 66636239 | 63-124 | 5 | LCKSSGQWQTPGATRSLSKA VCKPVRCPAPVSFENGIYTP RLGSYPVGGNVSFECEDGFI LR (SEQ ID NO 28) |
| 42867414 | 167-205 | 4 | FGHGDKVRYRCSSNLVLTGS SERECQGNGVWSGTEPICR (SEQ ID NO 29) |
| | | | |
| Complement C3: | | | |
| Possible hits of up-regulated markers: 9140, 6090, 5900, 5540, 5330, 4460, 2960 | | | |
| 91396841 | 1073-1155 | 5 | APSTWLTAYVVKVFSLAVNL IAIDSQVLCGAVKWLILEKQ KPDGVFQEDAPVIHQEMIGG LRNNNEKDMALTAFVLISLQ EAK (SEQ ID NO 30) |
| 60898032 | 208-258 | 3 | AYYENSPQQVFSTEFEVKEY VLPSFEVIVEPTEKFYYIYN EKGLEVTITAR (SEQ ID NO 31) |
| 59014994 | 623-678 | 4 | ADIGCTPGSGKDYAGVFSDA GLTFTSSSGQQTAQRAELQC PQPAARRRRSVQLTEK (SEQ ID NO 32) |
| 55414229 | 156-205 | 2 | LLPVGRTVMVNIENPEGIPV KQDSLSSQNQLGVLPLSWDI PELVNMGQWK (SEQ ID NO 33) |
| 53329345 | 1304-1351 | 5 | SSKITHRIHWESASLLRSEE TKENEGFTVTAEGKGQGTLS VVTMYHAK (SEQ ID NO 34) |
| 44682808 | 137-176 | 3 | TIYTPGSTVLYRIFTVNHKL LPVGRTVMVNIENPEGIPVK (SEQ ID NO 35) |
| 44539780 | 1392-1431 | 3 | YRGDQDATMSILDISMMTGF APDTDDLKQLANGVDRYISK (SEQ ID NO 36) |
| 29583902 | 1498-1522 | 4 | EDGKLNKLCRDELCRCAEEN CFIQK (SEQ ID NO-37) |
| 29572528 | 1285-1310 | 2 | DAPDHQELNLDVSLQLPSRS SKITHR (SEQ ID NO 38) |
| | | | |
| Possibel hits of down-regulated markers: 6880, 4660, 4290 | | | |
| 68838812 | 980-1041 | 3 | ILLQGTPVAQMTEDAVDAER LKHLIVTPSGCGEQNMIGMT PTVIAVHYLDETEQWEKFGL EK (SEQ ID NO 39) |

64

(continued)

| Complement C3: | | | |
|---|---|---|---|
| Possible hits of up-regulated markers: 9140, 6090, 5900, 5540, 5330, 4460, 2960 | | | |
| 46624092 | 1204-1244 | 5 | GPLLNKFLTTAKDKNRWEDP GKQLYNVEATSYALLALLQL K (SEQ ID NO 40) |
| 46593269 | 1002-1042 | 2 | HLIVTPSGCGEQNMIGMTPT VIAVHYLDETEQWEKFGLEK R (SEQ ID NO 41) |
| 42967974 | 206-241 | 2 | IRAYYENSPQQVFSTEFEVK EYVLPSFEVIVEPTEK (SEQ ID NO 42) |

**References**

1. WO 01/25791

2. US 6,455,668

3. WO 01/36977

4. WO 99/11663

5. US 5,766,624

6. US 2001/0044113

7. Gryfe R, Swallow C, Bapat B, Redston M, Gallinger S, Couture J. Molecular biology of colorectal cancer, Curr Probl Cancer 1997 Sep-Oct;21(5):233-300.

9. Arends JW, Molecular interactions in the Vogelstein model of colorectal carcinoma. J Pathol 2000 Mar;190(4): 412-6.

Further aspects of the invention :

1. Use of a degradation product of Human Serum Albumin as a marker for cancer.

2. Use according to claim 1, wherein the degradation product is selected from the group consisting of the polypeptides having apparent molecular weight of 60500 Da, 6187 Da, 6090 Da, 5920 Da, 5906 Da, 5901 Da, 5900 Da, 5333 Da, 2363 Da, and 1687 Da.

3. Use of at least one polypeptide having an apparent molecular weight of 6187 Da, 5901 Da or 5333 Da as a marker for cancer.

4. Use according to claim 3, wherein one of the polypeptides is $\alpha$-fibrinogen protein.

5. Use according to any of claims 1-4, wherein the cancer is colorectal cancer.

6. Use of at least one marker selected from the group consisting of the polypeptides having apparent molecular weights of 66800 Da, 66500 Da, 66300 Da, 64860 Da, 60730 Da, 60500 Da, 60475 Da, 46000 Da, 45500 Da, 44300 Da, 33000 Da, 28040 Da, 28025 Da, 28010 Da, 28000 Da, 27700 Da, 19966 Da, 19900 Da, 19865 Da, 16150 Da, 15935 Da, 15580 Da, 15200 Da, 15140 Da, 14470 Da, 14300 Da, 14100 Da, 14030 Da, 13870 Da, 13747 Da, 11723 Da, 13700 Da, 13331 Da, 13265 Da, 12000 Da, 11989 Da, 11987 Da, 11900 Da, 11700 Da, 11650 Da, 11550 Da, 11500 Da, 11133 Da, 11080 Da, 10830 Da, 9950 Da, 9700 Da, 9600 Da, 9197 Da, 9140 Da, 9090 Da, 9079 Da, 8971 Da, 8940 Da, 8931 Da, 8930 Da, 8652 Da, 8580 Da, 8230 Da, 7469 Da, 7324 Da, 7023 Da, 6880 Da, 6850 Da, 6660 Da, 6650 Da, 6635 Da, 6450 Da, 6436 Da, 6435 Da, 6430 Da, 6125 Da, 6110 Da, 6090 Da, 5920 Da, 5906 Da, 5905 Da, 5900 Da, 5871 Da, 5857 Da, 5540 Da, 5360 Da, 5330 Da, 5266 Da, 5260 Da, 5234 Da, 5075 Da, 4977 Da, 4749 Da, 4660 Da, 4640 Da, 4634 Da, 4500 Da, 4480 Da, 4460 Da, 4330 Da, 4300 Da, 4290 Da, 4281 Da, 4270 Da, 4266 Da, 4264 Da, 4168 Da, 4136 Da, 4039 Da, 4024 Da, 4000 Da, 3984 Da, 3980 Da, 3960 Da, 3895 Da 3882 Da, 3878 Da, 3816 Da, 3777 Da, 3712 Da, 3680 Da, 3651 Da, 3574 Da, 3570 Da (def 2), 3487 Da, 3480 Da (def 3),3450 Da (def 1),3444 Da, 3408 Da, 3372 Da, 3280, 3275 Da, Da, 3160, Da, 2960 Da, 2955 Da, 2933 Da, 2878 Da, 2850 Da, 2840 Da, 2799 Da, 2693 Da, 2462 Da, 2450 Da, 2364 Da, 2330 Da, 2275 Da, 2230 Da, 2210 Da, 1945 Da,1930 Da, 1688 Da, 1536 Da, 1365 Da, 1256 Da, 1042 Da, 1026 Da, and 1005 Da, for the prediction of the clinical outcome, complications and mortality of an individual diagnosed with colorectal cancer.

7. A method of diagnosing colorectal cancer in a sample from a mammal, the method comprising
obtaining a sample from said mammal
assaying said sample by a quantitative detection assay and determining the intensity signal of at least one marker selected from the group consisting of the polypeptides having apparent molecular weights of

66800 Da, 66500 Da, 66300 Da, 64860 Da, 60730 Da, 60500 Da, 60475 Da, 46000 Da, 45500 Da, 44300 Da, 33000 Da, 28040 Da, 28025 Da, 28010 Da, 28000 Da, 27700 Da, 19966 Da, 19900 Da, 19865 Da, 16150 Da, 15935 Da, 15580 Da, 15200 Da, 15140 Da, 14470 Da, 14300 Da, 14100 Da, 14030 Da, 13870 Da, 13747 Da, 11723 Da, 13700 Da, 13331 Da, 13265 Da, 12000 Da, 11989 Da, 11987 Da, 11900 Da, 11700 Da, 11650 Da, 11550 Da, 11500 Da, 11133 Da, 11080 Da, 10830 Da, 9950 Da, 9700 Da, 9600 Da, 9197 Da, 9140 Da, 9090 Da, 9079 Da, 8971 Da, 8940 Da, 8931 Da, 8930 Da, 8652 Da, 8580 Da, 8230 Da, 7469 Da, 7324 Da, 7023 Da, 6880 Da, 6850 Da, 6660 Da, 6650 Da, 6635 Da, 6450 Da, 6436 Da, 6435 Da, 6430 Da, 6125 Da, 6110 Da, 6090 Da, 5920 Da, 5906 Da, 5905 Da, 5900 Da, 5871 Da, 5857 Da, 5540 Da, 5360 Da, 5330 Da, 5266 Da, 5260 Da, 5234 Da, 5075 Da, 4977 Da, 4749 Da, 4660 Da, 4640 Da, 4634 Da, 4500 Da, 4480 Da, 4460 Da, 4330 Da, 4300 Da, 4290 Da, 4281 Da, 4270 Da, 4266 Da, 4264 Da, 4168 Da, 4136 Da, 4039 Da, 4024 Da, 4000 Da, 3984 Da, 3980 Da, 3960 Da, 3895 Da 3882 Da, 3878 Da, 3816 Da, 3777 Da, 3712 Da, 3680 Da, 3651 Da, 3574 Da, 3570 Da (def 2), 3487 Da, 3480 Da (def 3),3450 Da (def 1),3444 Da, 3408 Da, 3372 Da, 3280, 3275 Da, Da, 3160, Da, 2960 Da, 2955 Da, 2933 Da, 2878 Da, 2850 Da, 2840 Da, 2799 Da, 2693 Da, 2462 Da, 2450 Da, 2364 Da, 2330 Da, 2275 Da, 2230 Da, 2210 Da, 1945 Da,1930 Da, 1688 Da, 1536 Da, 1365 Da, 1256 Da, 1042 Da, 1026 Da, and 1005 Da, comparing said intensity signal(s) with reference value(s)
identifying whether the intensity signal of at least one marker from the sample is significantly different from the reference value.

8. A method according to claim 7, wherein the reference value(s) is/are intensity signal value(s) calculated from data of said marker(s) obtained from a sample without colorectal cancer from the same mammal.

9. A method according to claim 7 or 8, wherein the reference value(s) is/are intensity signal value(s) calculated from data of said marker(s) obtained from samples from at least one normal mammal.

10. A method according to any of claims 7-9, wherein the quantitative detection assay is selected from the group consisting of immunoassay, kinetic/real-time PCR, 2D gel, protein array, gene array and other nano-technology methods.

11. A method according to any of claims 7-10, wherein the signal is selected from the group consisting of fluorescence signal, mass spectrometry images, radioactivity and enzyme activity.

12. A method according to any of claims 7-11, wherein the intensity signal for at least one of the markers 60500 Da, 60730 Da, 60475 Da, 33000 Da, 19966 Da, 19900 Da, 19865 Da, 11900 Da, 16150 Da, 15935 Da, 15200 Da, 14470 Da, 14300 Da, 14100 Da, 14030 Da, 13870 Da, 13747 Da, 11987 Da, 11723 Da, 11700 Da, 11650 Da, 11550 Da, 11500 Da, 11080 Da, 10830 Da, 9950 Da, 9140 Da, 8930 Da, 7469 Da, 6850 Da, 6125 Da, 6110 Da, 6090 Da, 5905 Da, 5920 Da, 5906 Da, 5900 Da, 5871 Da, 5857 Da, 5540 Da, 5330 Da, 5266 Da, 5260 Da, 5234 Da, 4977 Da, 4480 Da, 4460 Da, 4281 Da, 4270 Da, 4266 Da, 4264 Da, 4136 Da, 4039 Da, 4024 Da, 3895 Da, 3882 Da, 3878 Da, 3712 Da, 3651 Da, 3570 Da, 3574 Da, 3487 Da, 3480 Da, 3450 Da, 3444 Da, 3408 Da, 3372 Da, 3275 Da, 2960 Da, 2955 Da, 2933 Da, 2878 Da, 2850 Da, 2840 Da, 2799 Da, 2693 Da, 2462 Da, 2364 Da, and 1688 Da is increased.

13. A method according to any of claims 7-12, wherein the intensity signal for at least one of the markers 66800 Da, 66500 Da, 66300 Da, 64860 Da, 46000 Da, 45500 Da, 44300 Da, 28040 Da, 28025 Da, 28010 Da, 28000 Da, 27700 Da, 15580 Da, 15140 Da, 13700 Da, 13331 Da 13265 Da, 12000 Da, 11989 Da, 11133 Da, 9700 Da, 9600 Da, 9197 Da, 9090 Da, 9079 Da, 8971 Da, 8940 Da, 8931 Da, 8652 Da, 8580 Da, 8230 Da, 7324 Da, 7023 Da, 6880 Da, 6660 Da, 6650 Da, 6635 Da, 6450 Da, 6436 Da, 6435 Da,6430 Da, 5360 Da, 5075 Da, 4749 Da, 4660 Da, 4640 Da, 4634 Da, 4500 Da, 4480 Da, 4330 Da, 4300 Da, 4290 Da, 4168 Da, 4000 Da, 3984 Da, 3980 Da , 3960 Da, 3816 Da, 3777 Da, 3680 Da, 3280 Da, 3160 Da, 2450 Da, 2330 Da, 2275 Da, 2230 Da, 2210, 1945 Da, 1930 Da 1536 Da, 1365 Da, 1256 Da, 1042 Da, 1026 Da, and 1005 Da is decreased.

14. A method according to any of claims 7-13, wherein the sample is selected from the group consisting of blood, serum, plasma and a tissue sample

15. A method of diagnosing colorectal cancer by means of a sample from a mammal, the method comprising obtaining a serum sample from said mammal
assaying said sample by a quantitative detection assay and determining the intensity signal of at least one marker selected from the group consisting of the polypeptides having apparent molecular weights of
66500 Da, 60500 Da, 46000 Da, 45500 Da, 44300 Da, 28040 Da, 27700 Da, 33000 Da, 19900 Da, 16150 Da, 15935 Da, 15580 Da, 15200 Da, 15200 Da, 13700 Da, 11900 Da, 11700 Da, 11650 Da, 11550 Da, 11500 Da, 11080 Da, 10830 Da, 9140 Da, 8940 Da, 8930 Da, 8230 Da, 6880 Da, 6650 Da, 6660 Da, 6450 Da, 6430 Da, 6125 Da, 6110 Da, 6090 Da, 5920 Da, 5900 Da, 5540 Da, 5330 Da, 5260 Da, 4660 Da, 4640 Da, 4460 Da, 4330 Da, 4300 Da, 4290 Da, 4000 Da, 3980 Da , 3960 Da, 3680 Da, 3280 Da, 3275 Da, Da, 3160 Da, 2955 Da, 2450 Da, and 1536 Da,
comparing said intensity signal(s) with reference value(s)
identifying whether the intensity signal of at least one marker from the sample is significantly different from the reference value for said marker.

16. A method of diagnosing colorectal cancer in a sample from a mammal, the method comprising
obtaining a tissue sample from said mammal
assaying said sample by a quantitative detection assay and determining the intensity signal of at least one marker selected from the group consisting of the polypeptides having apparent molecular weights of
15140 Da, 11989 Da, 11987 Da, 9700 Da, 9600 Da, 9197 Da, 9079 Da, 8971 Da, 8652 Da, 8580 Da, 7324 Da, 7023 Da, 5871 Da, 5857 Da, 5360 Da, 5234 Da, 5075 Da, 4749 Da, 4634 Da, 4281 Da, 4266 Da, 4168 Da, 4039 Da, 4024 Da, 3984 Da, 3878 Da, 3777 Da, 3712 Da, 3651 Da, 3574 Da, 3487 Da, 3444 Da, 3408 Da, 3372 Da, 2933 Da, 2878 Da, 2840 Da, 2799 Da, 2693 Da, 2462 Da, 2364 Da, 2330 Da, 1930 Da, 1688 Da, 1365 Da, 1256 Da, 1042 Da, 1026 Da, and 1005 Da
comparing said intensity signal(s) with reference value(s)
identifying whether the intensity signal of at least one marker from the sample is significantly different from the reference value for said marker.

17. A method of diagnosing colorectal cancer in a sample from a mammal, the method comprising
obtaining a plasma sample from said mammal
assaying said sample by a quantitative detection assay and determining the intensity signal of at least one marker selected from the group consisting of the polypeptides having apparent molecular weights of
66800 Da, 66500 Da, 66300 Da, 64860 Da, 60730 Da, 60475 Da, 19966 Da, 19865 Da, 14470 Da, 14300 Da, 14100 Da, 14030 Da, 13870 Da, 13747 Da, 11723 Da, 9950 Da, 8931 Da, 7469 Da, 6635 Da, 6435 Da, 5905 Da, 5266 Da, 4977 Da, 4480 Da, 4136 Da, and 3895 Da,
comparing said intensity signal(s) with reference value(s)
identifying whether the intensity signal of at least one marker from the sample is significantly different from the reference value for said marker.

18. A method according to any of claims 7-17, wherein the intensity signal for at least one of the markers 60500 Da, 19900 Da, 11080 Da, 10830 Da, 9140 Da, 8930 Da, 6110 Da, 6090 Da, 5920 Da, 5900 Da, 5540 Da, 5330 Da, 5260 Da, 4460 Da and 2960 Da is increased and the intensity signal for at least one of markers 66500 Da, 44300 Da, 28040 Da, 27700 Da, 15580 Da, 13700 Da, 6880 Da, 6660 Da, 6430 Da, 4660 Da, 4640 Da, 4330 Da, 4300 Da, 4290 Da, 4000 Da, 3980 Da , 3960 Da, 3680 Da, 3280 Da, and 3160 Da is decreased when assaying a serum sample on IMAC30 chip (Ciphergen).

19. A method according to any of claims 7-18, wherein the intensity signal for at least one of the markers 11900 Da, 11700 Da, 11650 Da, 11550 Da and 11500 Da is increased and the intensity signal for at least one of the markers 46000 Da, 45500 Da, 8940 Da, 8230 Da, 6650 Da, and 6450 Da is decreased when assaying a serum sample on H50 protein chip.

20. A method according to any of claims 7-19, wherein the intensity signal for at least one of the markers 15200 Da, 6125 Da, 5900 Da, 3275 Da and 2955 Da is increased and the intensity signal for at least one of the markers 4290 Da, 2450 Da, and 1536 Da is decreased when assaying a serum sample on CM10 protein chip.

21. A method according to any of claims 7-20, wherein the intensity signal for at least one of the markers 33000 Da, 16150 Da, 15935 Da, and 15200 Da is increased when assaying a serum sample on Sax2protein chip.

22. A method according to any of claims 7-21, wherein the intensity signal for at least one of the markers 5857 Da, 4264 Da, 3878 Da, 3712 Da, 3651 Da, 3574 Da, 3487 Da, 3444 Da, 3372 Da and 1688 Da is increased and the intensity signal for at least one of the markers 9700 Da, 8652 Da, 8652 Da, 8580 Da, 7023 Da, 5360 Da, 4168 Da, 1365 Da, 1256 Da, 1042 Da, 1026 Da, and 1005 Da is decreased when assaying a tissue sample on NP20 protein chip.

23. A method according to any of claims 7-22, wherein the intensity signal for at least one of the markers 11987 Da, 5871 Da, 5234 Da, 4281 Da, 4266 Da, 4039 Da, 4024 Da, 3408 Da, 2933 Da, 2878 Da, 2840 Da, 2799 Da, 2693 Da, 2462 Da, and 2364 Da is increased and the intensity signal for at least one of the markers 15140 Da, 11989 Da, 9600 Da, 9197 Da, 9079 Da, 8971 Da, 7324 Da, 5075 Da, 4749 Da, 4634 Da, 3984 Da, 3777 Da, 2330 Da, and 1930 Da is decreased when assaying a tissue sample on Sax2protein chip.

24. A method according to claim 7, wherein the intensity signal(s) for at least one of the markers 5340 Da and 5906 Da is/are increased and the intensity signal(s) for at least one of the markers 3980 Da, 6880 Da, and 28010 is/are decreased when assaying a serum sample on a IMac30 chip.

25. A method of diagnosing colorectal cancer by means of a sample from a mammal, the method comprising

a) preparing a normalized protein expression data set from the sample, wherein the expression data set comprises a plurality of expression intensities of proteins on at least one protein chip,

b) selecting one marker from the normalized protein expression data set obtained in a), from the group consisting of the polypeptides having apparent molecular weight of
66800 Da, 66500 Da, 66300 Da, 64860 Da, 60730 Da, 60500 Da, 60475 Da, 46000 Da, 45500 Da, 44300 Da, 33000 Da, 28040 Da, 28025 Da, 28010 Da, 28000 Da, 27700 Da, 19966 Da, 19900 Da, 19865 Da, 16150 Da, 15935 Da, 15580 Da, 15200 Da, 15140 Da, 14470 Da, 14300 Da, 14100 Da, 14030 Da, 13870 Da, 13747 Da, 11723 Da, 13700 Da, 13331 Da, 13265 Da, 12000 Da 11989 Da, 11987 Da, 11900 Da, 11700 Da, 11650 Da, 11550 Da, 11500 Da, 11133 Da, 11080 Da, 10830 Da, 9950 Da, 9700 Da, 9600 Da, 9197 Da, 9140 Da, 9090 Da, 9079 Da, 8971 Da, 8940 Da, 8931 Da, 8930 Da, 8652 Da, 8580 Da, 8230 Da, 7469 Da, 7324 Da, 7023 Da, 6880 Da, 6850 Da, 6660 Da, 6650 Da, 6635 Da, 6450 Da, 6436 Da, 6435 Da, 6430 Da, 6125 Da, 6110 Da, 6090 Da, 5920 Da, 5906 Da, 5905 Da, 5900 Da, 5871 Da, 5857 Da, 5540 Da, 5360 Da, 5330 Da, 5266 Da, 5260 Da, 5234 Da, 5075 Da, 4977 Da, 4749 Da, 4660 Da, 4640 Da, 4634 Da, 4500 Da, 4480 Da, 4460 Da, 4330 Da, 4300 Da, 4290 Da, 4281 Da, 4270 Da, 4266 Da, 4264 Da, 4168 Da, 4136 Da, 4039 Da, 4024 Da, 4000 Da, 3984 Da, 3980 Da, 3960 Da, 3895 Da 3882 Da, 3878 Da, 3816 Da, 3777 Da, 3712 Da, 3680 Da, 3651 Da, 3574 Da, 3570 Da (def 2), 3487 Da, 3480 Da (def 3),3450 Da (def 1),3444 Da, 3408 Da, 3372 Da, 3280, 3275 Da, Da, 3160, Da, 2960 Da, 2955 Da, 2933 Da, 2878 Da, 2850 Da, 2840 Da, 2799 Da, 2693 Da, 2462 Da, 2450 Da, 2364 Da, 2330 Da, 2275 Da, 2230 Da, 2210 Da, 1945 Da,1930 Da, 1688 Da, 1536 Da, 1365 Da, 1256 Da, 1042 Da, 1026 Da, and 1005 Da,

c) setting a weight for said marker

d) multiplying the intensity of said marker with the weight of said marker

e) repeating steps b) - d) for a number of markers and calculating the sum of the multiplications

f) comparing the sum with a cut-off value

g) readjusting the weight for each marker for obtaining the highest sensitivity and specificity.

26. A method according to claim 25, wherein the weight for each marker is determined/set by a number between -0.9 and +0.9, that number resulting in the highest sensitivity and specificity.

27. A method according to claim 25, wherein the determination is based on the following algorithm:

Give the selected markers weights between -0.9 and 0.9, i.e. marker A, weight a, marker B, weight b, marker C, weight c, marker D, weight d and marker N, weight n;

get intensities of A, B, C, D,..N markers in the following order: A m/z, B m/z, C m/z, D m/z, N .. m/z;

multiply the first intensity with weight a;
multiply the second intensity with weight b;
multiply the third intensity with weight c;
multiply the fourth intensity with weight d;
multiply the n intensities with weight n; and

calculate the sum of the above multiplications;

if sum lower than cutoff value => sample is negative for colon cancer, and
if sum higher than cutoff value => sample is positive for colon cancer.

28. A computer system for monitoring the likelihood of a mammal having colorectal cancer, the computer system comprising:

a) storage means for electronically storing data,

b) processing means for storing input data from a mass spectrometer,

b) input means for interfacing between an mass spectrometer and the computer system, and

d) an interface between a user and the computer system,

wherein the processing means determines the likelihood of colorectal cancer by applying the following algorithm:

Give the selected markers weights between -0.9 and 0.9, i.e. marker A, weight a, marker B, weight b, marker C, weight c, marker D, weight d and marker N, weight n;

get intensities of A, B, C, D,..N markers in the following order: A m/z, B m/z, C m/z, D m/z, N .. m/z;

multiply the first intensity with weight a;
multiply the second intensity with weight b;
multiply the third intensity with weight c;
multiply the fourth intensity with weight d;
multiply the n intensities with weight n; and
calculate the sum of the above multiplications;

if sum lower than cutoff value => sample is negative for colon cancer, and
if sum higher than cutoff value => sample is positive for colon cancer.

29. A kit comprising:

- a first antibody including a portion bound to a solid phase and a region which specifically binds to alpha-fetoprotein,
- a second antibody including a region which specifically binds to alpha-fetoprotein and a portion which has a label, and
- optionally a reference protein.

30. A kit comprising:

- a first antibody including a portion bound to a solid phase and a region which specifically binds to alpha-fibrinogen,
- a second antibody including a region which specifically binds to alpha-fibrinogen and a portion which has a label, and
- optionally a reference protein.

31. A kit comprising:

- a first antibody including a portion bound to a solid phase and a region which specifically binds to human serum albumin (HSA) or fragments of HSA,
- a second antibody including a region which specifically binds to human serum albumin (HSA) or fragments of HSA and a portion which has a label, and
- optionally a reference protein.

32. A kit according to claims 29-31, wherein a combination of two or more of the proteins are detected.

## Claims

1. Use of at least one polypeptide marker selected from the group consisting of and having the molecular weight of +/- 0.2%:
   3895 Da, 3980 Da, 5906 Da, 6880 Da, 8940 Da, 13747 Da, and 28040 Da,
   for the prediction of the clinical outcome, complications and mortality of an individual diagnosed with colorectal cancer in a sample obtained from a mammal.

2. Use according to claim 1, further comprising use of at least one additional marker selected from the group consisting of the polypeptides having molecular weights of +/-0.2%:
   2955 Da, 4300 Da, 5075 Da, 6850 Da, and 15140 Da.

3. Use of at least one polypeptide marker selected from the group consisting of and having the molecular weight of +/- 0.2%:
   3895 Da, 8940 Da, 13747 Da, and 28040 Da,
   for the prediction of the clinical outcome, complications and mortality of an individual diagnosed with colorectal cancer in a sample obtained from a mammal.

4. Use according to claim 3, further comprising use of at least one additional marker selected from the group consisting of the polypeptides having molecular weights of +/-0.2%:
2955 Da, 3980 Da, 4300 Da, 5075 Da, 5906 Da, 6850 Da, 6880 Da, and 15140 Da.

5. A method of diagnosing colorectal cancer in a sample from a mammal, the method comprising assaying a sample obtained from said mammal by a quantitative detection assay and determining the intensity signal of at least one marker selected from the group consisting of the polypeptides having the molecular weight +/- 0.2%:
3895 Da, 3980 Da, 5906 Da, 6880 Da, 8940 Da, 13747 Da, and 28040 Da;
comparing said intensity signal with a reference value; and identifying whether the intensity signal of at least one marker from the sample is significantly different from the reference value for said marker.

6. A method according to claim 5, wherein the marker(s) is further combined with at least one additional marker selected from the group consisting of the polypeptides having molecular weights of +/- 0.2%:
2955 Da, 4300 Da, 5075 Da, 6850 Da, and 15140 Da;
comparing said intensity signal with a reference value; and identifying whether the intensity signal of at least one marker from the sample is significantly different from the reference value for said marker.

7. A method of diagnosing colorectal cancer in a sample from a mammal, the method comprising assaying a sample obtained from said mammal by a quantitative detection assay and determining the intensity signal of at least one marker selected from the group consisting of the polypeptides having the molecular weight +/- 0.2%:
3895 Da, 8940 Da, 13747 Da, and 28040 Da;
comparing said intensity signal with a reference value; and identifying whether the intensity signal of at least one marker from the sample is significantly different from the reference value for said marker.

8. A method according to claim 7, wherein the marker(s) is further combined with at least one additional marker selected from the group consisting of the polypeptides having molecular weights of +/- 0.2%:
2955 Da, 3980 Da, 4300 Da, 5075 Da, 5906 Da, 6850 Da, 6880 Da, and 15140 Da;
comparing said intensity signal with a reference value; and identifying whether the intensity signal of at least one marker from the sample is significantly different from the reference value for said marker.

9. A method according to any of the claims 5-8, wherein the reference value(s) is/are intensity signal value(s) calculated from data of said marker(s) obtained from a sample without colorectal cancer from the same mammal.

10. A method according to any of the claims 5-8, wherein the reference value(s) is/are intensity signal value(s) calculated from data of said marker(s) obtained from samples from at least one normal mammal.

11. A method according to any of claims 5 - 10, wherein the quantitative detection assay is selected from the group consisting of immunoassay, kinetic/real-time PCR, protein array, gene array and other nano-technology methods.

12. A method according to any of claims 5-11, wherein the signal is selected from the group consisting of fluorescence signal, mass spectrometry images, radioactivity and enzyme activity.

**Average intensity values of possible biomarkers**

Fig. 1

Fig.2

EP 1 895 302 A2

**Average intensity of possible biomarkers in serum**

Fig.3

EP 1 895 302 A2

Fig. 4

Fig. 5

Cancer Serum    Normal Serum

Fig. 6

Fig. 7

Fig. 8

Cancer Serum    Normal Serum

Fig. 9

Fig. 10

Cancer Serum     Normal Serum

Fig. 11

Cancer Serum          Normal Serum

Fig. 12

Fig. 13

Fig. 14

EP 1 895 302 A2

Fig. 15

Fig. 16

Fig. 17

Fig. 18

Fig. 19

Fig. 20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0125791 A2
- US 6455668 B
- WO 0055633 A
- US 20010044113 A

- WO 9911663 A
- WO 0125791 A
- WO 0136977 A
- US 5766624 B

**Non-patent literature cited in the description**

- **GRYFE R ; SWALLOW C ; BAPAT B ; REDSTON M ; GALLINGER S ; COUTURE J.** Molecular biology of colorectal cancer. *Curr Probl Cancer,* September 1997, vol. 21 (5), 233-300

- **ARENDS JW.** Molecular interactions in the Vogelstein model of colorectal carcinoma. *J Pathol,* March 2000, vol. 190 (4), 412-6

SEQUENCE LISTING

<110> Colotech
      Raskov, Hans Henrik
      Albrethsen, Jacob
      Gammeltoft, Steen
      Bøgebo, Rikke Maria

<120> A method for detection of colorectal
   cancers in human samples

<130> P32733PC01

<140> PCT/DK04/000263
<141> 2004-04-07

<150> DKPA200300541
<151> 2003-04-08

<150> DKPA200301085
<151> 2003-07-16

<160> 42

<170> FastSEQ for Windows Version 4.0

<210> 1
<211> 19
<212> PRT
<213> Homo sapiens

<220>
<221> PEPTIDE
<222> (0)...(0)
<223> Fragment of human serum albumin/alpha-fetoprotein

<400> 1
Phe Leu Gly Met Phe Leu Tyr Glu Tyr Ala Arg Arg His Pro Asp Tyr
 1               5                   10                  15
Ser Val Val

<210> 2
<211> 11
<212> PRT
<213> Homo sapiens

<220>
<221> PEPTIDE
<222> (0)...(0)

```
<400> 2
Thr His Leu Ala Pro Tyr Ser Asp Glu Leu Arg
 1               5                   10


<210> 3
<211> 11
<212> PRT
<213> Homo sapiens

<220>
<221> PEPTIDE
<222> (0)...(0)

<400> 3
Leu Ser Pro Leu Gly Glu Glu Met Arg Asp Arg
 1               5                   10


<210> 4
<211> 15
<212> PRT
<213> Homo sapiens

<220>
<221> PEPTIDE
<222> (0)...(0)

<400> 4
Gln Lys Val Glu Pro Leu Arg Ala Glu Leu Gln Glu Gly Ala Arg
 1               5                   10                  15


<210> 5
<211> 28
<212> PRT
<213> Homo sapiens

<220>
<221> PEPTIDE
<222> (0)...(0)

<400> 5
Asp Leu Ala Thr Val Tyr Val Asp Val Leu Lys Asp Ser Gly Arg Asp
 1               5                   10                  15
Tyr Val Ser Gln Phe Glu Gly Ser Ala Leu Gly Lys
                20                  25


<210> 6
<211> 54
<212> PRT
<213> Homo sapiens

<220>
<221> PEPTIDE
<222> (0)...(0)

<400> 6
```

```
Ser Ser Ser Tyr Ser Lys Gln Phe Thr Ser Ser Thr Ser Tyr Asn Arg
1               5                   10                  15
Gly Asp Ser Thr Phe Glu Ser Lys Ser Tyr Lys Met Ala Asp Glu Ala
            20                  25                  30
Gly Ser Glu Ala Asp His Glu Gly Thr His Ser Thr Lys Arg Gly His
        35                  40                  45
Ala Lys Ser Arg Pro Val
        50
```

```
<210> 7
<211> 49
<212> PRT
<213> Homo sapiens

<220>
<221> PEPTIDE
<222> (0)...(0)

<400> 7
Gly Ile Phe Thr Asn Thr Lys Glu Ser Ser Ser His His Pro Gly Ile
1               5                   10                  15
Ala Glu Phe Pro Ser Arg Gly Lys Ser Ser Ser Tyr Ser Lys Gln Phe
            20                  25                  30
Thr Ser Ser Thr Ser Tyr Asn Arg Gly Asp Ser Thr Phe Glu Ser Lys
        35                  40                  45
Ser
```

```
<210> 8
<211> 49
<212> PRT
<213> Homo sapiens

<220>
<221> PEPTIDE
<222> (0)...(0)

<400> 8
Ser Gly Ile Phe Thr Asn Thr Lys Glu Ser Ser Ser His His Pro Gly
1               5                   10                  15
Ile Ala Glu Phe Pro Ser Arg Gly Lys Ser Ser Ser Tyr Ser Lys Gln
            20                  25                  30
Phe Thr Ser Ser Thr Ser Tyr Asn Arg Gly Asp Ser Thr Phe Glu Ser
        35                  40                  45
Lys
```

```
<210> 9
<211> 57
<212> PRT
<213> Homo sapiens

<220>
<221> PEPTIDE
<222> (0)...(0)
```

```
<400> 9
Gly Ser Glu Ser Gly Ile Phe Thr Asn Thr Lys Glu Ser Ser Ser His
1               5                   10                  15
His Pro Gly Ile Ala Glu Phe Pro Ser Arg Gly Lys Ser Ser Ser Tyr
            20                  25                  30
Ser Lys Gln Phe Thr Ser Ser Thr Ser Tyr Asn Arg Gly Asp Ser Thr
        35                  40                  45
Phe Glu Ser Lys Ser Tyr Lys Met Ala
    50                  55


<210> 10
<211> 8
<212> PRT
<213> Homo sapiens

<220>
<221> PEPTIDE
<222> (0)...(0)

<400> 10
Phe Leu Gly Met Phe Leu Tyr Glu
1               5


<210> 11
<211> 19
<212> PRT
<213> Homo sapiens

<220>
<221> PEPTIDE
<222> (0)...(0)

<400> 11
Phe Leu Gly Met Phe Leu Tyr Glu Tyr Ala Arg Arg His Pro Asp Tyr
1               5                   10                  15
Ser Val Val


<210> 12
<211> 53
<212> PRT
<213> Homo sapiens

<220>
<221> PEPTIDE
<222> (0)...(0)

<400> 12
Ala Leu Val Leu Ile Ala Phe Ala Gln Tyr Leu Gln Gln Cys Pro Phe
1               5                   10                  15
Glu Asp His Val Lys Leu Val Asn Glu Val Thr Glu Phe Ala Lys Thr
            20                  25                  30
Cys Val Ala Asp Glu Ser Ala Glu Asn Cys Asp Lys Ser Leu His Thr
        35                  40                  45
Leu Phe Gly Asp Lys
    50
```

```
<210> 13
<211> 51
<212> PRT
<213> Homo sapiens

<220>
<221> PEPTIDE
<222> (0)...(0)

<400> 13
Asp Ala His Lys Ser Glu Val Ala His Arg Phe Lys Asp Leu Gly Glu
1               5                   10                  15
Glu Asn Phe Lys Ala Leu Val Leu Ile Ala Phe Ala Gln Tyr Leu Gln
                20                  25                  30
Gln Cys Pro Phe Glu Asp His Val Lys Leu Val Asn Glu Val Thr Glu
        35                  40                  45
Phe Ala Lys
        50


<210> 14
<211> 46
<212> PRT
<213> Homo sapiens

<220>
<221> PEPTIDE
<222> (0)...(0)

<400> 14
Cys Cys Thr Glu Ser Leu Val Asn Arg Arg Pro Cys Phe Ser Ala Leu
1               5                   10                  15
Glu Val Asp Glu Thr Tyr Val Pro Lys Glu Phe Asn Ala Glu Thr Phe
                20                  25                  30
Thr Phe His Ala Asp Ile Cys Thr Leu Ser Glu Lys Glu Arg
            35                  40                  45


<210> 15
<211> 38
<212> PRT
<213> Homo sapiens

<220>
<221> PEPTIDE
<222> (0)...(0)

<400> 15
Glu Phe Asn Ala Glu Thr Phe Thr Phe His Ala Asp Ile Cys Thr Leu
1               5                   10                  15
Ser Glu Lys Glu Arg Gln Ile Lys Lys Gln Thr Ala Leu Val Glu Leu
                20                  25                  30
Val Lys His Lys Pro Lys
            35


<210> 16
```

```
<211> 82
<212> PRT
<213> Homo sapiens

<220>
<221> PEPTIDE
<222> (0)...(0)

<400> 16
Tyr Val Met Leu Pro Val Ala Asp Gln Asp Gln Cys Ile Arg His Tyr
1               5                   10                  15
Glu Gly Ser Thr Val Pro Glu Lys Lys Thr Pro Lys Ser Pro Val Gly
            20                  25                  30
Val Gln Pro Ile Leu Asn Glu His Thr Phe Cys Ala Gly Met Ser Lys
        35                  40                  45
Tyr Gln Glu Asp Thr Cys Tyr Gly Asp Ala Gly Ser Ala Phe Ala Val
    50                  55                  60
His Asp Leu Glu Glu Asp Thr Trp Tyr Ala Thr Gly Ile Leu Ser Phe
65                  70                  75                  80
Asp Lys


<210> 17
<211> 48
<212> PRT
<213> Homo sapiens

<220>
<221> PEPTIDE
<222> (0)...(0)

<400> 17
Tyr Val Met Leu Pro Val Ala Asp Gln Asp Gln Cys Ile Arg His Tyr
1               5                   10                  15
Glu Gly Ser Thr Val Pro Glu Lys Lys Thr Pro Lys Ser Pro Val Gly
            20                  25                  30
Val Gln Pro Ile Leu Asn Glu His Thr Phe Cys Ala Gly Met Ser Lys
        35                  40                  45


<210> 18
<211> 26
<212> PRT
<213> Homo sapiens

<220>
<221> PEPTIDE
<222> (0)...(0)

<400> 18
Leu Lys Gln Lys Val Ser Val Asn Glu Arg Val Met Pro Ile Cys Leu
1               5                   10                  15
Pro Ser Lys Asp Tyr Ala Glu Val Gly Arg
            20                  25


<210> 19
<211> 97
```

```
<212> PRT
<213> Homo sapiens

<220>
<221> PEPTIDE
<222> (0)...(0)

<400> 19
Leu Pro Pro Asn Val Val Glu Glu Ser Ala Arg Ala Ser Val Ser Val
1               5                   10                  15
Leu Gly Asp Ile Leu Gly Ser Ala Met Gln Asn Thr Gln Asn Leu Leu
            20                  25                  30
Gln Met Pro Tyr Gly Cys Gly Glu Gln Asn Met Val Leu Phe Ala Pro
        35                  40                  45
Asn Ile Tyr Val Leu Asp Tyr Leu Asn Glu Thr Gln Gln Leu Thr Pro
    50                  55                  60
Glu Val Lys Ser Lys Ala Ile Gly Tyr Leu Asn Thr Gly Tyr Gln Arg
65                  70                  75                  80
Gln Leu Asn Tyr Lys His Tyr Asp Gly Ser Tyr Ser Thr Phe Gly Glu
                85                  90                  95
Arg


<210> 20
<211> 79
<212> PRT
<213> Homo sapiens

<220>
<221> PEPTIDE
<222> (0)...(0)

<400> 20
Ser Pro Cys Tyr Gly Tyr Gln Trp Val Ser Glu Glu His Glu Glu Ala
1               5                   10                  15
His His Thr Ala Tyr Leu Val Phe Ser Pro Ser Lys Ser Phe Val His
            20                  25                  30
Leu Glu Pro Met Ser His Glu Leu Pro Cys Gly His Thr Gln Thr Val
        35                  40                  45
Gln Ala His Tyr Ile Leu Asn Gly Gly Thr Leu Leu Gly Leu Lys Lys
        50                  55                  60
Leu Ser Phe Tyr Tyr Leu Ile Met Ala Lys Gly Gly Ile Val Arg
65                  70                  75


<210> 21
<211> 52
<212> PRT
<213> Homo sapiens

<220>
<221> PEPTIDE
<222> (0)...(0)

<400> 21
Ser Ser Ser Asn Glu Glu Val Met Phe Leu Thr Val Gln Val Lys Gly
1               5                   10                  15
Pro Thr Gln Glu Phe Lys Lys Arg Thr Thr Val Met Val Lys Asn Glu
```

77

```
                    20                  25                  30
Asp Ser Leu Val Phe Val Gln Thr Asp Lys Ser Ile Tyr Lys Pro Gly
        35                  40                  45
Gln Thr Val Lys
        50


<210> 22
<211> 46
<212> PRT
<213> Homo sapiens

<220>
<221> PEPTIDE
<222> (0)...(0)

<400> 22
Met Cys Pro Gln Leu Gln Gln Tyr Glu Met His Gly Pro Glu Gly Leu
1               5                   10                  15
Arg Val Gly Phe Tyr Glu Ser Asp Val Met Gly Arg Gly His Ala Arg
            20                  25                  30
Leu Val His Val Glu Glu Pro His Thr Glu Thr Val Arg Lys
        35                  40                  45


<210> 23
<211> 26
<212> PRT
<213> Homo sapiens

<220>
<221> PEPTIDE
<222> (0)...(0)

<400> 23
Val Tyr Asp Tyr Tyr Glu Thr Asp Glu Phe Ala Ile Ala Glu Tyr Asn
1               5                   10                  15
Ala Pro Cys Ser Lys Asp Leu Gly Asn Ala
            20                  25


<210> 24
<211> 41
<212> PRT
<213> Homo sapiens

<220>
<221> PEPTIDE
<222> (0)...(0)

<400> 24
Tyr Ser Asp Ala Ser Asp Cys His Gly Glu Asp Ser Gln Ala Phe Cys
1               5                   10                  15
Glu Lys Phe Ser Gly Gln Leu Asn Ser His Gly Cys Phe Tyr Gln Gln
            20                  25                  30
Val Lys Thr Lys Val Phe Gln Leu Lys
        35                  40
```

```
<210> 25
<211> 41
<212> PRT
<213> Homo sapiens

<220>
<221> PEPTIDE
<222> (0)...(0)

<400> 25
Ser Ser Gly Ser Leu Leu Asn Asn Ala Ile Lys Gly Gly Val Glu Asp
1               5                   10                  15
Glu Val Thr Leu Ser Ala Tyr Ile Thr Ile Ala Leu Leu Glu Ile Pro
            20                  25                  30
Leu Thr Val Thr His Pro Val Val Arg
        35                  40


<210> 26
<211> 62
<212> PRT
<213> Homo sapiens

<220>
<221> PEPTIDE
<222> (0)...(0)

<400> 26
Leu Cys Lys Ser Ser Gly Gln Trp Gln Thr Pro Gly Ala Thr Arg Ser
1               5                   10                  15
Leu Ser Lys Ala Val Cys Lys Pro Val Arg Cys Pro Ala Pro Val Ser
            20                  25                  30
Phe Glu Asn Gly Ile Tyr Thr Pro Arg Leu Gly Ser Tyr Pro Val Gly
        35                  40                  45
Gly Asn Val Ser Phe Glu Cys Glu Asp Gly Phe Ile Leu Arg
    50                  55                  60


<210> 27
<211> 24
<212> PRT
<213> Homo sapiens

<220>
<221> PEPTIDE
<222> (0)...(0)

<400> 27
Ala Pro Arg Ser Lys Val Pro Pro Pro Arg Asp Phe His Ile Asn Leu
1               5                   10                  15
Phe Arg Met Gln Pro Trp Leu Arg
            20


<210> 28
<211> 62
<212> PRT
<213> Homo sapiens
```

```
<220>
<221> PEPTIDE
<222> (0)...(0)

<400> 28
Leu Cys Lys Ser Ser Gly Gln Trp Gln Thr Pro Gly Ala Thr Arg Ser
1               5                   10                  15
Leu Ser Lys Ala Val Cys Lys Pro Val Arg Cys Pro Ala Pro Val Ser
            20                  25                  30
Phe Glu Asn Gly Ile Tyr Thr Pro Arg Leu Gly Ser Tyr Pro Val Gly
            35                  40                  45
Gly Asn Val Ser Phe Glu Cys Glu Asp Gly Phe Ile Leu Arg
    50                  55                  60


<210> 29
<211> 39
<212> PRT
<213> Homo sapiens

<220>
<221> PEPTIDE
<222> (0)...(0)

<400> 29
Phe Gly His Gly Asp Lys Val Arg Tyr Arg Cys Ser Ser Asn Leu Val
1               5                   10                  15
Leu Thr Gly Ser Ser Glu Arg Glu Cys Gln Gly Asn Gly Val Trp Ser
            20                  25                  30
Gly Thr Glu Pro Ile Cys Arg
            35


<210> 30
<211> 83
<212> PRT
<213> Homo sapiens

<220>
<221> PEPTIDE
<222> (0)...(0)

<400> 30
Ala Pro Ser Thr Trp Leu Thr Ala Tyr Val Val Lys Val Phe Ser Leu
1               5                   10                  15
Ala Val Asn Leu Ile Ala Ile Asp Ser Gln Val Leu Cys Gly Ala Val
            20                  25                  30
Lys Trp Leu Ile Leu Glu Lys Gln Lys Pro Asp Gly Val Phe Gln Glu
            35                  40                  45
Asp Ala Pro Val Ile His Gln Glu Met Ile Gly Gly Leu Arg Asn Asn
    50                  55                  60
Asn Glu Lys Asp Met Ala Leu Thr Ala Phe Val Leu Ile Ser Leu Gln
65                  70                  75                  80
Glu Ala Lys


<210> 31
<211> 51
```

```
<212> PRT
<213> Homo sapiens

<220>
<221> PEPTIDE
<222> (0)...(0)

<400> 31
Ala Tyr Tyr Glu Asn Ser Pro Gln Gln Val Phe Ser Thr Glu Phe Glu
1               5                   10                  15
Val Lys Glu Tyr Val Leu Pro Ser Phe Glu Val Ile Val Glu Pro Thr
            20                  25                  30
Glu Lys Phe Tyr Tyr Ile Tyr Asn Glu Lys Gly Leu Glu Val Thr Ile
        35                  40                  45
Thr Ala Arg
    50


<210> 32
<211> 56
<212> PRT
<213> Homo sapiens

<220>
<221> PEPTIDE
<222> (0)...(0)

<400> 32
Ala Asp Ile Gly Cys Thr Pro Gly Ser Gly Lys Asp Tyr Ala Gly Val
1               5                   10                  15
Phe Ser Asp Ala Gly Leu Thr Phe Thr Ser Ser Ser Gly Gln Gln Thr
            20                  25                  30
Ala Gln Arg Ala Glu Leu Gln Cys Pro Gln Pro Ala Ala Arg Arg Arg
        35                  40                  45
Arg Ser Val Gln Leu Thr Glu Lys
    50                  55


<210> 33
<211> 50
<212> PRT
<213> Homo sapiens

<220>
<221> PEPTIDE
<222> (0)...(0)

<400> 33
Leu Leu Pro Val Gly Arg Thr Val Met Val Asn Ile Glu Asn Pro Glu
1               5                   10                  15
Gly Ile Pro Val Lys Gln Asp Ser Leu Ser Ser Gln Asn Gln Leu Gly
            20                  25                  30
Val Leu Pro Leu Ser Trp Asp Ile Pro Glu Leu Val Asn Met Gly Gln
        35                  40                  45
Trp Lys
    50


<210> 34
```

<211> 48
<212> PRT
<213> Homo sapiens

<220>
<221> PEPTIDE
<222> (0)...(0)

<400> 34
Ser Ser Lys Ile Thr His Arg Ile His Trp Glu Ser Ala Ser Leu Leu
1               5                   10                  15
Arg Ser Glu Glu Thr Lys Glu Asn Glu Gly Phe Thr Val Thr Ala Glu
            20                  25                  30
Gly Lys Gly Gln Gly Thr Leu Ser Val Val Thr Met Tyr His Ala Lys
            35                  40                  45


<210> 35
<211> 40
<212> PRT
<213> Homo sapiens

<220>
<221> PEPTIDE
<222> (0)...(0)

<400> 35
Thr Ile Tyr Thr Pro Gly Ser Thr Val Leu Tyr Arg Ile Phe Thr Val
1               5                   10                  15
Asn His Lys Leu Leu Pro Val Gly Arg Thr Val Met Val Asn Ile Glu
            20                  25                  30
Asn Pro Glu Gly Ile Pro Val Lys
            35                  40


<210> 36
<211> 40
<212> PRT
<213> Homo sapiens

<220>
<221> PEPTIDE
<222> (0)...(0)

<400> 36
Tyr Arg Gly Asp Gln Asp Ala Thr Met Ser Ile Leu Asp Ile Ser Met
1               5                   10                  15
Met Thr Gly Phe Ala Pro Asp Thr Asp Asp Leu Lys Gln Leu Ala Asn
            20                  25                  30
Gly Val Asp Arg Tyr Ile Ser Lys
            35                  40


<210> 37
<211> 25
<212> PRT
<213> Homo sapiens

<220>

```
<221> PEPTIDE
<222> (0)...(0)

<400> 37
Glu Asp Gly Lys Leu Asn Lys Leu Cys Arg Asp Glu Leu Cys Arg Cys
1               5                   10                  15
Ala Glu Glu Asn Cys Phe Ile Gln Lys
                20                  25


<210> 38
<211> 26
<212> PRT
<213> Homo sapiens

<220>
<221> PEPTIDE
<222> (0)...(0)

<400> 38
Asp Ala Pro Asp His Gln Glu Leu Asn Leu Asp Val Ser Leu Gln Leu
1               5                   10                  15
Pro Ser Arg Ser Ser Lys Ile Thr His Arg
                20                  25


<210> 39
<211> 62
<212> PRT
<213> Homo sapiens

<220>
<221> PEPTIDE
<222> (0)...(0)

<400> 39
Ile Leu Leu Gln Gly Thr Pro Val Ala Gln Met Thr Glu Asp Ala Val
1               5                   10                  15
Asp Ala Glu Arg Leu Lys His Leu Ile Val Thr Pro Ser Gly Cys Gly
                20                  25                  30
Glu Gln Asn Met Ile Gly Met Thr Pro Thr Val Ile Ala Val His Tyr
                35                  40                  45
Leu Asp Glu Thr Glu Gln Trp Glu Lys Phe Gly Leu Glu Lys
        50                  55                  60


<210> 40
<211> 41
<212> PRT
<213> Homo sapiens

<220>
<221> PEPTIDE
<222> (0)...(0)

<400> 40
Gly Pro Leu Leu Asn Lys Phe Leu Thr Thr Ala Lys Asp Lys Asn Arg
1               5                   10                  15
Trp Glu Asp Pro Gly Lys Gln Leu Tyr Asn Val Glu Ala Thr Ser Tyr
```

```
                        20                25                    30
    Ala Leu Leu Ala Leu Leu Gln Leu Lys
            35                40


    <210> 41
    <211> 41
    <212> PRT
    <213> Homo sapiens

    <220>
    <221> PEPTIDE
    <222> (0)...(0)

    <400> 41
    His Leu Ile Val Thr Pro Ser Gly Cys Gly Glu Gln Asn Met Ile Gly
     1               5                10                  15
    Met Thr Pro Thr Val Ile Ala Val His Tyr Leu Asp Glu Thr Glu Gln
                20                25                    30
    Trp Glu Lys Phe Gly Leu Glu Lys Arg
            35                40


    <210> 42
    <211> 36
    <212> PRT
    <213> Homo sapiens

    <220>
    <221> PEPTIDE
    <222> (0)...(0)

    <400> 42
    Ile Arg Ala Tyr Tyr Glu Asn Ser Pro Gln Gln Val Phe Ser Thr Glu
     1               5                10                  15
    Phe Glu Val Lys Glu Tyr Val Leu Pro Ser Phe Glu Val Ile Val Glu
                20                25                    30
    Pro Thr Glu Lys
            35
```